# FASCICULE DE BREVET EUROPEEN

(11) **EP 2 421 864 B1**
(45) Date de publication et mention de la délivrance du brevet: **06.08.2014**
(21) Numéro de dépôt: 10723723.2
(22) Date de dépôt: 22.04.2010
(51) Int. Cl.: C07D 471/04, A61K 31/4745, A61P 35/00

(54) **DERIVES DE 1-PYRAZOLO[4,3-c]ISOQUINOLEINES, LEUR PREPARATION ET LEUR APPLICATION EN THERAPEUTIQUE**
1-PYRAZOLO[4,3-C]ISOCHINOLINDERIVATE, DEREN HERSTELLUNG UND DEREN THERAPEUTISCHE VERWENDUNG
1-PYRAZOLO[4,3-C]ISOQUINOLINE DERIVATIVES, PREPARATION THEREOF AND THERAPEUTIC USE THEREOF

(30) Priorité: 24.04.2009 FR 0901995
(43) Date de publication de la demande: 29.02.2012
(73) Titulaire: SANOFI, 75008 Paris (FR)
(72) Inventeur: DELETTRE, Georges, F-75013 Paris (FR); DEPRETS, Stéphanie, F-75013 Paris (FR); HALLEY, Frank, F-75013 Paris (FR); SCHIO, Laurent, F-75013 Paris (FR); THOMPSON, Fabienne, F-75013 Paris (FR)
(74) Mandataire: Jacobson, Claude
(86) Numéro de dépôt international: PCT/FR2010/050773
(87) Numéro de publication internationale: WO 2010/122272

(56) Documents cités:
- WO-A-2007/060198
- GB-A- 2 185 255
- US-A1- 2004 097 541
- US-A1- 2007 032 515

## Description

La présente invention se rapporte à des dérivés de 1H-pyrazolo[4,3-c]isoquinoléines et à leur application en thérapeutique, notamment comme anticancéreux. L'invention se rapporte aussi au procédé de préparation des composés de l'invention ainsi qu'à certains des intermédiaires réactionnels dudit procédé.

WO 2007/060198 décrit des pyrazolo[3,4-f]isoquinoléines et US 2007/032515 décrit des pyrazolo[3,4-c]isoquinoléines.

GB 2 185 255 et US 2004/097541 divulguent des pyrazolo[4,3-c]isoquinoléines qui diffèrent des composés de l'invention par la nature du substituant en position 3 de l'anneau pyrazolique.

### [Description de l'invention]

### Définitions utilisées

Dans le cadre de la présente invention, on entend par :
- atome d'halogène (Hal) : un atome de fluor, de chlore, de brome ou d'iode ;
- groupe alkyle : un groupe hydrocarboné aliphatique saturé comprenant de 1 à 6 atomes de carbone (avantageusement, de 1 à 4 atomes de carbone) linéaire ou ramifié, obtenu en enlevant un atome d'hydrogène d'un alcane. A titre d'exemples, on peut citer les groupes méthyle, éthyle, propyle, isopropyle, butyle, iso-butyle ou tertio-butyle ;
- groupe alcaxy : un groupe -O-alkyle, où le groupe affcyle est tel que défini ci-dessus ;
- groupe filuoroalkyle : un groupe alkyle comprenant un ou plusieurs atome(s) de fluor à la place d'un ou de plusieurs atome(s) d'hydrogène ; -,
- groupe cycloalkyle : un groupe alkyle cyclique comprenant entre 3 et 7 atomes de carbone, tous engagés dans la structure cyclique. A titre d'exemples, on peut citer les groupes cyclopropyle, cyclobutyle, cyclopentyle ou cyclohexyle ;
- groupe protecteur : un groupe destiné à protéger une fonction chimique de réactions chimiques indésirées introduit au cours d'une étape de protection et qui est libéré au cours d'une étape ultérieure. On trouvera des exemples de groupements protecteurs dans T.W.Greene et coll. « Protective Groups in Organic Synthesis », 3rd edition, 1999, Wiley-Interscience ou dans J.F.W. McOmie « Protective Groups in Organic Chemistry », Plenum Press, 1973. Comme exemples de groupements protecteurs, on peut citer le carbamate de *tert*-butyle (BOC) ou le groupement [2-(triméthylsilyl)éthoxy]méthyl (SEM).

**Selon un 1^{er} aspect**, la présente invention a pour objet un composé de formule (I) : 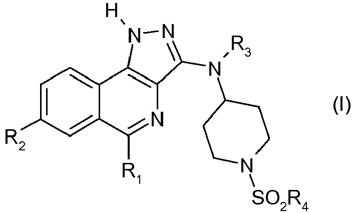
**R₁** représente un groupe phényle éventuellement substitué par un ou plusieurs atome(s) d'halogène. Il s'agit plus particulièrement du groupe 2,6-difluoro-phényle.
**R₂** représente :
   - un atome d'hydrogène ou d'halogène ou un groupe cyano ;
   - un groupe -C(=O)Y dans lequel Y représente un atome d'hydrogène, un groupe -NH₂ ou - ORa ;
   - un groupe-C(=S)NH2 ;
   - un groupe-C(=NH)NH-OH ;
   - un groupe -CH₂OH ou -CH₂F ;
   - un groupe -CH=N-OH ;
   - un groupe -CH=CH₂ ou -C≡C-Rₐ ;
   - un groupe 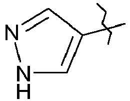 (4-pyrazolyle) ou 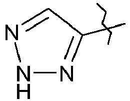 (3-triazolyle) ;
**Rₐ** représentant un atome d'hydrogène ou un groupe (C₁-C₄)alkyle. Plus particulièrement, Rₐ représente un atome d'hydrogène. **R₂** peut être choisi plus particulièrement parmi les groupes **R₂** exemplifiés dans le Tableau 1.
**R₃** représente un atome d'hydrogène ou un groupe (C₁-C₄)alkyle. Plus particulièrement, **R₃** représente un atome d'hydrogène.
**R₄** représente un groupe -NH₂, (C₁-C₄)alkyle, (C₁-C₄)fluoroalkyle, ou (C₃-C₇)cycloalkyle. **R₄** peut être choisi plus particulièrement parmi les groupes **R₄** exemplifiés dans le Tableau I.

Parmi les composés de formule (I), on distingue le sous-groupe de composés de formule (II) : 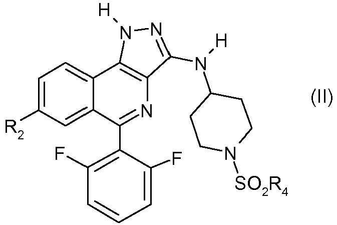 dans laquelle **R₂** et **R₄** sont tels que définis précédemment.

Parmi les composés de l'invention, on peut citer plus particulièrement les composés du Tableau I.

Les composés de l'invention peuvent exister à l'état de bases ou de sels d'addition à des acides. Ces sels sont avantageusement préparés avec des acides pharmaceutiquement acceptables, mais les sels d'autres acides utiles, par exemple, pour la purification ou l'isolement des composés font également partie de l'invention. Les composés peuvent aussi éventuellement comporter un ou plusieurs atomes de carbone asymétriques. Ils peuvent donc exister sous forme d'énantiomères ou de diastéréoisomères. Ces énantiomères, diastéréoisomères, ainsi que leurs mélanges font partie de l'invention.

**Selon un 2^{ème} aspect**, l'invention a pour objet un procédé de préparation des composés de l'invention ainsi que certains des intermédiaires réactionnels impliqués dans ledit procédé.

### préparation des composés de formule (I)

Les composés de formule (I) sont préparés en plusieurs étapes à partir de **P₁** et **P₂** selon le **Schéma 1 :**

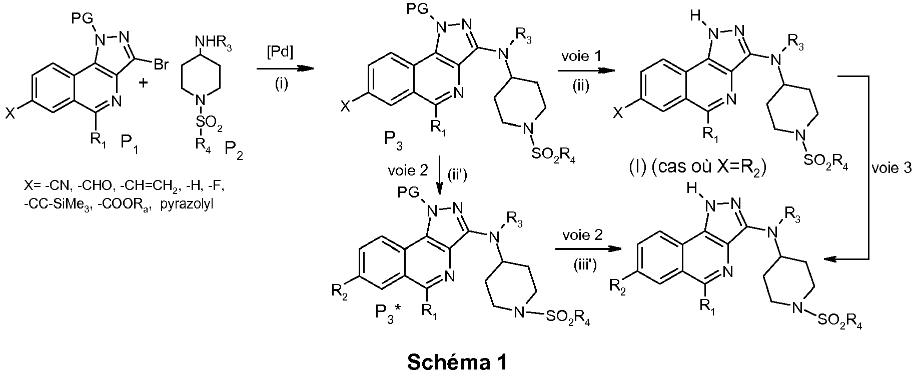

On réalise à l'étape (i) un couplage de type Büchwald-Hartwig entre **P₁** et **P₂** conduisant à **P₃**. PG désigne un groupe protecteur de la fonction NH du cycle pyrazole et X représente l'un des groupes suivants : -CN, -CHO, -CH=CH₂, -H, -F, -C≡C-SiMe₃, -COORₐ ou

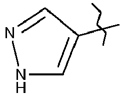

Ce type de couplage est réalisé en présence d'un complexe de palladium (à l'état d'oxydation (0) ou (II) éventuellement préparé *in situ*). On peut utiliser par exemple Pd(PPh₃)₄, PdCl₂(PPh₃)₂, Pd(OAc)₂ ou PdCl₂(dppf), Pd₂(dba)₃ ou un mélange de Pd(OAc)₂ et de 9,9-diméthyl-4,5-bis(diphénylphosphino)xanthène. Les complexes les plus fréquemment utilisés sont des complexes de palladium(0). Le couplage est favorisé en présence d'une base, qui peut être par exemple K₂CO₃, NaHCO, Et₃N, K₃PO₄, Ba(OH)₂, NaOH, KF, CsF, Cs₂CO₃... Le couplage peut être conduit dans un solvant polaire tel que par exemple le 1,4-dioxane. On trouvera des exemples de systèmes catalytiques à base de palladium dans : Guram, A.; Rennels, R.; Buchwald, S. ACIEE 1995, 34, 1348 ; Louie, J.; Hartwig, J. Tet. Lett. 1995, 3609 ; Wolfe, J.; Wagaw, S.; Buchwald, S. J. Am. Chem. Soc. 1996, 118, 7215 ; Driver, M. ; Hartwig, J. J. Am. Chem. Soc. 1996, 118, 7217 ; Hamann, B.; Hartwig, J. J. Am. Chem. Soc. 1998, 120, 7369 ; Kawatsura, M.; Hartwig, J. J. Am. Chem. Soc. 1999, 121, 1473.

Dans le cas où le substituant **X** représente l'un des substituants **R₂** défini précédemment, le composé de formule (I) est obtenu après déprotection du groupe protecteur PG à l'étape (ii) de la voie 1. Les conditions de la déprotection dépendent de la nature de PG et sont connues de l'homme du métier (voir par ex. T.W.Greene et coll. « Protective Groups in Organic Synthesis », 3rd edition, 1999). Par exemple, dans le cas du SEM (β-(triméthylsilyl)éthoxy]méthyle), la déprotection est réalisée en présence d'un acide tel que l'acide trifluoroacétique (TFA) ou l'acide chlorhydrique.

Dans le cas où le substituant **X** ne représente pas l'un des substituants **R₂**, on utilise une ou plusieurs réaction(s) chimique(s) connue(s) de l'homme du métier pour transformer le substituant X en substituant R₂. Selon la voie 2, **P₃** est transformé en **P₃*** à l'étape (ii'), puis le composé de formule (I) est obtenu après déprotection de PG à l'étape (iii'). Selon la voie alternative 3, la transformation s'effectue directement sur un composé de formule (I).

On trouvera des exemples de transformations X ⇒ R₂ ci-dessous :
- X= -CN ⇒ R₂= -C(=O)NH₂ (ét.2-ex.4 et ex.14) ou -C(=O)OH : hydrolyse de la fonction nitrile, par exemple à l'aide d'hydroxyde de sodium ;
- X= -CN ⇒ R₂= -C(=S)NH₂ (ex.9) : cette réaction peut s'effectuer à l'aide de sulfure d'ammonium dans le méthanol sous microondes (voir Synlett 2004, 14, 2615-2617) ;
- X= -CN ⇒ R₂= -C(=O)H : cette réduction peut s'effectuer en présence d'hydrure de diisobutylaluminium (ét.1-ex.23) ;
- X= -C(=O)H ⇒ R₂= -CH=N-OH : transformation de la fonction aldéhyde en fonction oxime en présence de NH₂OH (ex.20) ;
- X= -C(=O)H ⇒ R₂= -CH₂OH (ex.3) : réduction de la fonction aldéhyde à l'aide d'un agent réducteur de la fonction aldéhyde, par exemple à l'aide de NaBH₄ ;
- X= -C=C-SiMe₃ ⇒ R₂= -C≡CH (ét.4-ex.7) : réaction pouvant avoir lieu en présence de fluorure de tétrabutylammonium ;
- X= -C(=O)H ⇒ -CH₂OH ⇒ R₂= -CH₂F (ét.1-ex.17) : la fluoration est réalisée à l'aide de trifluorure de diéthylaminosulfure (DAST) (cf. A.H. Fauq, "N,N-Diethylaminosulfur Trifluoride" in Encyclopedia of Reagents for Organic Synthesis, Ed: L. Paquette 2004, J. Wiley & Sons, New York.) ;
- X= -C≡C-SiMe₃ ⇒ -C≡CH ⇒ R₂= 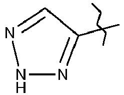 réaction de cycloaddition de Huisgen entre la fonction alcyne et l'azoture de triméthylsilyle N₃SiMe₃ permettant de préparer le cycle 1,2,3-triazole.

### préparation des composés P₁

Les composés **P₁** pour lesquels X représente -H, -Hal, -COOH ou -CN sont obtenus selon le **Schéma 2** par bromation en milieu basique de **P₄** suivie d'une protection par le groupe protecteur PG. La bromation s'effectue par exemple à l'aide de brome en présence de KOH dans un solvant polaire tel que le DMF. Le groupement SEM introduit par SEM-CI en présence d'une base telle que par exemple la düsopropyléthylamine (DIPEA) ou le groupement SO₂NMe₂ (voir ex.1) introduit par Cl-SO₂NMe₂ sont des exemples de groupes protecteurs utilisables.

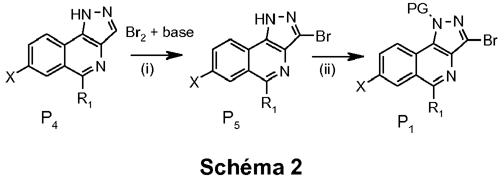

Les composés P₁ pour lesquels X représente -CHO, -CH=CH₂, -C≡SiMe₃, 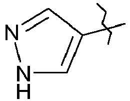 sont obtenus en une ou plusieurs étapes à partir du composé P₅ ou P₁ précédent pour lequel X représente -1 et A représente un atome d'hydrogène (P₅) ou PG (P₁) (Schéma 3) :

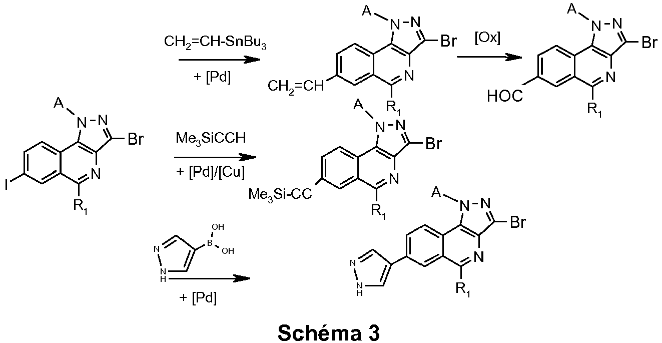

Les réactions détaillées sur le **Schéma 3** sont :
- pour X= -CH=CH₂ : réaction de Stille utilisant -CH=CH-SnBu₃ et un complexe de palladium(0) ;
- pour X= -CHO : réaction d'oxydation utilisant par exemple le tétroxyde d'osmium ;
- pour X= -C≡C-SiMe₃ : réaction de Sonogashira en présence de HC≡C-SiMe₃ et d'un complexe de palladium (0) ou (II) et d'un sel de cuivre (pour plus de détails sur cette réaction, voir Chem.Rev. 2007, 107(3), 874-922 ; cf. aussi ét.2-ex.7) ;
- pour X= 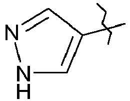 couplage de Suzuki avec l'acide 4-pyrazole boronique en présence d'un complexe de palladium (0) ou (II) et d'une base (il est possible d'utiliser l'ester pinacolique correspondant) ;

Les composés **P₁** pour lesquels X représente -COO(C₁-C₄)alkyle sont quant à eux obtenus par esterification des composés **P₁** correspondants pour lesquels X=-COOH.

### Préparation des composés P₄

Les composés **P₄** pour lesquels X représente -H ou -Hal sont obtenus à partir de **P₆ (Schéma 4)** qui est soit un produit commercial (par ex. X=I, CAS N°31827-94-8) soit préparé par bromation en milieu acide de l'acétophénone correspondante (cf.ét.1-ex.1) : 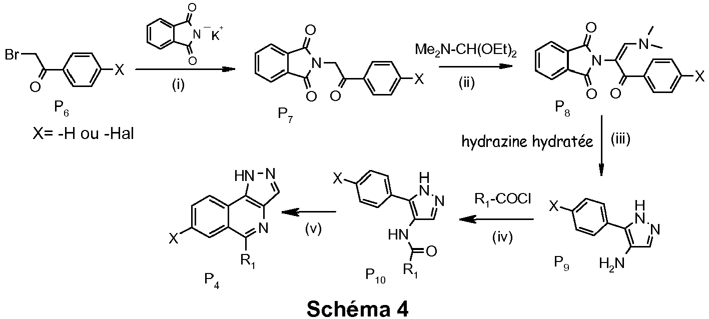
étape (i) : réaction entre **P₆** et le phtalimide de potassium conduisant à **P₇**. La réaction peut être conduite dans un solvant polaire tel que le DMF (cf.ét.2 - ex.1 ou ét.1 - ex.6) ;
étape (ii) : réaction de **P₇** avec le *N*,*N*-diméthylformamide-diéthylacétal conduisant à **P₈**. La réaction peut être conduite directement dans le *N*,*N*-diméthylformamide-diéthylacétal porté au reflux (cf.ét.1-ex.3) ;
étape (iii) : réaction de **P₈** avec l'hydrate d'hydrazine conduisant à **P₉**. La réaction peut être conduite dans un solvant polaire tel que l'éthanol (cf.ét.4-ex.1 ou ét.3-ex.6) ;
étape (iv) : réaction d'acylation de **P₉** avec le chlorure d'acide R₁COCl conduisant à **P₁₀**. La réaction peut être conduite dans un solvant chloré tel que le DCM en présence d'un catalyseur nucléophile tel qu'une pyridine ;
étape (v) : réaction de cyclisation de Bischler-Napieralski de **P₁₀** conduisant à **P₄**. La réaction peut être conduite en présence de P₂O₅ et de dichlorure de l'acide phénylphosphine à une température >100°C (cf.ét.6-ex.1 ou ét.5-ex.6). Il est possible aussi d'utiliser POCl₃.

Les composés **P₄** pour lesquels X représente -CN sont obtenus par cyanation des composés **P₄** correspondants protégés pour lesquels X représente -I. On utilise par exemple Zn(CN)₂ et un complexe de palladium(0) (réaction de Rosenmund-Braun : voir Chem.Rev.1951, 49, 392).

### préparation de P₂

**P₂** peut être préparé selon le **Schéma 5** (cf. également WO 2003082871 ét.1-4 - ex.4 pp.34-36) à partir de l'amino pipéridine protégée sur la fonction amine : 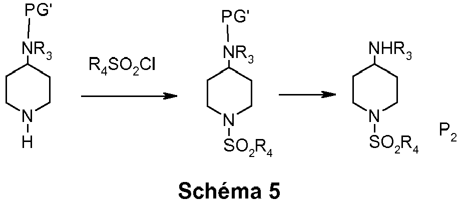 PG' représente un groupe protecteur de la fonction amine. Il s'agit avantageusement du BOC (tert-butoxycarbonyle).

L'invention est donc aussi relative à un procédé de préparation d'un composé de formule : 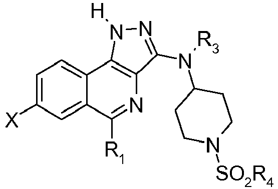 dans laquelle R₁, R₃ et R₄ sont tels que définis précédemment, et X représente un atome d'hydrogène ou de fluor, un groupe cyano, -C(=O)H, -CH=CH₂ , -C≡C-SiMe₃, -COORₐ, Rₐ représentant un atome d'hydrogène ou un groupe (C₁-C₄)alkyle, ou le groupe 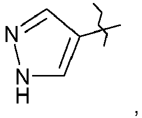 consistant à :
(i) coupler les composés **P₁** et **P₂** définis sur le **Schéma 1** en présence d'un complexe de palladium et éventuellement d'une base,
(ii) déproteger le produit obtenu à l'étape (i) précédente.

L'invention est aussi relative à un procédé de préparation d'un composé de formule : 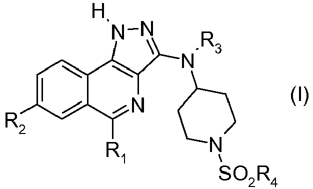 dans laquelle R₁, R₃ et R₄ sont tels que définis précédemment et R₂ représente l'un des groupes suivants : -C(=O)NH₂, -C(=S)NH₂, -C(=O)H, -CH=N-OH, -CH₂OPH, -CH₂F, -C=CH ou 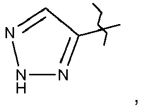 à partir du composé de formule : 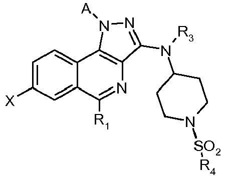 dans laquelle X représente un groupe - CN, -CHO, -CH₂OH, -C≡CH, -C≡C-SiMe₃ et A représente un atome d'hydrogène ou un groupe protecteur PG , consistant à :
(i)
   - hydrolyser le groupe X= -CN en groupe R₂= -C(=O)NH₂ ou -C(=O)OH ;
   - transformer le groupe X= -CN en groupe R₂= -C(=S)NH₂ en présence de sulfure d'ammonium sous microondes ;
   - réduire le groupe X= -CN en groupe R₂= - C(=O)H ;
   - transformer le groupe X= -C(=O)H en groupe R₂= -CH=N-OH en présence de NH₂OH ;
   - réduire le groupe X= -C(=O)H en groupe R₂= -CH₂OH ;
   - transformer le groupe X= -C≡C-SiMe₃ en groupe R₂= -C≡CH ;
   - fluorer le groupe X= -CH₂OH en groupe R₂= -CH₂F ;
   - transformer le groupe X= -C≡CH en groupe R₂= 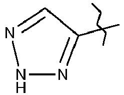 par cycloaddition en présence d'azoture de triméthylsilyle N₃SiMe₃.
(ii) le cas échéant déprotéger le produit obtenu à l'étape (i) précédente.

Les composés de formule (I) peuvent aussi être préparés à partir de **P₁** et **P'₂** selon le **Schéma 6:**

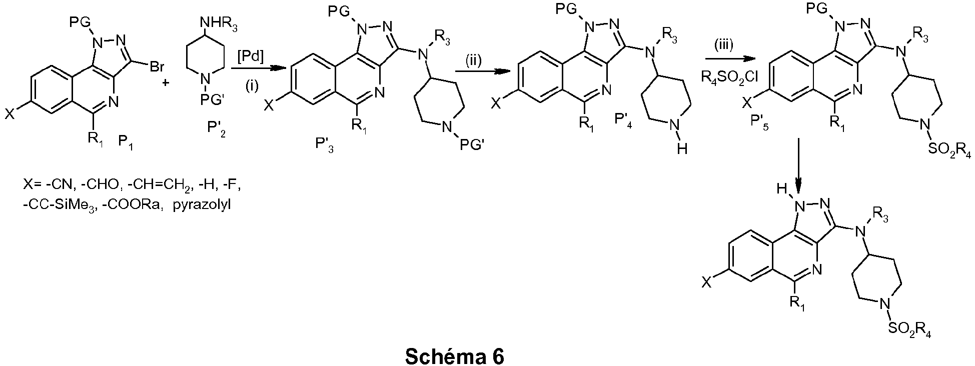

On réalise à l'étape (i) un couplage de type Büchwald-Hartwig entre **P₁** et **P'₂** conduisant à **P'₃**. PG désigne un groupe protecteur de la fonction NH du cycle pyrazole, PG' représente un groupe protecteur de la fonction amine et X représente l'un des groupes suivants : -CN, -CHO, -CH=CH₂, -H, -F, -C≡C-SiMe₃, -COORₐ ou

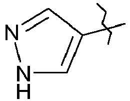

A l'étape (ii), on déprotège la fonction amine pour obtenir **P'₄** qui réagit ensuite à l'étape (iii) avec R₄SO₂Cl pour conduire à **P'₅**. On déprotège ensuite la fonction NH du cycle pyrazole pour obtenir le composé de formule (I).

L'invention est donc aussi relative à un procédé de préparation d'un composé de formule : 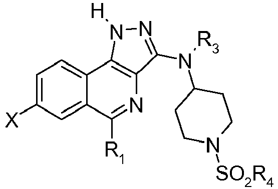 dans laquelle R₁, R₃ et R₄ sont tels que définis précédemment, et X représente un atome d'hydrogène ou de fluor, un groupe cyano, -C(=O)H, -CH=CH₂ , -C≡C-SiMe₃, -COORₐ, Rₐ représentant un atome d'hydrogène ou un groupe (C₁-C₄)alkyle, ou le groupe 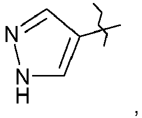 consistant à :
(i') faire réagir le composé **P'₄** de formule : 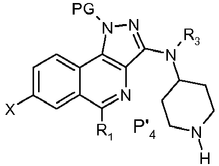 avec R₄SO₂CI,
(ii') déproteger le produit obtenu à l'étape (i) précédente.

L'invention est aussi relative aux composés de formule 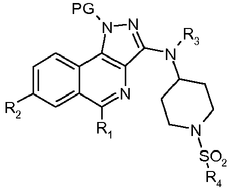 (Schéma 1) ou de formule 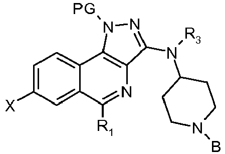 (Schéma 6) dans lesquelles :
■ R₁, R₂, R₃ et R₄ sont tels que définis précédemment ;
■ X représente un atome d'hydrogène ou de fluor, un groupe cyano, -C(=O)H, -CH=CH₂ , -C≡C-SiMe₃, -C≡CH, -COORₐ, Rₐ représentant un atome d'hydrogène ou un groupe (C₁-C₄)alkyle, ou
   le groupe 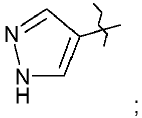
■ PG représente un groupe protecteur de la fonction NH du groupe pyrazole ;
■ B représente un atome d'hydrogène ou un groupe protecteur PG' de la fonction amine.

PG représente plus particulièrement le SEM ou SO₂NMe₂ et PG' le BOC.

**Selon un 3^{ème} aspect**, l'invention concerne une composition pharmaceutique comprenant un composé tel que défini précédemment en combinaison avec un excipient pharmaceutiquement acceptable. L'excipient est choisi selon la forme pharmaceutique et le mode d'administration souhaité, parmi les excipients habituels qui sont connus de l'homme du métier.

**Selon un 4^{ème} aspect**, l'invention a pour objet un médicament qui comprend un composé tel que défini précédemment ainsi que l'utilisation d'un composé tel que défini précédemment, pour la fabrication d'un médicament. Le composé peut être administré en association avec un (ou plusieurs) autre(s) anticancéreux. Ce traitement peuvent être administré simultanément, séparément ou bien séquentiellement. Le traitement sera adapté par le praticien en fonction du malade et de la tumeur à traiter.

**Selon un 5^{ème} aspect**, l'invention concerne également une méthode de traitement des pathologies indiquées précédemment qui comprend l'administration, à un patient, d'une dose efficace d'un composé selon l'invention ou un de ses sels pharmaceutiquement acceptables ou hydrates ou solvats.

### [Exemples]

Les exemples suivants illustrent la préparation de certains composés conformes à l'invention.

### Analyse LC-MS-DAD-ELSD

Colonne : Atlantis T3 ; 3,0 x 50 mm ; granulométrie 3 µm ; réf. Waters 186003721 débit éluant= 0,8 mL/min ; température four : LC=35°C ; DAD longueur d'onde 200-400 nm Gradient en 7 min utilisant un mélange A (eau/ acide formique 0,1%) et de l'acétonitrile B : 0 min (95% A ; 5% B) ; 5 min (5 % A ; 95% B) ; 5,5 min (5% A ; 95% B) ; 6,5 min (95% A ; 5% B) ; 7 min (95% A ; 5% B).

### Analyse ¹H RMN

Les spectres de ¹H RMN ont été obtenus sur un appareil Brüker 400 MHz dans le DMSO-d6. Les déplacements chimiques sont donnés en ppm.

### Préparation des composés du tableau I

### Exemple 1: 5-(2,6-difluorophényl)-3-{[1-(méthylsulfonyl)pipéridin-4-yl]amino}-1H-pyrazolo[4,3-c]isoquinoléine-7-carboxamide

**Etape 1 :** Dans un tricol de 1 L, on introduit 50 g de 4-iodoacétophénone dans 500 mL d'acide acétique glacial. Puis, on coule goutte à goutte, à température ambiante (TA), 10,4 mL de brome. Le milieu réactionnel est agité pendant 2 h à TA puis évaporé sous pression réduite (PR). Le brut est repris 3 fois avec environ 200 mL de toluène, puis une fois avec 200 mL de chlorure de méthylène (DCM) et concentré à chaque fois sous pression réduite (PR) afin d'éliminer toutes traces d'acide acétique et de brome. Le solide orangé obtenu est séché sous vide au dessiccateur à TA. On obtient ainsi 66 g de 2-bromo-1-(4-iodophényl)-éthanone sous forme d'un solide orangé utilisé tel quel à l'étape suivante. Spectre de masse (SM) (E/I) : m/z = 404 (M+) ; RMN ¹H : 4,90 (s, 2H) ; 7,74 (d, J = 8,5 Hz, 2H) ; 7,96 (d, J = 8,5 Hz, 2H).
Etape 2 : Dans un ballon de 500 mL, on introduit 66 g de 2-bromo-1-(4-iodophényl)-éthanone dans 140 mL de DMF et 38 g de phtalimide de potassium. Le mélange est agité à TA pendant 4 h et le précipité formé est filtré sur verre fritté, lavé avec 3 fois 50 mL d'éther isopropylique. On obtient ainsi, après séchage sous PR, 80 g de 2-[2-(4-iodophényl)-2-oxoéthyl]-isoindole-1,3-dione sous forme de solide blanc utilisé tel quel à l'étape suivante. SM (E/I) : m/z = 391 (M+) ; RMN 1H : 5,21 (s, 2H) ; 7,84 (d, J = 8,5 Hz, 2H) ; de 7,88 à 7,98 (m, 4H) ; 8,00 (d, J = 8,5 Hz, 2H). PF (Kofler) : 230°C
Etape 3: Dans un ballon de 500 mL, on introduit 81,6 g de 2-[2-(4-iodophényl)-2-oxoéthyl]-isoindole-1,3-dione dans 134 mL de N,N-diméthyformamide diéthyl acétal. Le milieu réactionnel est porté au reflux 5 h puis est refroidi à l'aide d'un bain de glace vers 0°C. Le précipité formé est essoré sur verre fritté, lavé par deux fois 50 mL d'éther isopropylique, puis on effectue 2 lavages par clairçage (lavage du gâteau sans agitation) avec 50 mL d'éther isopropylique. Après séchage sous PR, on obtient 79 g de mélange des isomères E et Z de 2-[2-diméthylamino-1-(4-iodobenzoyl)-vinyl]-isoindole-1,3-dione sous forme de solide jaune orangé. SM (E/I) : m/z = 446 (M+) ; RMN ¹H : 2,92 (m large, 6H) ; 7,22 (d, J = 8,5 Hz, 2H) ; 7,49 (s large, 1H) ; 7,80 (d, J = 8,5 Hz, 2H) ; 7,92 (m, 4H) ; PF (Kofler) : 226°C
Etape 4 : Dans un ballon 1 L, on introduit à TA, 78,3 g de mélange des isomères E et Z de 2-[2-diméthylamino-1-(4-iodobenzoyl)-vinyl]-isoindole-1,3-dione dans 535 mL d'éthanol. On obtient une suspension brune à laquelle on additionne, 21 mL d'hydrate d'hydrazine. La suspension est portée au reflux. Après 30 min de chauffage, on observe un épaississement du mélange qui prend une couleur jaune citron. Cette suspension est agitée au reflux pendant 4h30 puis le mélange est ramené à TA. On essore la suspension sur fritté et le précipité est lavé 2 fois par 100 mL d'éthanol. On obtient un filtrat qui, après évaporation, donne 46 g d'un solide jaune 1, et il reste le précipité d'origine 2, que l'on sèche sous PR à 40°C, et obtient ainsi 39 g d'une poudre de couleur crème. 1 est agité 15 min dans 300 mL d'AcOEt. On filtre et on recommence cette opération 13 fois en utilisant 150 mL d'AcOEt à chaque fois. Les différents filtrats sont réunis et concentrés sous PR. On obtient ainsi 24,3 g d'un solide de couleur jaune or. 2 est repris dans 300 mL d'AcOEt. On filtre et on recommence l'opération 7 fois en utilisant 150 mL d'AcOEt à chaque fois. Les différents filtrats sont réunis et on concentre sous PR. On obtient 7,3 g d'un solide de couleur jaune citron. Les deux lots sont réunis pour donner 31,6 g de 3-(4-iodophényl)-1H-pyrazol-4-ylamine (solide orangé). SM (E/I) : m/z = 285 (M+) ; RMN ¹H : 4,04 (s large, 2H) ; 7,15 (s, 1H) ; 7,57 (d large, J = 8,5 Hz, 2H) ; 7,72 (d, J = 8,5 Hz, 2H) ; 12,35 (m étalé, 1H).
Etape 5 : Dans un ballon 2 L, on introduit, 31,5 g de 3-(4-iodophényl)-1H-pyrazol-4-ylamine dans 425 mL de DCM et 278 mL de pyridine. Le mélange est refroidi à 0°C à l'aide d'un bain de glace et 13,9 mL du chlorure de l'acide 2,6-difluorobenzoïque sont additionnés. On laisse remonter progressivement à la TA sur 3 h. Après évaporation du solvant sous PR, on ajoute de l'eau sur le brut réactionnel puis la phase aqueuse est extraite à l'AcOEt. Les extraits organiques sont regroupés, lavés avec une solution saturée en NaCl, séchés sur MgSO₄. On évapore alors l'AcOEt sous vide et obtient ainsi 49,2 g d'un mélange de 2,6-difluoro-N-[5-(4-iodophényl)-1H-pyrazol-4-yl]-benzamide et de N-[1-(2,6-difluoro-benzoyl)-5-(4-iodophényl)-1H-pyrazol-4-yl]-2,6-difluorobenzamide sous forme d'une meringue orangée qui est utilisée telle quelle. Le brut réactionnel est traité de façon à obtenir uniquement le produit attendu: dans un ballon de 3 L, on dissout à TA et sous argon, 49,2 g du mélange de 2,6-difluoro-N-[5-(4-iodophényl)-1H-pyrazol-4-yl]-benzamide et de N-[1-(2,6-difluoro-benzoyl)-5-(4-iodophényl)-1H-pyrazol-4-yl]-2,6-difluoro-benzamide dans 950 mL d'éthanol et 470 mL de THF, puis on ajoute 385 mL de solution aqueuse de soude 5N. Après 1 h d'agitation à TA, on coule le mélange dans 1025 mL d'acide chlorhydrique 2N. On extrait à l'AcOEt, lave les phases organiques avec une solution saturée en NaCl, sèche sur MgSO₄ puis concentre sous PR. On obtient un produit brut que l'on met en suspension dans 570 mL de DCM et on agite au reflux du DCM pendant 15 min. Après refroidissement à l'aide d'un bain de glace, on essore le précipité, lave 2 fois avec 30 mL de DCM glacé. On obtient 25,8 g de 2,6-difluoro-N-[5-(4-iodophényl)-1H-pyrazol-4-yl]-benzamide (solide crème). SM (E/I) : m/z = 425 (M+) ; RMN ¹H : de 7,15 à 7,25 (m, 2H) ; 7,51 (d, J = 8,5 Hz, 2H) ; 7,57 (m, 1H) ; 7,79 (d, J = 8,5 Hz, 2H) ; 7,93 (m étalé, 1 H) ; 10,2 (s, 1 H) ; 13,3 (m étalé, 1 H) ; PF (Banc Kofler) : 204°C.
Etape 6 : Dans un ballon de 500 mL, muni d'une agitation magnétique, d'un septum surmonté d'une arrivée d'argon, on introduit 9,2 g de 2,6-difluoro-N-[5-(4-iodophényl)-1H-pyrazol-4-yl]-benzamide, 168 mL de dichlorure de l'acide phénylphosphine, et 46,3 g de pentoxyde de phosphore. Après 23 h à 165°C, le mélange est refroidi à TA puis versé dans un mélange de 260 mL d'eau et de 400 g de glace. La température s'élève jusqu'à 40°C. On neutralise par addition lente de 930 mL d'une solution de carbonate de sodium 3M puis de 110 mL d'ammoniaque aqueuse à 28%. On extrait avec 1 fois 1150 mL et 2 fois 550 mL d'AcOEt. Les extraits organiques sont regroupés, lavés avec une solution saturée en NaCl et séchés sur MgSO₄, puis concentrés sous PR. Le solide obtenu est agité pendant 15 min au reflux dans 220 mL de DCM, refroidi à l'aide d'un bain de glace puis filtré et lavé par 2 fois 28 mL de DCM glacé. Après séchage sous vide à 40°C, on obtient 7,4 g de 5-(2,6-difluorophényl)-7-iodo-1H-pyrazolo[4,3-c]isoquinoléine (solide crème). SM (E/I) : m/z = 407 (M+) ; RMN 1 H :7,39 (t, J = 8,0 H, 2H) ; 7,71 (m, 1 H) ; 7,99 (s large, 1 H) ; 8,33 (m, 2H) ; 8,45 (s large, 1 H) ; 14,35 (s large, 1 H) ; PF (Banc Kofler)= 260°C.
Etape 7 : Dans un ballon de 1000 mL sous argon, on introduit 7,4 g de 5-(2,6-difluorophényl)-7-iodo-1H-pyrazolo[4,3-c]isoquinoléine dans 190 mL de tétrahydrofurane et de 20 mL de diméthylformamide (DMF), après refroidissement vers 0°C à l'aide d'un bain de glace, on ajoute 1 g de NaH (à 50% en suspension dans l'huile de vaseline) et agite 30 min à 0°C, puis additionne à cette même température 2,7 mL de chlorure de diméthylsulfamoyle. Après 1 h à TA, le brut est versé sur une solution saturée en NH₄CI. La phase aqueuse est extraite avec 3 fois 300 mL d'AcOEt. Les extraits organiques sont regroupés, séchés sur MgS0₄, puis concentrés sous PR. Après purification par flash-chromatographie sur gel de silice (40-63 µm), en éluant par un mélange DCM/méthanol (98/02 vol), on obtient 9 g d'un mélange des régioisomères de position 5-(2,6-difluorophényl)-7-iodo-pyrazolo[4,3-c]isoquinoléine-1-acide diméthylamide sulfonique et 5-(2,6-difluoro-phényl)-7-iodo-pyrazolo[4,3-c]isoquinoléine-2-acide diméthylamide sulfonique sous forme d'un solide. SM(E/I) : m/z = 514 (M+) ; RMN ¹H : 3,02 (s, 6H) ; 7,40 (t, J = 8,0 Hz, 2H) ; 7,74 (m, 1 H) ; 7,92 (s large, 1 H) ; 8,32 (dd, J = 1,5 et 8,5 Hz, 1 H) ; 8,39 (d, J = 8,5 Hz, 1 H) ; 9,31 (s, 1 H) ; PF = 224°C
Etape 8 : Dans un ballon de 500 mL, muni d'une agitation magnétique, d'un réfrigérant surmonté d'une arrivée d'argon, on introduit 5,4 g du solide obtenu à l'étape 7 dans 100 mL de DMF. Après un barbotage de 10 min d'argon dans le milieu réactionnel, on ajoute 1,6 g de cyanure de zinc, 0,28 g de Pd(PPh₃)₄ et l'on porte le mélange à 130°C sous agitation pendant 1 h30. Le brut réactionnel est versé dans 100 volumes d'eau, la phase aqueuse est extraite avec 3 fois 400 mL d'AcOEt puis les extraits organiques sont regroupés, séchés sur MgSO₄ et concentrés sous PR. Le résidu obtenu est empâté dans l'oxyde de diisopropyle, filtré et séché au dessiccateur à 35°C. On obtient ainsi 3,3 g de mélange des régioisomères 7-cyano-5-(2,6-difluorophényl)-pyrazolo[4,3-c]isoquinoléine-1-acide diméthylamide sulfonique (solide crème). SM (E/I) : m/z = 413 (M+) ; RMN ¹H : 3,03 (s, 6H) ; 7,39 (t, J = 8,0 Hz, 2H) ; 7,75 (m, 1 H) ; 8,24 (s large, 1 H) ; 8,38 (dd, J = 1,5 et 8,5 Hz, 1 H) ; 8,76 (d, J = 8,5 Hz, 1 H) ; 9,41 (s, 1 H) ; PF : 231 °C (Banc Kofler)
Etape 9 : Dans un ballon de 250 mL, on introduit 4 g de mélange des régioisomères 7-cyano-5-(2,6-difluorophényl)-pyrazolo[4,3-c]isoquinoléine-1-acide diméthylamide sulfonique dans 160 mL de DCM. Après refroidissement vers 0°C à l'aide d'un bain de glace, on ajoute 108 mL de TFA et agite à TA pendant 6 h. Le mélange est concentré sous PR et le solide obtenu est repris dans 600 mL d'eau et 50 mL d'une solution d'ammoniaque 0,75 M sont ajoutés. La phase aqueuse est extraite avec 3 fois 150 mL d'AcOEt. Les extraits organiques sont regroupés, lavés avec une solution saturée en NaCl, puis séchés sur MgSO₄. Après concentration de l'AcOEt sous PR, on obtient 2,6 g de 5-(2,6-difluorophényl)-1H-pyrazolo[4,3-c]isoquinoléine-7-carbonitrile (solide beige). SM (E/I) m/z = 306 (M+) ; RMN 1 H : 7,38 (t, J = 8,0 Hz, 2H) ; 7,72 (m, 1 H) ; 8,24 (s large, 1 H) ; 8,34 (dd, J = 1,5 et 8,5 Hz, 1 H) ; 8,59 (s, 1 H) ; 8,70 (d, J = 8,5 Hz, 1 H) ; 14,5 (m très étalé, 1 H).
Etape 10 : Dans un ballon de 250 mL, muni d'une agitation magnétique et d'un septum surmonté d'une arrivée d'argon, on introduit 2,6 g de 5-(2,6-difluorophényl)-1H-pyrazolo[4,3-c]isoquinoléine-7-carbonitrile dans 85 mL de DMF, 0,87 mL de brome et 1,5 g de KOH. Après 1 h d'agitation à TA, on verse le mélange sur de l'eau glacée. La phase aqueuse est extraite avec 3 fois 150 mL d'AcOEt. Les extraits organiques sont regroupés, lavés avec une solution saturée en NaCl, séchés sur MgSO₄ et évaporés sous PR. On obtient ainsi 3,3 g de 3-bromo-5-(2,6-difluorophényl)-1H-pyrazolo[4,3-c]isoquinoléine-7-carbonitrile utilisé tel quel pour l'étape suivante (solide jaune clair). SM (E/I) : m/z = 385 (M+) ; RMN ¹H : 7,39 (t, J = 8,0 Hz, 2H) ; 7,75 (m, 1H) ; 8,34 (s large, 1H) ; 8,41 (dd, J = 1,5 et 8,5 Hz, 1 H) ; 8,68 (d, J = 8,5 Hz, 1 H) ; 14,85 (m étalé, 1 H).
Etape 11 : Dans un ballon de 250 mL, muni d'une agitation magnétique, d'un septum surmonté d'une arrivée d'argon, on introduit 3,3 g de 3-bromo-5-(2,6-difluorophényl)-1H-pyrazolo[4,3-c]isoquinoléine-7-carbonitrile dans 85 mL de DMF, 10,6 mL de N,N-dopropyléthylamine et 3,2 mL de 2-(triméthylsilyl)éthoxyméthyl chloride. Après 5 h à TA, le mélange est versé dans 8 volumes d'eau glacée. La phase aqueuse est extraite avec 3 fois 200 mL d'AcOEt, les extraits organiques sont regroupés, lavés avec une solution saturée en NaCl, séchés sur MgSO₄ et concentrés sous PR. On purifie par flash-chromatographie sur gel de silice (40-63 µm), en éluant par un mélange de DCM et de méthanol (99,5/0,5 vol), on obtient d'une part 2,1 g de 3-bromo-5-(2,6-difluorophényl)-1-{[2-(triméthylsilyl)éthoxy]méthyl}-1H-pyrazolo[4,3-c]isoquinoléine-7-carbonitrile sous forme d'un solide blanc. SM (E/I) : m/z =515 (M+) ; RMN ¹H : -0,12 (s, 9H) ; 0,87 (t, J = 8,0 Hz, 2H) ; 3,65 (t, J = 8,0 Hz, 2H) ; 6,17 (s, 2H) ; 7,40 (t, J = 8,0 Hz, 2H) ; 7,77 (m, 1H) ; 8,41 (s large, 1H) ; 8,47 (dd, J = 1,5 et 8,5 Hz, 1H) ; 8,77 (d, J = 8,5 Hz, 1H). Et d'autre part 1,2 g de 3-bromo-5-(2,6-difluorophényl)-2-{[2-(triméthylsilyl)éthoxy]méthyl}-2H-pyrazolo[4,3-c]isoquinoléine-7-carbonitrile (solide blanc). SM (E/I) : m/z =515 (M+) ; RMN ¹H : -0,05 (s, 9H) ; 0,90 (t, J = 8,0 Hz, 2H) ; 3,73 (t, J = 8,0 Hz, 2H) ; 5,92 (s, 2H) ; 7,38 (t, J = 8,0 Hz, 2H) ; 7,76 (m, 1H) ; 8,24 (s large, 1H) ; 8,34 (dd, J = 1,5 et 8,5 Hz, 1 H) ; 8,69 (d, J = 8,5 Hz, 1 H).
Etape 12 : Dans un ballon de 150 mL, muni d'une agitation magnétique et d'un septum surmonté d'une arrivée d'argon, on introduit 1 g de 3-bromo-5-(2,6-difluorophényl)-1-{[2-(triméthylsilyl)éthoxy]méthyl}-1H-pyrazolo[4,3-c]isoquinoléine-7-carbonitrile dans 27 mL de 1-4-dioxane. Après un barbotage de 10 min d'argon dans le milieu réactionnel, on ajoute 692 mg de 1-méthanesulfonyl-pipéridin-4-ylamine, 4 g de carbonate de césium, 135 mg de 9,9-diméthyl-4,5-bis(diphénylphosphino)xanthène et 43 mg d'acétate de palladium (II). Le mélange réactionnel est chauffé au reflux pendant 4 h. Après refroidissement, le mélange est coulé dans de l'eau, extrait à l'AcOEt, lavé avec une solution saturée en NaCl, séché sur MgSO₄, filtré et concentré sous PR. On purifie par flash-chromatographie sur gel de silice (40-63 µm), en éluant par un mélange de DCM et d'AcOEt (85/15 vol), on obtient ainsi, 1 g de 5-(2,6-difluorophényl)-3-{[1-(méthylsulfonyl)pipéridin-4-yl]amino}-1-{[2-(triméthylsilyl)éthoxy]méthyl}-1H-pyrazolo[4,3-c]isoquinoléine-7-carbonitrile (solide jaune). SM (E/I) : m/z =596 (M+) ; RMN ¹H : -0,05 (s, 9H) ; 0,89 (t, J = 8,0 Hz, 2H) ; 1,61 (m, 2H) ; 2,11 (m, 2H) ; 2,74 (m, 2H) ; 2,85 (s, 3H) ; 3,59 (m, 2H) ; 3,68 (t, J = 8,0 Hz, 2H) ; 4,46 (m, 1H) ; 5,69 (s, 2H) ; 6,70 (d, J = 8,0 Hz, 1H) ; 7,34 (t, J = 8,0 Hz, 2H) ; 7,68 (m, 1H) ; 7,97 (s large, 1H) ; 8,14 (dd, J = 1,5 et 8,5 Hz, 1H) ; 8,48 (d, J = 8,5 Hz, 1H).
Etape 13 : Dans un ballon de 250 mL, muni d'une agitation magnétique, d'un septum surmonté d'une arrivée d'argon, on introduit 1 g de 5-(2,6-difluorophényl)-3-{[1-(méthylsulfonyl)pipéridin-4-yl]amino}-1-{[2-(triméthylsilyl)éthoxy]méthyl}-1H-pyrazolo[4,3-c]isoquinoléine-7-carbonitrile dans 96 mL de DCM. Après refroidissement vers 0°C à l'aide d'un bain de glace, on ajoute 10,7 mL de TFA et agite à TA pendant 20 h. Le mélange réactionnel est concentré sous PR et le solide obtenu est repris dans 100 mL d'eau et extrait avec trois fois 100 mL d'AcOEt, sèche sur MgS0₄, filtre et concentre sous PR. On obtient ainsi, 992 mg de trifluoroacétate de 5-(2,6-difluorophényl)-3-{[1-(méthylsulfonyl)pipéridin-4-yl]amino}-1H-pyrazolo[4,3-c]isoquinoléine-7-carbonitrile, utilisé tel quel dans l'étape suivante. SM (E/1) : m/z =482 (M+)
Etape 14 : Dans un ballon de 250 mL, muni d'une agitation magnétique, d'un septum surmonté d'une arrivée d'argon, on introduit 992 mg de trifluoroacétate de 5-(2,6-difluorophényl)-3-{[1-(méthylsu lfonyl)pipéridin-4-yl]amino }-1 H-pyrazolo[4,3-c]isoquinoléine-7-carbonitrile, 63 mL d'éthanol, 30 mL de THF et 10 mL d'hydroxyde de sodium 9,3N. Après 3 h à reflux, on refroidit le mélange et verse dans 250 mL d'une solution aqueuse 1M de potassium dihydrogène phosphate. On extrait à l'AcOEt, lave avec une solution aqueuse saturée en NaCl, sèche sur MgSO₄, filtre et concentre à sec sous PR. Le solide brut est dissous dans 20 mL de DMF puis sont ajoutés 1 g de benzotriazol-1-yloxytris(diméthylamino)phosphonium hexafluor (BOP), 323 mg 1-hydroxybenzotriazole (HOBT), 170 mg de chlorure d'ammonium et 1 mL de N,N-diisopropyléthylamine (DIPEA). Après 2 h d'agitation à TA, on verse le mélange réactionnel dans de l'eau, extrait à l'AcOEt, lave avec une solution aqueuse 1 M de potassium dihydrogène phosphate puis avec une solution aqueuse saturée en NaCl. Après séchage sur MgSO₄, filtration et concentration sous PR, on purifie par flash-chromatographie sur gel de silice (40-63 µm), en éluant par un mélange de DCM, de méthanol et d'ammoniac méthanolique 4N (90/10/1 vol), on obtient ainsi 321 mg de 5-(2,6-difluorophényl)-3-{[1-(méthylsulfonyl)pipéridin-4-yl]amino}-1H-pyrazolo[4,3-c]isoquinoléine-7-carboxamide (solide jaune citron). SM (E/I) : m/z = 500 (M+) ; RMN ¹H : 1,68 (m, 2H) ; 2,11 (m, 2H) ; 2,88 (s, 3H) ; 2,90 (m, 2H) ; 3,56 (m, 2H) ; 3,80 (m, 1H) ; 6,09 (d, J = 8,0 Hz, 1H) ; 7,33 (t, J = 8,0 Hz, 2H) ; 7,57 (s large, 1 H) ; 7,69 (m, 1 H) ; 8,17 (s large, 1 H) ; 8,23 (s large, 1H) ; 8,36 (d large, J = 8,5 Hz, 1 H) ; 8,45 (d, J = 8,5 Hz, 1 H) ; 12,85 (s, 1 H).

### Exemple 2 5-(2,6-difluorophényl)-3-{[1-(méthylsulfonyl)pipéridin-4-yl]amino}-1H-pyrazolo[4,3-c]isoquinoléine-7-carbaldéhyde oxime.

Etape 1 : Dans un ballon de 250 mL, muni d'un septum surmonté d'une arrivée d'argon, on introduit 2,4 g de 5-(2,6-difluorophényl)-7-iodo-1 H-pyrazolo[4,3-c]isoquinoléine préparé à l'étape 6 de l'exemple 1 dans 80 mL de DMF, 0,80 mL de brome et 1,4 g de KOH. Après 1 h à TA, on verse le mélange sur de l'eau glacée. La phase organique est extraite avec 3 fois 150 mL d'AcOEt. Les extraits organiques sont regroupés, lavés avec une solution saturée en NaCl, séchés sur MgSO₄ et évaporés sous PR. On obtient 2,23 g de 3-bromo-5-(2,6-difluorophényl)-7-iodo-1H-pyrazolo[4,3-c]isoquinoléine (solide crème), utilisé tel quel pour l'étape suivante. SM (E/I) : m/z = 486 (M+)
Etape 2 : Dans un ballon de 250 mL, muni d'une agitation magnétique, d'un septum surmonté d'une arrivée d'argon, on introduit 2,22 g de 3-bromo-5-(2,6-difluorophényl)-7-iodo-1H-pyrazolo[4,3-c]isoquinoléine dans 11 mL de DMF. Après un barbotage de 10 min d'argon dans le milieu, on ajoute 1,47 mL de tributyl-vinyl-stannane et 481 mg de bis(triphénylphosphène palladium (II) dichloride. On chauffe 1 h le mélange à 100 °C puis après refroidissement, on évapore à sec sous vide. On obtient une huile que l'on reprend par 350 mL d'AcOEt et 300 mL d'une solution aqueuse à 20% de KF. On agite à TA pendant 30 min puis on filtre la suspension sur verre fritté muni d'un lit de Clarcel Flo. On extrait la phase organique à l'AcOEt puis on la lave avec une solution aqueuse saturée en NaCl, sèche sur sur MgSO₄ et évapore à sec sous PR. On obtient 3,1 g de produit brut que l'on purifie par chromatographie sur gel de silice (20-45 µm), en éluant par un mélange de DCM et de méthanol (95/5 vol), on obtient 1,44 g de 3-bromo-5-(2,6-difluorophényl)-7-vinyl-1H-pyrazolo[4,3-c]isoquinoléine (solide jaune clair). SM (E/I) : m/z = 386 (M+) ; RMN ¹H : 5,43 (d, J = 11,2 Hz, 1H) ; 6,00 (d, J = 17,6 Hz, 1H) ; 6,92 (dd, J=17,6, 11,2 Hz, 1H) ; 7,37 (t, J=7,9 Hz, 2H) ; 7,65 (s large, 1 H) ; 7,73 (m, 1 H) ; 8,33 (dd, J = 8,8, 1,5 Hz, 1 H) ; 8,51 (d, J = 8,8 Hz, 1 H) ; 14,59 (m étalé, 1 H).
Etape 3 : Dans un ballon de 150 mL, muni d'une agitation magnétique, d'un septum surmonté d'une arrivée d'argon, on introduit 1 g de 3-bromo-5-(2,6-difluorophényl)-7-vinyl-1H-pyrazolo[4,3-c]isoquinoléine dans 13 mL de THF puis on ajoute 7 mL d'eau, 13 mL de terbutanol, 0,79 mL d'une solution de tétroxyde d'osmium à 2,5% dans le 2-méthyl-2-propanol, et 1,7 g de periodate de sodium. On agite 3 h à TA puis le mélange est coulé dans de l'eau, extrait à l'AcOEt, lavé avec une solution saturée en NaCl, séché sur MgSO₄, filtré et concentré sous PR. On obtient 1,047 g de produit brut que l'on purifie par chromatographie sur gel de silice (20-45µm), en éluant par un mélange de DCM et de méthanol (95/5 vol), on obtient 686 mg de 3-bromo-5-(2,6-difluorophényl)-1H-pyrazolo[4,3-c]isoquinoléine-7-carbaldéhyde (solide crème). SM (E/I) : m/z = 388 (M+); RMN ¹H : 7,41 (d, J = 7,9 Hz, 2H) ; 7,77 (m, 1 H) ; 8,34 (s large, 1H) ; 8,47 (dd, J=8,5, 1,2 Hz, 1H) ; 8,69 (d, J = 8,5 Hz, 1 H) ; 10,15 (s, 1 H) ; 14,89 (m étalé, 1 H).
Etape 4 : Dans un ballon de 150 mL, muni d'une agitation magnétique, d'un septum surmonté d'une arrivée d'argon, on introduit 780 mg de 3-bromo-5-(2,6-difluorophényl)-1H-pyrazolo[4,3-c]isoquinoléine-7-carbaldéhyde dans 20 mL de DMF, 2,5 mL de diisopropyléthylamine et 0,75 mL de 2-(triméthylsilyl)éthoxyméthyl chloride. On agite 24 h le mélange à TA puis on coule dans de l'eau, extrait à l'AcOEt, lave avec une solution saturée en NaCl, sèche sur MgSO₄, filtre et concentre sous PR. On obtient 1,18 g de produit brut que l'on purifie par chromatographie sur gel de silice (20-45 µm), en éluant par un mélange de DCM et de méthanol (99,5/0,5 vol), on obtient d'une part 545 mg de 3-bromo-5-(2,6-difluorophényl)-1-(2-triméthylsilanyl-éthoxyméthyl)-1H-pyrazolo[4,3-c]isoquinoléine-7-carbaldéhyde sous forme de solide crème : SM (E/I) : m/z = 388 (M+) ; RMN ¹H : -0,13 (s, 9H) ; 0,86 (t, J=7,8 Hz, 2H) ; 3,66 (t, J=7,8 Hz, 2H) ; 6,18 (s, 2H) ; 7,43 (t, J = 7,9 Hz, 2H) ; 7,79 (m, 1 H) ; 8,39 (s large, 1 H) 8,48 (dd, J=8,3, 1,5 Hz, 1 H) 8,80 (d, J=8,3 Hz, 1H) 10,17 (s, 1H) et d'autre part 354 mg de 3-bromo-5-(2,6-difluoro-phényl)-2-(2-triméthylsilanyléthoxyméthyl)-2H-pyrazolo[4,3-c]isoquinoléine-7-carbaldéhyde (solide crème). SM (E/I) : m/z = 388 (M+) ; RMN ¹H : -0,08 (s, 9H) ; 0,90 (t, J=8,0 Hz, 2H) ; 3,73 (t, J=8,0 Hz, 2H) ; 5,91 (s, 2H) ; 7,41 (t, J = 7,8 Hz, 2H) ; 7,77 (m, 1 H) ; 8,22 (s large, 1 H) 8,42 (dd, J=8,3, 1,5 Hz, 1 H) 8,72 (d, J=8,3 Hz, 1H) 10,13 (s, 1H)
Etape 5 : Dans un ballon de 50 mL, muni d'une agitation magnétique, d'un septum surmonté d'une arrivée d'argon, on introduit 348,4 mg de 3-bromo-5-(2,6-difluorophényl)-2-(2-triméthylsilanyléthoxyméthyl)-2H-pyrazolo[4,3-c]isoquinoléine-7-carbaldéhyde dans 12,5 mL de 1-4 dioxanne. Après un barbotage de 10 min d'argon, on ajoute 320 mg de 1-méthanesulfonyl-pipéridin-4-ylamine, 1,1 g de carbonate de césium, 62 mg de 9,9-diméthyl-4,5-bis(diphénylphosphino)xanthène et 20 mg d'acétate de palladium (II). Le mélange est chauffé 4 h à reflux. Après refroidissement, le mélange est coulé dans de l'eau, extrait à l'AcOEt, lavé avec une solution saturée en NaCl, séché sur MgSO₄, filtré et concentré sous PR. On purifie par flash-chromatographie sur gel de silice (20-45 µm), en éluant par un mélange de DCM et d'AcOEt (85/15 vol), on obtient ainsi, 217 mg de 5-(2,6-difluorophényl)-3-{[1-(méthylsulfonyl)pipéridin-4-yl]amino}-1-{[2-(triméthylsilyl)éthoxy]méthyl}-1H-pyrazolo[4,3-c]isoquinoléine-7-carbaldéhyde (solide jaune). SM (E/I) : m/z =615 (M+) ; RMN ¹H : 0,05 (s, 9H) ; 0,89 (t, J=8,1 Hz, 2H) ; 1,62 (m, 2H) ; 2,12 (m, 2H) ; 2,75 (m, 2H) ; 2,85 (s, 3H) ; 3,59 (m, 2H) ; 3,67 (t, J=8,1 Hz, 2H) ; 4,46 (m, 1 H) ; 5,68 (s, 2H) ; 6,63 (d, J=8,3Hz, 1 H) ; 7,37 (t large, J=7,8Hz, 2H) ; 7,68 (m, 1 H) ; 8,06 (s large, 1 H) ; 8,25 (dd, J=8,4, 1,7Hz, 1H) ; 8,52 (d, J=8,4Hz, 1 H) ; 10,08 (s, 1 H).
Etape 6 : Dans un ballon de 100 mL, muni d'une agitation magnétique, d'un septum surmonté d'une arrivée d'argon, on introduit 211 mg de 5-(2,6-difluorophényl)-3-{[1-(méthylsulfonyl)pipéridin-4-yl]amino}-1-{[2-(triméthylsilyl)éthoxy]méthyl}-1H-pyrazolo[4,3-c]isoquinoléine-7-carbaldéhyde dans 8,5 mL de pyridine, on ajoute 36 mg de chlorhydrate d'hydroxylamine et on agite 64 h à TA. Le mélange est concentré sous PR et le solide obtenu est repris dans de l'AcOEt, lavé avec une solution aqueuse 1 M de phosphate de potassium puis avec une solution aqueuse saturée en NaCl, séché sur MgSO₄, filtré et concentré sous PR. On purifie par flash-chromatographie sur gel de silice (20-45 µm), en éluant par un mélange de DCM et d'AcOEt (75/25 vol), on obtient ainsi 184 mg de 5-(2,6-difluorophényl)-3-{[1-(méthylsulfonyl)pipéridin-4-yl]amino}-1-{[2-(triméthylsilyl)éthoxy]méthyl}-1H-pyrazolo[4,3-c]isoquinoléine-7-carbaldéhyde oxime (solide jaune). SM (E/I) : m/z =630 (M+) ; RMN ¹H : 0,04 (s, 9H) ; 0,89 (m, 2H) ; 1,61 (m, 2H) ; 2,11 (m, 2H) ; 2,74 (m, 2H) ; 2,85 (s, 3H) ; 3,58 (m, 2H) ; 3,67 (m, 2H) ; 4,46 (m, 1 H) ; 5,65 (s, 2H) ; 6,50 (d, J=8,3Hz, 1H) ; 7,33 (t large, J=8.1 Hz, 2H) ; 7,65 (m, 1H) ; 7,72 (s large, 1 H) ; 8,01 (dd, J=8,4, 1,7Hz, 1 H) ; 8,27 (s, 1 H) ; 8,35 (d, J=8,4 Hz, 1 H) ; 11,39 (s, 1 H).
Etape 7 : Dans un ballon de 50 mL muni d'un septum surmonté d'une arrivée d'argon, on introduit 180 mg de 5-(2,6-difluorophényl)-3-{[1-(méthylsulfonyl)pipéridin-4-yl]amino}-1-{[2-(triméthylsilyl)éthoxy]méthyl}-1H-pyrazolo[4,3-c]isoquinoléine-7-carbaldéhyde oxime dans 15 mL de DCM. Après refroidissement vers 0°C à l'aide d'un bain de glace, on ajoute 1,8 mL de TFA et agite 6 h à TA. Le mélange est concentré sous PR et le solide obtenu est repris dans 50 mL d'eau et 2 mL d'une solution aqueuse 0,75 M d'ammoniaque. On extrait à l'AcOEt, lave avec une solution saturée en NaCl, sèche sur MgSO₄, filtre et concentre sous PR. Après chromatographie sur gel de silice (20-45 µm), en éluant par un mélange de DCM et de méthanol (92,5/7,5 vol), on obtient 63 mg de 5-(2,6-difluorophényl)-3-{[1-(méthylsulfonyl)pipéridin-4-yl]amino}-1H-pyrazolo[4,3-c]isoquinoléine-7-carbaldéhyde oxime (solide jaune citron). SM (E/I) : m/z = 500 (M+) ; RMN ¹H : 1,67 (m, 2H) ; 2,11 (m, 2H) ; 2,87 (s, 3H) ; 2,89 (m, 2H) ; 3,56 (m, 2H) ; 3,79 (m, 1H) ; 6,06 (d, J = 7,8 Hz, 1 H) ; 7,32 (t large, J = 7,6 Hz, 2H) ; 7,67 (m, 1 H) ; 7,84 (s large, 1 H) ; 8,14 (d large, J=8,5 Hz, 1 H) ; 8,30 (s, 1 H) ; 8,41 (d, J=8,5 Hz, 1 H) ; 11,43 (s, 1 H); 12,81 (s, 1 H).

### Exemple 3 [5-(2,6-difluorophényl)-3-{[1-(méthylsulfonyl)pipéridin-4-yl]amino}-1-pyrazolo[4,3-c]isoquinoléin-7-yl]-méthanol

Etape 1 : Dans un ballon de 250 mL, muni d'une agitation magnétique, d'un septum surmonté d'une arrivée d'argon, on introduit 45 mg de 5-(2,6-difluorophényl)-3-{[1-(méthylsulfonyl)pipéridin-4-yl]amino}-1-{[2-(triméthylsilyl)éthoxy]méthyl}-1 H-pyrazolo[4,3-c]isoquinoléine-7-carbaldéhyde préparé à l'étape 5 de l'ex.2 dans 1,5 mL de THF et 1,5 mL de méthanol. Après refroidissement vers 0°C à l'aide d'un bain de glace, on ajoute 5,5 mg de NaBH₄ et on agite 30 min. On ajoute alors 1,5 mL d'acétone puis on coule le mélange dans une solution aqueuse saturée de bicarbonate de sodium. On extrait à l'AcOEt, lave avec une solution saturée en NaCl, sèche sur MgSO₄, filtre et concentre sous PR. On obtient 46 mg de [5-(2,6-difluorophényl)-3-{[1-(méthylsulfonyl)pipéridin-4-yl]amino}-1-{[2-(triméthylsilyl)éthoxy]méthyl}-1H-pyrazolo[4,3-c]isoquinoléin-7-yl]-méthanol utilisé tel quel pour l'étape suivante. SM (E/I) : m/z = 617 (M+)
Etape 2 : Dans un ballon de 30 mL, muni d'une agitation magnétique, d'un septum surmonté d'une arrivée d'argon, on introduit 46 mg de [5-(2,6-difluorophényl)-3-{[1-(méthylsulfonyl)pipéridin-4-yl]amino}-1-{[2-(triméthylsilyl)éthoxy]méthyl}-1H-pyrazolo[4,3-c]isoquinoléin-7-yl]-méthanol dans 4 mL de DCM. Après refroidissement vers 0°C à l'aide d'un bain de glace, on ajoute 0,45 mL de TFA et agite 2 h à TA. Le mélange est concentré sous PR et le solide obtenu est repris dans 25 mL d'eau et 0,75 mL d'une solution aqueuse 0,75 M d'ammoniaque. On extrait à l'AcOEt, lave avec une solution saturée en NaCl, sèche sur MgSO₄, filtre et concentre sous PR. Après chromatographie sur gel de silice (20-45 µm), en éluant par un mélange de DCM et de méthanol (92,5/7,5 vol), on obtient 13 mg de [5-(2,6-difluorophényl)-3-{[1-(méthylsulfonyl)pipéridin-4-yl]amino}-1 H-pyrazolo[4,3-c]isoquinoléin-7-yl]-méthanol (solide jaune). SM (E/I) : m/z = 487 (M+) ; RMN ¹H : 1,67 (m, 2H) ; 2,11 (m, 2H) ; 2,87 (s, 3H) ; 2,90 (m, 2H) ; 3,56 (m, 2H) ; 3,79 (m, 1H) ; 4,63 (d, J=5,7Hz, 2H) ; 5,34 (t, J=5,7Hz, 1 H) ; 5,98 (d, J=7,8Hz, 1 H) ; 7,31 (m, 2H) ; 7,59 (s large, 1 H) ; 7,66 (m, 1 H) ; 7,83 (d large, J=8,3 Hz, 1 H) ; 8,35 (d, J=8,3, 1H); 12,68 (s, 1 H).

### Exemple 4 5-(2,6-difluoro-phényl)-3-{[1-(trifluorométhanesulfonyl)pipéridin-4-yl]amino}-1H-pyrazolo[4,3-c]isoquinoléine-7-carboxamide

Etape 1 : on opère comme dans l'exemple 1, étape 12. Dans un ballon de 50 mL, muni d'une agitation magnétique et d'un septum surmonté d'une arrivée d'argon, on introduit 200 mg de 3-bromo-5-(2,6-difluorophényl)-1-{[2-(triméthylsilyl)éthoxy]méthyl}-1H-pyrazolo[4,3-c]isoquinoléine-7-carbonitrile dans 4 mL de 1-4-dioxane. Après barbotage de 10 min d'argon, on ajoute 269 mg de trifluoroacétate de 1-trifluorométhanesulfonyl-pipéridin-4-ylamine, 543 mg de carbonate de césium, 22 mg de 9,9-diméthyl-4,5-bis(diphénylphosphino)xanthène et 8,7 mg d'acétate de palladium (II). Le mélange est chauffé 2 h à reflux. Après refroidissement, le mélange est coulé dans de l'eau, extrait à l'AcOEt, lavé avec une solution saturée en NaCl, séché sur MgSO₄, filtré et concentré sous PR. On purifie par flash-chromatographie sur gel de silice (15-40 µm), en éluant par un mélange d'AcOEt et d'heptane (40/60 vol), on obtient ainsi, 272 mg de 5-(2,6-difluoro-phényl)-3-{[1-(trifluorométhanesulfonyl)pipéridin-4-yl]amino}-1-{[2-(triméthylsilyl)éthoxy]méthyl}-1 H-pyrazolo[4,3-c]isoquinoléine-7-carbonitrile (solide jaune pâle). SM (E/I) : m/z = 666 (M+)
   **Synthèse de (1-trifluorométhanesulfonyl-pipéridin-4-yl)-carbamate de 1,1-diméthyléthyle**
   Dans un ballon de 150 mL, muni d'une agitation magnétique, on introduit sous argon, 2 g de 4-N-boc-aminopipéridine dans 35 mL de DCM, 82 mg de 4-diméthylaminopyridine (DMAP) et 1,86 mL de triéthylamine. Après refroidissement vers 0°C à l'aide d'un bain de glace, on ajoute goutte à goutte, 1,6 mL de chlorure de trifluorométhanesulfonyle. On agite 30 min à 0°C puis 1 h à TA. Le mélange est versé dans 100 mL d'une solution aqueuse saturée de bicarbonate de sodium. On extrait à l'AcOEt, lave avec une solution aqueuse saturée en NaCl, sèche sur MgSO₄, filtre et concentre à sec sous PR. Après chromatographie sur gel de silice (15-40 µm), en éluant par un mélange de DCM et de méthanol (98/02 vol), on obtient 1,5 g de (1-trifluorométhanesulfonylpipéridin-4-yl)-carbamate de 1,1-diméthyléthyle (solide crème). SM (E/I) : m/z = 232 (M+).
   Dans un ballon de 150 mL, on introduit 1,37 g de (1-trifluorométhanesulfonyl-pipéridin-4-yl)-carbamate de 1,1-diméthyléthyle dans 30 mL de DCM. On ajoute 3 mL de TFA et on agite à TA pendant 1 h. Le mélange est concentré sous PR et le solide obtenu est repris dans 30 mL d'eau et 2 mL d'une solution aqueuse 0,75M d'ammoniaque. On extrait à l'AcOEt, sèche sur MgSO₄, filtre et concentre sous PR. On obtient 1,2 g de trifluoroacétate de 1-trifluorométhanesulfonyl-pipéridin-4-ylamine (solide blanchâtre). SM (E/I) : m/z = 346 (M+)
Etape 2: Dans un ballon de 30 mL, muni d'une agitation magnétique, d'un septum surmonté d'une arrivée d'argon, on introduit 272 mg de 5-(2,6-difluoro-phényl)-3-{[1-(trifluorométhanesulfonyl)pipéridin-4-yl]amino}-1-{[2-(triméthylsilyl)éthoxy]méthyl}-1 H-pyrazolo[4,3-c]isoquinoléine-7-carbonitrile, 10 mL d'éthanol, 5 ml de THF et 0,611 mL de NaOH 9,3N. Après 2 h de reflux, on refroidit le mélange et on verse 50 mL d'une solution aqueuse 1M de potassium dihydrogène phosphate. On extrait à l'AcOEt, lave avec une solution aqueuse saturée en NaCl, sèche sur MgSO₄, filtre et concentre à sec sous PR. Le solide brut obtenu est dissous dans 4 mL de DMF puis sont ajoutés 275 mg de benzotriazol-1-yloxytris(diméthylamino)phosphonium hexafluor (BOP), 84 mg 1-hydroxybenzotriazole (HOBT), 44 mg de chlorure d'ammonium et 2 mL de N,N-diisopropyléthylamine (DIPEA). Après 3 h à TA, on verse le mélange dans de l'eau, extrait à l'AcOEt, on lave avec une solution aqueuse saturée en NaCl. Après séchage sur MgSO₄, filtration et concentration sous PR, on obtient 300 mg de 5-(2,6-difluoro-phényl)-3-{[1-(trifluorométhanesulfonyl)pipéridin-4-yl]amino}-1-{[2-(triméthylsilyl)éthoxy]méthyl}-1 H-pyrazolo[4,3-c]isoquinoléine-7-carboxamide. SM (E/I) : m/z = 684 (M+).
Etape 3: Dans un ballon de 50 mL, muni d'une agitation magnétique, d'un septum surmonté d'une arrivée d'argon, on introduit 300 mg de 5-(2,6-difluoro-phényl)-3-{[1-(trifluorométhanesulfonyl)pipéridin-4-yl]amino}-1-{[2-(triméthylsilyl)éthoxy]méthyl}-1H-pyrazolo[4,3-c]isoquinoléine-7-carboxamide dans 8 mL de DCM. On ajoute 3 mL de TFA et on agite 2 h à TA. Le mélange est concentré sous PR et le solide obtenu est repris dans 30 mL d'eau et 2 mL d'une solution aqueuse 0,75 M d'ammoniaque. On extrait à l'AcOEt, sèche sur MgSO₄, filtre et concentre sous PR. Après chromatographie sur gel de silice (15-40 µm), en éluant par de l'AcOEt, on obtient 30 mg 5-(2,6-difluoro-phényl)-3-{[1-(trifluorométhanesulfonyl)pipéridin-4-yl]amino}-1H-pyrazolo[4,3-c]isoquinoléine-7-carboxamide (solide jaune pâle). SM (E/I) : m/z = 554 (M+) ; RMN ¹H : 1,69 (m, 2H) ; 2,17 (m, 2H) ; 3,38 (m, 2H) ; 3,83 (m, 2H) ; 3,93 (m, 1H) ; 6,24 (m, 1H) ; 7,33 (t, J = 8,0 Hz, 2H) ; 7,58 (s large, 1 H) ; 7,69 (m, 1 H) ; 8,17 (s large, 1 H) ; 8,23 (s large, 1 H) ; 8,36 (d large, J = 8,5 Hz, 1 H) ; 8,44 (d, J = 8,5 Hz, 1 H) ; 12,9 (s large, 1 H).

### Exemple 5 5-(2,6-difluorophényl)-3-{[1-(méthylsulfonyl)pipéridin-4-yl]amino}-7-vinyl-1H-pyrazolo[4,3-c]isoquinoléine

Etape 1 : Dans un ballon de 250 mL, muni d'une agitation magnétique, d'un septum surmonté d'une arrivée d'argon, on introduit 380 mg de 3-bromo-5-(2,6-difluorophényl)-7-vinyl-1H-pyrazolo[4,3-c]isoquinoléine tel que préparé à l'étape 2 de l'ex.2 dans 10 mL de DMF, 1,2 mL de diisopropyléthylamine et 0,37 mL de 2-(triméthylsilyl)éthoxyméthyl chloride. On agite 24 h le mélange à TA puis on coule dans de l'eau, extrait à l'AcOEt, lave avec une solution saturée en NaCl, sèche sur MgSO₄, filtre et concentre sous PR. On obtient 548 mg de produit brut que l'on purifie par chromatographie sur gel de silice (20-45 µm), en éluant par un mélange de DCM et de méthanol (99,5/0,5 vol), on obtient d'une part 198 mg de 3-bromo-5-(2,6-difluorophényl)-1-(2-triméthylsilanyl-éthoxyméthyl)-7-vinyl-1 H-pyrazolo[4,3-c]isoquinoléine sous forme d'un solide crème et d'autre part 194 mg de 3-bromo-5-(2,6-difluorophényl)-2-(2-triméthylsilanyl-éthoxyméthyl)-7-vinyl-2H-pyrazolo[4,3-c]isoquinoléine (solide crème). SM (E/I) : m/z = 516 (M+) ; RMN ¹H : 0,05 (s, 9H) ; 0,92 (t, J=7,8 Hz, 2H) ; 3,73 (t, J=7,8 Hz, 2H) ; 5,39 (d, J=11,1 Hz, 1 H) ; 5,87 (s, 2H) ; 5,94 (d, J=17,7 Hz, 1 H) 6,90 (dd, J=17,7, 11,1 Hz, 1 H) 7,39 (t, J=7,8 Hz, 2 H) 7,57 (s large, 1 H) 7,73 (m, 1 H) 8,21 (dd, J=8,5, 1,5 Hz, 1 H) 8,51 (d, J=8,5 Hz, 1 H).
Etape 2 : Dans un ballon de 30 mL, muni d'une agitation magnétique et d'un septum surmonté d'une arrivée d'argon, on introduit 189 mg de 3-bromo-5-(2,6-difluorophényl)-2-(2-triméthylsilanyléthoxyméthyl)-7-vinyl-2H-pyrazolo[4,3-c]isoquinoléine dans 5 mL de 1-4-dioxane. Après un barbotage de 10 min d'argon, on ajoute 130 mg de 1-méthanesulfonyl-pipéridin-4-ylamine, 453 mg de carbonate de césium, 25 mg de 9,9-diméthyl-4,5-bis(diphénylphosphino)xanthène et 8 mg d'acétate de palladium (II). Le mélange est chauffé 4 h à reflux. Après refroidissement, le mélange est coulé dans de l'eau, extrait à l'AcOEt, lavé avec une solution saturée en NaCl, séché sur MgSO₄, filtré et concentré sous PR. On purifie par flash-chromatographie sur gel de silice (15-40 µm), en éluant par un mélange de DCM et d'AcOEt (85/15 vol), on obtient ainsi, 96 mg de 5-(2,6-difluorophényl)-3-{[1-(méthylsulfonyl)pipéridin-4-yl]amino}-2-{[2-(triméthylsilyl)éthoxy]méthyl}-7-vinyl-2H-pyrazolo[4,3-c]isoquinoléine (solide jaune). SM (E/I) : m/z =613 (M+) ; RMN ¹H : 0,04 (s, 9 H) 0,89 (t, J=8,3 Hz, 2 H) 1,60 (m, 2 H) 2,10 (m, 2H) 2,74 (m, 2 H) 2,84 (s, 3 H) 3,58 (m, 2 H) 3,66 (t, J=8,3 Hz, 2 H) 4,44 (m, 1 H) 5,33 (d, J=11,0 Hz, 1 H) 5,64 (s, 2 H) 5,87 (d, J=17,7 Hz, 1 H) 6,47 (d, J=7,8 Hz, 1 H) 6,83 (dd, J=17,7, 11,0 Hz, 1 H) 7,33 (t, J=7,8 Hz, 2 H) 7,42 (s large, 1 H) 7,64 (m, 1 H) 8,02 (dd, J=8,3, 1,5 Hz, 1 H) 8,31 (d, J=8,3 Hz, 1 H)
Etape 3 : Dans un ballon de 30 mL, muni d'une agitation magnétique, d'un septum surmonté d'une arrivée d'argon, on introduit 92 mg de 5-(2,6-difluorophényl)-3-{[1-(méthylsulfonyl)pipéridin-4-yl]aminol-2-{[2-(triméthylsilyl)éthoxy]méthyl}-7-vinyl-2H-pyrazolo[4,3-c]isoquinoléine dans 8 mL de DCM. Après refroidissement vers 0°C à l'aide d'un bain de glace, on ajoute 0,9 mL de TFA et agite 2 h à TA. Le mélange est concentré sous PR et le solide obtenu est repris dans 30 mL d'eau et 1,5 mL d'une solution aqueuse 0,75 M d'ammoniaque. On extrait à l'AcOEt, sèche sur MgSO₄, filtre et concentre sous PR. On purifie par flash-chromatographie sur gel de silice (15-40 µm), en éluant par un mélange DCM/méthanol (95/05 vol). On obtient ainsi, 14 mg de 5-(2,6-difluorophényl)-3-{[1-(méthylsulfonyl)pipéridin-4-yl]amino}-7-vinyl-2H-pyrazolo[4,3-c]isoquinoléine (solide jaune). SM (E/I) : m/z =483 (M+) ; RMN ¹H : 1,67 (m, 2 H) 2,11 (m, 2 H) 2,87 (s, 3 H) 2,90 (m, 2 H) 3,56 (m, 2 H) 3,79 (m, 1 H) 5,37 (d, J=11,2 Hz, 1 H) 5,94 (d, J=17,6 Hz, 1 H) 6,01 (d large, J=8,3 Hz, 1 H) 6,87 (dd, J=17,6, 11,2 Hz, 1 H) 7,32 (t, J=7,8 Hz, 2 H) 7,53 (s large, 1 H) 7,67 (m, 1 H) 8,17 (d large, J=8,8 Hz, 1 H) 8,39 (d, J=8,8 Hz, 1 H) 12,73 (s, 1 H).

### Exemple 6 5-(2,6-difluorophényl)-7-fluoro-3-{[1-(méthylsulfonyl)pipéridin-4-yl]amino}-1H-pyrazolo[4,3-c]isoquinoléine

Etape 1 : Dans un ballon de 50 mL muni d'une agitation magnétique et d'un septum surmonté d'une arrivée d'argon, on introduit 4,2 g de 2-bromo-1-(4-fluorophényl)-éthanone dans 10 mL de DMF et 3,6 g de phtalimide de potassium. Le milieu est agité 4 h à TA et le précipité formé est filtré sur verre fritté, lavé avec 3 fois 50 mL d'éther isopropylique. On obtient ainsi, après séchage sous PR, 5 g de 2-[2-(4-fluorophényl)-2-oxoéthyl]-isoindole-1,3-dione (solide blanc) utilisé tel quel à l'étape suivante. Point de fusion (Kofler) : 140°C ; SM (E/I) : m/z = 283 (M+) ; RMN'H : 5,25 (s, 2 H) 7,44 (t, *J*=9,0 Hz, 2 H) 7,85 - 8,00 (m, 4 H) 8,19 (dd, *J*=9,8, 5,4 Hz, 2H)
Etape 2 : Dans un ballon de 100 mL muni d'un septum surmonté d'une arrivée d'argon et d'une agitation magnétique, on introduit 5 g de 2-[2-(4-fluorophényl)-2-oxoéthyl]-isoindole-1,3-dione dans 25 mL de N,N-diméthyformamide diéthyl acétal. Le mélange est porté 15 h au reflux puis refroidi à l'aide d'un bain de glace vers 0°C. Le précipité formé est essoré sur verre fritté, lavé par deux fois 50 mL d'éther isopropylique, puis on effectue 2 lavages par clairçage (lavage du gâteau sans agitation) avec 50 mL d'éther isopropylique. Après séchage sous PR, on obtient 4,5 g de mélange des isomères E et Z de 2-[2-diméthylamino-1-(4-fluorobenzoyl)-vinyl]-isoindole-1,3-dione (solide jaune orangé). PF(Kofler) : 228°C ; SM (E/I) : m/z = 338 (M+) ; RMN ¹H : 2,94 (m étalé, 6H) 7,25 (t, *J*=9,0 Hz, 2 H) 7,46 - 7,57 (m, 3 H) 7,86 -8,00 (m, 4H)
Etape 3: Dans un ballon d'un L muni d'une bonne agitation magnétique et d'un réfrigérant surmonté d'une arrivée d'argon, on introduit à TA, 4,5 g de 2-[2-diméthylamino-1-(4-fluorobenzoyl)-vinyl]-isoindole-1,3-dione dans 30 mL d'éthanol. On obtient une suspension brune à laquelle on additionne, 1,62 mL d'hydrate d'hydrazine dans 20 mL d'éthanol. La suspension est portée à reflux. Après 30 min de chauffage, on peut observer un épaississement du mélange qui prend une couleur jaune citron. Cette suspension est agitée au reflux pendant 3 h puis le mélange est ramené à TA. On filtre l'insoluble sur fritté et on lave avec 2x20 mL d'éther isopropylique. On concentre le filtrat à sec et on obtient 6 g d'un solide beige. On ajoute au mélange brut 50 mL d'eau et 20 mL d'acide chlorhydrique 2M. On ajoute 50 mL d'AcOEt et on filtre l'insoluble. On sépare la phase aqueuse et on la reprend par 3x15 mL d'AcOEt. On basifie la phase aqueuse par ajout de soude 2M jusqu'à pH=11 puis on extrait par 3x20 mL d'acétate d'éyhyle. On lave la phase organique avec de l'eau jusqu'à obtenir un pH neutre. On extrait à l'AcOEt, sèche sur MgSO₄, filtre et concentre à sec. On obtient ainsi 1,93 g de 3-(4-fluorophényl)-1H-pyrazol-4-ylamine (huile violacée). SM (E/I) : m/z = 177 (M+) ; RMN ¹H : Pour ce lot, on observe un mélange de tautomères et toutes les absorptions sont larges avec : 3,69 - 4,15 (m, 2 H) 7,09 - 7,34 (m, 3 H) 7,51 - 8,08 (m, 2 H) 12,10 - 12,50 (m, 1 H).
Etape 4 : Dans un ballon, muni d'une agitation magnétique et d'un septum surmonté d'une arrivée d'argon, on introduit, 1,93 g de 3-(4-fluorophényl)-1H-pyrazol-4-ylamine dans 20 mL de DCM et 15 mL de pyridine. Le mélange est refroidi à 0°C à l'aide d'un bain de glace et 1,7 mL du chlorure de l'acide 2,6-difluorobenzoïque sont additionnés. On laisse remonter progressivement la température à l'ambiante sur 3 h. Après évaporation du solvant sous PR, on ajoute de l'eau sur le brut réactionnel puis la phase aqueuse est extraite à l'AcOEt. Les extraits organiques sont regroupés, lavés avec une solution saturée en NaCl, séchés sur MgSO₄. On évapore alors l'AcOEt sous vide et obtient ainsi 3 g d'un mélange de 2,6-difluoro-N-[5-(4-fluorophényl)-1H-pyrazol-4-yl]-benzamide et de N-[1-(2,6-difluoro-benzoyl)-5-(4-fluorophényl)-1H-pyrazol-4-yl]-2,6-difluoro-benzamide sous forme d'un solide beige qui est utilisé tel quel. Le brut réactionnel est traité de façon à obtenir uniquement le produit attendu: dans un ballon, on dissout à TA et sous argon, 3 g du mélange de 6-difluoro-N-[5-(4-fluorophényl)-1H-pyrazol-4-yl]-benzamide et de N-[1-(2,6-difluoro-benzoyl)-5-(4-fluorophényl)-1H-pyrazol-4-yl]-2,6-difluoro-benzamide dans 20 mL de méthanol et 12 mL de tétrahydrofurane, puis on ajoute 6 mL de solution aqueuse de soude 5N. Après 1 h à TA, on coule le mélange dans 6 mL d'acide chlorhydrique 5N. On extrait à l'AcOEt, lave les phases organiques avec une solution saturée en NaCl, sèche sur MgSO₄ puis concentre sous PR. On obtient 2,6 g de 2,6-difluoro-N-[5-(4-fluorophényl)-1H-pyrazol-4-yl]-benzamide (solide crème). SM (E/I) : m/z = 317 (M+) ; RMN ¹H : 7,21 (t, J=7,8 Hz, 2 H) 7,26 (m large, 2 H) 7,56 (m, 1 H) 7,73 (m large, 2 H) 8,02 (m large, 1 H) 10,21 (s, 1 H) 12,95 - 13,29 (m étalé, 1 H) ; PF = 112°C.
Etape 5 : Dans un ballon de 250 mL, muni d'une agitation magnétique, d'un septum surmonté d'une arrivée d'argon, on introduit 2,6 g de 2,6-difluoro-N-[5-(4-fluorophényl)-1H-pyrazol-4-yl]-benzamide, 60 mL de dichlorure de phénylphosphine, et 17,5 g de pentoxyde de phosphore. Après 23 h de chauffage à 165°C, le mélange est refroidi à TA puis versé dans un mélange de 260 mL d'eau et de 400 g de glace. La température s'élève jusqu'à 40°C. On neutralise par addition lente de 120 mL d'ammoniaque aqueuse à 28%. On extrait à l'AcOEt, lave avec une solution saturée en NaCl et sèche sur MgSO₄, puis concentre sous PR. On purifie par flash-chromatographie sur gel de silice (15-40µm), en éluant par un mélange de DCM et de méthanol (99/1 vol), on obtient 680 mg de 5-(2,6-difluorophényl)-7-fluoro-1H-pyrazolo[4,3-c]isoquinoléine (solide crème). SM (E/I) : m/z = 299 (M+) ; RMN ¹H: 7,34 (t, J=7,8 Hz, 2 H) 7,38 (m large, 1 H) 7,70 (m, 1 H) 7,96 (t large, J=9,1 Hz, 1 H) 8,48 (s large, 1 H) 8,63 (dd, *J*=9,1, 5,6 Hz, 1 H) 14,29 (s large, 1 H).
Etape 6 : Dans un ballon de 250 mL, muni d'une agitation magnétique et d'un septum surmonté d'une arrivée d'argon, on introduit 680 mg de 5-(2,6-difluorophényl)-7-fluoro-1H-pyrazolo[4,3-c]isoquinoléine dans 10 mL de DMF, 0,21 mL de brome et 344 mg de KOH. Après 5 h à TA, on verse le mélange sur de l'eau glacée. La phase aqueuse est extraite avec 3x150 mL d'AcOEt. Les extraits organiques sont regroupés, lavés avec une solution saturée en NaCl, séchés sur MgSO₄ et évaporés sous PR. On obtient ainsi 770 mg de 3-bromo-5-(2,6-difluorophényl)-7-fluoro-1H-pyrazolo[4,3-c]isoquinoléine (solide jaune), utilisé tel quel pour l'étape suivante. SM (E/I) : m/z = 378 (M+) ; RMN ¹H : 7,38 (t, *J*=8,1 Hz, 2 H) 7,45 (d large, *J*=9,2 Hz, 1 H) 7,74 (m, 1 H) 8,03 (td large, *J*=9,2, 2,9 Hz, 1 H) 8,63 (dd, *J*=9,2, 5,4 Hz, 1 H) 14,69 (m étalé, 1 H).
Etape 7 : Dans un ballon de 50 mL, muni d'une agitation magnétique, d'un septum surmonté d'une arrivée d'argon, on introduit 770 mg de 3-bromo-5-(2,6-difluorophényl)-7-fluoro-1H-pyrazolo[4,3-c]isoquinoléine dans 15 mL de DMF, 2,5 mL de N,N-diisopropyléthylamine et 0,76 mL de 2-(triméthylsilyl)éthoxyméthyl chloride. Après 5 h à TA, le mélange est versé dans 8 volumes d'eau glacée. La phase aqueuse est extraite avec 3 fois 200 mL d'AcOEt, les extraits organiques sont regroupés, lavés avec une solution saturée en NaCl, séchés sur MgSO₄ et concentrés sous PR. On obtient 520 mg 3-bromo-5-(2,6-difluorophényl)-7-fluoro-1-{[2-(triméthylsilyl)éthoxy]méthyl}-1H-pyrazolo[4,3-c]isoquinoléine (solide orange). SM (E/I) : m/z =508 (M+) ; RMN ¹H : 0,12 (s, 9 H) 0,85 (t, *J*=7,9 Hz, 2 H) 3,64 (t, J=7,9 Hz, 2 H) 6,13 (s, 2 H) 7,39 (t, *J*=7,8 Hz, 2 H) 7,51 (dd, J=9,2, 2,7 Hz, 1 H) 7,75 (m, 1 H) 8,06 (td, J=9,2 ; 2,7 Hz, 1 H) 8,71 (dd, J=9,2,5,4 Hz, 1 H)
Etape 8 : Dans un ballon de 50 mL, muni d'une agitation magnétique et d'un septum surmonté d'une arrivée d'argon, on introduit 520 mg de 3-bromo-5-(2,6-difluorophényl)-7-fluoro-1-{[2-(triméthylsilyl)éthoxy]méthyl}-1H-pyrazolo[4,3-c]isoquinoléine dans 14 mL de 1-4-dioxane. Après un barbotage de 10 min d'argon, on ajoute 225 mg de 1-méthanesulfonyl-pipéridin-4-ylamine, 780 mg de carbonate de césium, 92 mg de 9,9-diméthyl-4,5-bis(diphénylphosphino)xanthène et 40 mg d'acétate de palladium (II). Le mélange est chauffé 5 h à reflux. Après refroidissement, le mélange est coulé dans de l'eau, extrait à l'AcOEt, lavé avec une solution saturée en NaCl, séché sur MgSO₄, filtré et concentré sous PR. On purifie par flash-chromatographie sur gel de silice (15-40µm), en éluant par un mélange de DCM et d'AcOEt (95/05 vol), on obtient 94 mg de 5-(2,6-difluorophényl)-7-fluoro-3-{[1-(méthylsulfonyl)pipéridin-4-yl]amino}1-{[2-(triméthylsilyl)éthoxy]méthyl}-1H-pyrazolo[4,3-c]isoquinoléine (solide jaune). SM (E/I) : m/z =605 (M+) ; RMN ¹H: -0,13 (s, 9 H) 0,84 (m, 2 H) 1,69 (m, 2 H) 2,10 (m, 2 H) 2,88 (s, 3 H) 2,90 (m partiellement masqué, 2 H) 3.57 (m, 2H) 3.62 (m, 2H) 3,77 (m, 1H) 5,89 (s, 2H) 6,28 (d, J=8,3 Hz, 1 H) 7,28 - 7,40 (m, 3H) 7,69 (m, 1 H) 7,91 (td, J=9,0, 2,9 Hz, 1 H) 8,60 (dd, J=9,0, 5,4 Hz, 1 H)
Etape 9 : Dans un ballon muni d'un septum surmonté d'une arrivée d'argon, on introduit 95 mg de 5-(2,6-difluorophényl)-7-fluoro-3-{[1-(méthylsulfonyl)pipéridin-4-yl]amino}1-{[2-(triméthylsilyl)éthoxy]méthyl}-1H-pyrazolo[4,3-c]isoquinoléine dans 10 mL de DCM. Après refroidissement vers 0°C à l'aide d'un bain de glace, on ajoute 0,173 mL de TFA et agite à TA pendant 20 h. Le mélange est concentré sous PR et le solide obtenu est repris dans 30 mL d'eau et 2 mL d'une solution aqueuse 0,75M d'ammoniaque. On extrait à l'AcOEt, sèche sur MgSO4, filtre et concentre sous PR. On purifie par flash-chromatographie sur gel de silice (15-40 µm), en éluant par un mélange de DCM et de méthanol (95/05 vol) On obtient ainsi, 6 mg de 5-(2,6-difluorophényl)-7-fluoro-3-{[1-(méthylsulfonyl)pipéridin-4-yl]amino}-1H-pyrazolo[4,3-c]isoquinoléine (solide jaune). SM (E/I) : m/z =475 (M+) ; RMN ¹H : 1,67 (m, 2 H) 2,10 (m, 2 H) 2,87 (s, 3 H) 2,90 (m, 2 H) 3,56 (m, 2 H) 3,79 (m, 1 H) 6,08 (d, *J*=8,3 Hz, 1 H) 7,29 (m, 1 H) 7,33 (t, *J*=7,8 Hz, 2 H) 7,68 (m, 1 H) 7,88 (t large, *J*=9,3 Hz, 1 H) 8,50 (dd, *J*=9,3, 5,4 Hz, 1 H) 12,80 (s, 1 H)*

### Exemple 7 5-(2,6-difluorophényl)-3-{[1-(méthylsulfonyl)pipéridin-4-yl]amino}-7-éthynyl -1H-pyrazolo[4,3-c]isoquinoléine

Etape 1 : Dans un ballon de 150 mL, muni d'une agitation magnétique, d'un septum surmonté d'une arrivée d'argon, on introduit 1,2 g de 3-bromo-5-(2,6-difluorophényl)-7-iodo-1H-pyrazolo[4,3-c]isoquinoléine préparé dans l'étape 1 de l'ex.6 dans 24 mL de DMF, 3 mL de diisopropyléthylamine et 0,92 mL de 2-(triméthylsilyl)éthoxyméthyl chloride. On agite le mélange 24 h à TA puis on coule dans de l'eau, extrait à l'AcOEt, lave avec une solution saturée en NaCl, sèche sur MgSO₄, filtre et concentre sous PR. On obtient 1,52 g de produit brut que l'on purifie par chromatographie sur gel de silice (20-45 µm), en éluant par un mélange DCM/méthanol (99,5/0,5 vol), on obtient d'une part 832 mg de 3-bromo-5-(2,6-difluorophényl)-1-(2-triméthylsilanyl éthoxyméthyl)-7-iodo-1H-pyrazolo[4,3-c]isoquinoléine sous forme d'un solide blanc. SM (E/I) : m/z = 616 (M+) et d'autre part 526 mg de 3-bromo-5-(2,6-difluorophényl)-2-(2-triméthylsilanyl-éthoxyméthyl)-7-iodo-2H-pyrazolo[4,3-c]isoquinoléine (huile ocre) utilisé tel quel dans l'étape suivante. SM (E/I) : m/z = 616 (M+).
Etape 2 : Dans un ballon de 10 mL, muni d'une agitation magnétique, d'un septum surmonté d'une arrivée d'argon, on introduit 263 mg de 3-bromo-5-(2,6-difluorophényl)-2-(2-triméthylsilanyl-éthoxyméthyl)-7-iodo-2H-pyrazolo[4,3-c]isoquinoléine dans 1,5 mL de DMF puis 1,5 mL de triéthylamine. Après un barbotage de 10 min d'argon dans le mélange, on ajoute 30 mg de bis(triphénylphosphène palladium (II) dichloride, 8,7 mg d'iodure de cuivre (I) et 118 µL d'éthynyl-triméthylsilane. On agite 2 h à TA, coule dans de l'eau, extrait à l'AcOEt, lave avec une solution saturée en NaCl, sèche sur MgSO4, filtre et concentre sous PR. On purifie par flash-chromatographie sur gel de silice (40-63 µm), en éluant par un mélange de DCM et de méthanol (99,5/0,5 vol), on obtient ainsi, 140 mg de 3-bromo-5-(2,6-difluorophényl)-2-(2-triméthylsilanyl-éthoxyméthyl)-7-triméthylsilanyléthynyl-2H-pyrazolo[4,3-c]isoquinoléine (solide crème). SM (E/I) : m/z = 586 (M+) RMN 1H : -0,05 (s, 9 H) 0,23 (s, 9 H) 0,89 (m, 2 H) 3,72 (m, 2 H) 5,88 (s, 2 H) 7,4 (t, J=8,1 Hz, 2H) 7,6 (s large, 1 H) 7,75 (m, 1 H) 8,01 (dd, J=8,3, 1,5Hz, 1 H) 8,52 (d, J=8,3 Hz, 1 H)
Etape 3 : Dans un ballon de 30 mL, muni d'une agitation magnétique et d'un septum surmonté d'une arrivée d'argon, on introduit 79 mg de 3-bromo-5-(2,6-difluorophényl)-2-(2-triméthylsilanyl-éthoxyméthyl)-7- triméthylsilanyléthynyl -2H-pyrazolo[4,3-c]isoquinoléine dans 5 mL de 1-4-dioxane. Après barbotage de 10 min d'argon dans le milieu réactionnel, on ajoute 81 mg de 1-méthanesulfonyl-pipéridin-4-ylamine, 282 mg de carbonate de césium, 16 mg de 9,9-diméthyl-4,5-bis(diphénylphosphino)xanthène et 5 mg d'acétate de palladium (II). Le mélange est chauffé 5 h à reflux. Après refroidissement, le mélange est coulé dans de l'eau, extrait à l'AcOEt, lavé avec une solution saturée en NaCl, séché sur MgSO₄, filtré et concentré sous PR. On purifie par flash-chromatographie sur gel de silice (40-63 µm), en éluant par un mélange DCM/méthanol (95/05 vol), on obtient 54 mg de 5-(2,6-difluorophényl)-3-{[1-(méthylsulfonyl)pipéridin-4-yl]amino}-2-{[2-(triméthylsilyl)éthoxy]méthyl}-7- triméthylsilanyléthynyl -2H-pyrazolo[4,3-c]isoquinoléine d'un solide jaune. SM (E/I) : m/z = 683 (M+)
Etape 4 : Dans un ballon de 30 mL, muni d'une agitation magnétique et d'un septum surmonté d'une arrivée d'argon, on introduit 74 mg de 5-(2,6-difluorophényl)-3-{[1-(méthylsulfonyl)pipéridin-4-yl]amino}-2-{[2-(triméthylsilyl)éthoxy]méthyl}-7- triméthylsilanyléthynyl -2H-pyrazolo[4,3-c]isoquinoléine dans 5 mL de THF, on ajoute 0,22 cm³ de fluorure de tétrabutylammonium et on agite 30 min à TA. Le mélange est coulé dans de l'eau, extrait à l'AcOEt, lavé avec une solution saturée en NaCl, séché sur MgSO₄, filtré et concentré sous PR. On purifie par flash-chromatographie sur gel de silice (40-63 µm), en éluant par un mélange de DCM et d'AcOEt (85/15 vol), on obtient 47 mg de 5-(2,6-difluorophényl)-3-{[1-(méthylsulfonyl)pipéridin-4-yl]amino}-2-{[2-(triméthylsilyl)éthoxy]méthyl}-7-éthynyl-2H-pyrazolo[4,3-c]isoquinoléine (solide jaune citron). SM (E/I) : m/z = 611 (M+)
Etape 5 : Dans un ballon de 30 mL muni d'un septum surmonté d'une arrivée d'argon, on introduit 47 mg de 5-(2,6-difluorophényl)-3-{[1-(méthylsulfonyl)pipéridin-4-yl]amino}-2-{[2-(triméthylsilyl)éthoxy]méthyl}-7-éthynyl-2H-pyrazolo[4,3-c]isoquinoléine dans 4 mL de DCM. Après refroidissement vers 0°C à l'aide d'un bain de glace, on ajoute 0,47 mL de TFA et agite 3 h à TA. Le mélange est concentré sous PR et le solide obtenu est repris dans 25 mL d'eau et 0,75 mL d'une solution aqueuse 0,75 M d'ammoniaque. On extrait à l'AcOEt, sèche sur MgSO₄, filtre et concentre sous PR. On purifie par flash-chromatographie sur gel de silice (15-40µm), en éluant par un mélange de DCM et de méthanol (92,5/7,5 vol). On obtient 6 mg de 5-(2,6-difluorophényl)-3-{[1-(méthylsulfonyl)pipéridin-4-yl]amino}-7-éthynyl-2H-pyrazolo[4,3-c]isoquinoléine (solide jaune). SM (E/I) : m/z = 481 (M+) ; RMN ¹H: 1,66 (m, 2 H) 2,11 (m, 2 H) 2,87 (s, 3 H) 2,91 (m, 2 H) 3,56 (m, 2 H) 3,79 (m, 1 H) 4,37 (s, 1 H) 6,10 (d, *J*=8,3 Hz, 1 H) 7,34 (t, *J*=7,9 Hz, 2 H) 7,65 (s large, 1 H) 7,69 (m, 1 H) 7,97 (d large, J=8,3 Hz, 1 H) 8,42 (d, J=8,3 Hz, 1 H) 12,88 (s large, 1 H).

### Exemple 8 5-(2,6-difluorophényl)-3-{[1-(méthylsulfonyl)pipéridin-4-yl]amino}-1H-pyrazolo[4,3-c]isoquinoléine-7-carboxylate de méthyle

Etape 1 : Dans un tricol de 250 mL, muni d'une agitation magnétique et d'une arrivée d'argon, on introduit 4 g de 5-(2,6-difluorophényl)-7-iodo-1H-pyrazolo[4,3-c]isoquinoléine préparé à l'étape 6 de l'ex. 1 dans 120 ml de THF et 3 ml de DMF. Le milieu est refroidi à 0°C à l'aide d'un bain de glace puis 952 mg d'hydrure de sodium (60%) sont additionnés. Après 15 min à 0°C, on additionne 1,3 ml de chlorure de diméthylsulfamoyle. Après 16 h à TA, le mélange est versé dans 300 ml d'eau et extrait à l'acétate d'éthyle. La phase organique est séchée sur MgSO₄, filtrée et concentrée sous PR. Le solide obtenu est trituré dans de l'éther diisopropylique. L'insoluble est filtré sous PR, séché sous vide pour conduire à 2,94 g de 5-(2,6-difluorophényl)-7-iodo-N,N-diméthyl-1H-pyrazolo[4,3-c]isoquinoléine-1-sulfonamide (solide beige). LC-MS-DAD-ELSD : 515(+)=(M+H)(+).
Etape 2 : Dans un ballon de 250 mL, muni d'une agitation magnétique, d'un septum surmonté d'une arrivée d'argon, on introduit 2,94 g de 5-(2,6-difluorophényl)-7-iodo-N,N-diméthyl-1H-pyrazolo[4,3-c]isoquinoléine-1-sulfonamide dans 90 mL de DMF. Après un barbotage de 10 min d'argon, on ajoute 1,81 g de tributyl-vinyl-stannane et 331 mg de triphénylphosphine palladium (0). On chauffe le mélange 3 h à reflux puis après refroidissement, on verse le mélange dans de l'eau et on extrait à l'acétate d'éthyle. La phase organique est séchée sur MgSO₄ et évaporée à sec sous PR. On obtient 2,36 g de 5-(2,6-difluorophényl)-7-éthènyl-N,N-diméthyl-1H-pyrazolo[4,3-c]isoquinoléine-1-sulfonamide (solide marron). Le produit est utilisé tel quel. LC-MS-DAD-ELSD : 415(+)=(M+H)(+).
Etape 3 : Dans un ballon de 250 mL, muni d'une agitation magnétique, d'un septum surmonté d'une arrivée d'argon, on introduit 2,36 g de 5-(2,6-difluorophényl)-7-éthènyl-N,N-diméthyl-1H-pyrazolo[4,3-c]isoquinoléine-1-sulfonamide dans 30 mL de THF puis on ajoute 23 mL d'eau, 30 mL de terbutanol, 2,14 mL d'une solution de tétroxyde d'osmium à 2,5% dans le 2-méthyl-2-propanol, et 5,04 g de periodate de sodium. On agite 4 h à TA puis le mélange est coulé dans 100 ml d'eau, extrait à l'AcOEt. La phase organique est lavée avec une solution saturée en NaCl, séchée sur MgSO₄, filtrée et concentrée sous PR. Le produit brut est purifié par chromatographie sur gel de silice (15-40 µm), en éluant par du DCM pour conduire à 1,92 g de 5-(2,6-difluorophényl)-7-formyl-N,N-diméthyl-1H-pyrazolo[4,3-c]isoquinoléine-1-sulfonamide (solide beige). LC-MS-DAD-ELSD : 417(+)=(M+H)(+).
Etape 4 : Dans un ballon de 250 ml, on introduit 1,41 g de 5-(2,6-difluorophényl)-7-formyl-N,N-diméthyl-1H-pyrazolo[4,3-c]isoquinoléine-1-sulfonamide dans 124 mL d'acétone. On ajoute 1,07 g de permanganate de potassium en solution dans 62 ml d'eau. Après 1 h à TA, l'acétone est évaporée puis le mélange acidifié à pH 1 par addition d'acide chlorhydrique 5N. Le mélange est extrait à l'acétate d'éthyle, la phase organique est lavée avec une solution saturée en NaCl, séchée sur MgSO₄, filtrée et concentrée sous PR pour conduire à 1,46 g de acide 5-(2,6-difluorophényl)-1-(diméthylsulfamoyl)-1H-pyrazolo[4,3-c]isoquinoléine-7-carboxylique (solide jaune). LC-MS-DAD-ELSD : 433(+)=(M+H)(+).
Etape 5 : Dans un ballon de 250 ml, on introduit 1.46 g de acide 5-(2,6-difluorophényl)-1-(diméthylsulfamoyl)-1H-pyrazolo[4,3-c]isoquinoléine-7-carboxylique dans 100 ml de DCM puis on ajoute 20 ml de TFA. On agite 16 h à TA. Le mélange est concentré sous PR et le solide obtenu est repris dans 30 mL d'une solution aqueuse saturée de NaHCO₃ et extrait avec trois fois 30 mL d'AcOEt. La phase aqueuse est acidifiée jusqu'à pH 1 par addition d'HCl 37% et extraite à l'AcOEt. La phase organique est lavée avec une solution saturée en NaCl, séchée sur MgSO₄, filtrée et concentrée sous PR pour conduire à 927 mg de acide 5-(2,6-difluorophényl)-2H-pyrazolo[4,3-c]isoquinoléine-7-carboxylique (solide beige). LC-MS-DAD-ELSD : 326(+)=(M+H)(+).
Etape 6 : Dans un ballon de 100 ml, on introduit 605 mg de acide 5-(2,6-difluorophényl)-2H-pyrazolo[4,3-c]isoquinoléine-7-carboxylique dans 20 ml de DMF. On ajoute 334 mg d'hydroxyde de potassium puis 595 mg de brome et on agite 1 h à TA. Le mélange est versé dans 100 ml d'eau et extrait à l'acétate d'éthyle. La phase organique est lavée avec une solution saturée en NaCl, séchée sur MgSO₄, filtrée et concentrée sous PR pour conduire à 1,2 g d'une huile orange que l'on purifie par flash-chromatographie sur gel de silice (15-40 µm), en éluant par un mélange de DCM/méthanol (80/20 vol) pour conduire à 562 mg de acide 3-bromo-5-(2,6-difluorophényl)-2H-pyrazolo[4,3-c]isoquinoléine-7-carboxylique (solide blanc). LC-MS-DAD-ELSD : 406(+)=(M+H)(+). Etape 7 : Dans un tricol de 25 mL, muni d'une agitation magnétique et d'une arrivée d'argon, on introduit 562 mg de acide 3-bromo-5-(2,6-difluorophényl)-2H-pyrazolo[4,3-c]isoquinoléine-7-carboxylique dans 11 ml de DMF, 1,38 ml de diisopropyléthylamine et 740 µl de 2-(triméthylsilyl)éthoxyméthyl chloride. Après 2 h à TA, le milieu réactionnel est versé dans 100 ml d'eau. La phase aqueuse est extraite avec 3 fois 100 mL d'AcOEt, les extraits organiques sont regroupés, lavés avec une solution saturée en NaCl, séchés sur MgSO₄ et concentrés sous PR. Le produit brut est purifié par flash-chromatographie sur gel de silice (15-40 µm), en éluant par un mélange cyclohexane/DCM (50/50 vol) puis par du DCM pour conduire à 502 mg d'un mélange d' acide 3-bromo-5-(2,6-difluorophényl)-2-{[2-(triméthylsilyl)éthoxy]méthyl}-2H-pyrazolo[4,3-c]isoquinoléine-7-carboxylique et d' acide 3-bromo-5-(2,6-difluorophényl)-1-{[2-(triméthylsilyl)éthoxy]méthyl}-1H-pyrazolo[4,3-c]isoquinoléine-7-carboxylique (huile jaune pâle). LC-MS-DAD-ELSD : 665(+)=(M+H)(+).
Etape 8: Dans un ballon de 25 ml, muni d'une agitation magnétique, on introduit 500 mg du mélange d'acide 3-bromo-5-(2,6-difluorophényl)-2-{[2-(triméthylsilyl)éthoxy]méthyl}-2H-pyrazolo[4,3-c]isoquinoléine-7-carboxylique et de acide 3-bromo-5-(2,6-difluorophényl)-1-{[2-(triméthylsilyl)éthoxy]méthyl}-1H-pyrazolo[4,3-c]isoquinoléine-7-carboxylique obtenu à l'étape 7 dans 10 ml de méthanol et 1 ml d'une solution 2N d'hydroxyde de sodium. Après 5 min à TA, le mélange est versé dans 10 ml d'AcOEt et 5 ml d'eau. La phase organique est lavée avec une solution aqueuse saturée en NaCl, séchée sur MgSO₄ et concentrée sous PR pour conduire à 336 mg d'un mélange de 3-bromo-5-(2,6-difluorophényl)-1-{[2-(triméthylsilyl)éthoxy]méthyl}-1H-pyrazolo[4,3-c]isoquinoléine-7-carboxylate de méthyle et de 3-bromo-5-(2,6-difluorophényl)-2-{[2-(triméthylsilyl)éthoxy]méthyl}-2H-pyrazolo[4,3-c]isoquinoléine-7-carboxylate de méthyle (solide beige). LC-MS-DAD-ELSD : 549(+)=(M+H)(+)
Etape 9 : Dans un tricol de 25 mL, muni d'une agitation magnétique et d'une arrivée d'argon, on introduit 113 mg du mélange de 3-bromo-5-(2,6-difluorophényl)-1-{[2-(triméthylsilyl)éthoxy]méthyl}-1H-pyrazolo[4,3-c]isoquinoléine-7-carboxylate de méthyle et de 3-bromo-5-(2,6-difluorophényl)-2-{[2-(triméthylsilyl)éthoxy]méthyl}-2H-pyrazolo[4,3-c]isoquinoléine-7-carboxylate de méthyle obtenu à l'étape 8 dans 3 mL de 1-4-dioxane. Après un barbotage de 10 min d'argon dans le mélange, on ajoute 37 mg de 1-méthanesulfonyl-pipéridin-4-ylamine, 255 mg de carbonate de césium, 18 mg de 9,9-diméthyl-4,5-bis(diphénylphosphino)xanthène et 5 mg d'acétate de palladium (II). Le mélange est chauffé à 110°C pendant 4 h. Après refroidissement, le mélange est coulé dans de l'eau, extrait à l'AcOEt. La phase organique est lavée avec une solution saturée en NaCl, séchée sur MgSO₄, filtrée et concentrée sous PR. On purifie par flash-chromatographie sur gel de silice (15-40 µm), en éluant par du DCM puis un mélange de DCM et d'AcOEt (80/20 vol) pour conduire à 46 mg d'un mélange de 5-(2,6-difluorophényl)-3-{[1-(méthylsulfonyl)pipéridin-4-yl]amino}-1-{[2-(triméthylsilyl)éthoxy]méthyl}-1H-pyrazolo[4,3-c]isoquinoléine-7-carboxylate de méthyle et de 5-(2,6-difluorophényl)-3-{[1-(méthylsulfonyl)pipéridin-4-yl]amino}-2-{[2-(triméthylsilyl)éthoxy]méthyl}-2H-pyrazolo[4,3-c]isoquinoléine-7-carboxylate de méthyle (solide jaune). LC-MS-DAD-ELSD : 646(+)=(M+H)(+).
Etape 10 : Dans un ballon de 25 mL, muni d'une agitation magnétique, on introduit 46 mg du mélange de 5-(2,6-difluorophényl)-3-{[1-(méthylsulfonyl)pipéridin-4-yl]amino}-1-{[2-(triméthylsilyl)éthoxy]méthyl}-1H-pyrazolo[4,3-c]isoquinoléine-7-carboxylate de méthyle et de 5-(2,6-difluorophényl)-3-{[1-(méthylsulfonyl)pipéridin-4-yl]amino}-2-{[2-(triméthylsilyl)éthoxy]méthyl}-2H-pyrazolo[4,3-c]isoquinoléine-7-carboxylate de méthyle obtenu à l'étape 9 dans 5 mL de DCM. Après refroidissement vers 0°C à l'aide d'un bain de glace, on ajoute 0,5 mL de TFA et on agite 18 h à TA. Le mélange est concentré sous PR et le solide obtenu est repris dans 20 mL d'eau et extrait avec trois fois 20 mL d'AcOEt. La phase organique est séchée sur MgSO₄, filtrée et concentrée sous PR. On purifie par flash-chromatographie sur gel de silice (15-40 µm), en éluant par du DCM puis un mélange de DCM/AcOEt (50/50 vol) pour conduire à 12 mg de 5-(2,6-difluorophényl)-3-{[1-(méthylsulfonyl)pipéridin-4-yl]amino}-1H-pyrazolo[4,3-c]isoquinoléine-7-carboxylate de méthyle (solide jaune). RMN ¹H (400 MHz, DMSO-d6) δ ppm 1,67 (m, 2 H) 2,11 (m, 2 H) 2,88 (s, 3 H) 2,92 (m, 2 H) 3,56 (m, 2 H) 3,80 (m, 1 H) 3,87 (s, 3 H) 6,16 (d, J=7,7 Hz, 1 H) 7,37 (m, 2 H) 7,71 (m, 1 H) 8,23 (s large, 1 H) 8,42 (d large, J=8,5 Hz, 1 H) 8,54 (d, J=8,5 Hz, 1 H) 13,01 (s, 1 H). LC-MS-DAD-ELSD : 516 (+)=(M+H)(+).

### Exemple 9: 5-(2,6-difluorophényl)-3-{[1-(méthylsulfonyl)pipéridin-4-yl]amino}-1H-pyrazolo[4,3-c]isoquinoléine-7-carbothioamide

Dans un tube micro-ondes de capacité 5 ml, on introduit 45 mg de 5-(2,6-difluorophényl)-3-{[1-(méthylsulfonyl)pipéridin-4-yl]amino}-1H-pyrazolo[4,3-c]isoquinoléine-7-carbonitrile préparé à l'exemple 12 et 0,2 ml d'une solution aqueuse à 20% de sulfide d'ammonium dans 2 ml de méthanol. Le mélange est chauffé au micro-ondes à 100°C pendant 20 min (puissance fixée à 100 Watts) puis coulé dans 50 ml d'eau, extrait à l'AcOEt. La phase organique est lavée avec une solution saturée en NaCl, séchée sur MgSO₄, filtrée et concentrée sous PR. Le solide obtenu est trituré dans de l'éther diisopropylique. L'insoluble est filtré sous PR, séché sous vide pour conduire à 36 mg de 5-(2,6-difluorophényl)-3-{[1-(méthylsulfonyl)pipéridin-4-yl]amino}-1H-pyrazolo[4,3-c]isoquinoléine-7-carbothioamide (solide jaune).1H NMR (400 MHz, DMSO-d6) δ ppm : 1,66 (m, 2 H) 2,11 (m, 2 H) 2,88 (s, 3 H) 2,90 (m, 2 H) 3,56 (m,2 H) 3,79 (m, 1 H) 6,10 (d, J=8,3 Hz, 1 H) 7,33 (t, J=7,8 Hz, 2 H) 7,68 (m, 1 H) 8,18 (s large, 1 H) 8,34(dd, J=8,3, 1,5 Hz, 1 H) 8,40 (d, J=8,3 Hz, 1 H) 9,74 (s large, 1 H) 10,01 (s large, 1 H) 12,87 (m étalé, 1H) LC-MS-DAD-ELSD : 517(+)=(M+H)(+).

### Exemple 10 5-(2,6-difluorophényl)-3-{[1-(méthylsulfonyl)pipéridin-4-yl]amino}-1H-pyrazolo[4,3-c]isoquinoléine

Etape 1 : Dans un ballon de 250 mL muni d'une agitation magnétique, on introduit 30 g de 2-chloroacétophénone et 36,3 g de phtalimide de potassium dans 120 mL de DMF. Après 4 h à TA, le milieu est concentré sous vide. Le solide obtenu est repris dans 120 ml de DCM.et 100 ml d'une solution 1 d'hydroxyde de sodium. La phase organique est lavée avec une solution aqueuse saturée de NaCl, séchée sur MgSO₄, filtrée et concentrée sous vide. Le solide obtenu est trituré dans de l'éther diisopropylique. L'insoluble est filtré et sécher sous vide pour conduire à 42,6 g de 2-(2-oxo-2-phényléthyl)-1H-isoindole-1,3(2H)-dione sous forme de solide beige utilisé tel quel à l'étape suivante.
Etape 2 : Dans un ballon de 100 mL muni d'une agitation magnétique, on introduit 42.4 g de 2-(2-oxo-2-phényléthyl)-1H-isoindole-1,3(2H)-dione dans 105 mL de *N,N*-diméthyformamide diéthyl acétal. Le milieu est porté 3 h à reflux puis concentré sous PR. On purifie par flash-chromatographie sur gel de silice (15-40 µm), en éluant par du DCM puis un mélange de DCM et de méthanol (90/10 vol) pour conduire à 40,69 g de mélange des isomères E et Z de 2-[1-(diméthylamino)-3-oxo-3-phénylprop-1-èn-2-yl]-1H-isoindole-1,3(2H)-dione sous forme de solide crème. LC-MS-DAD-ELSD : 321(+)=(M+H)(+).
Etape 3 : Dans un ballon 250 mL, on introduit à TA, 20.5 g de mélange des isomères E et Z de 2-[1-(diméthylamino)-3-oxo-3-phénylprop-1-èn-2-yl]-1H-isoindole-1,3(2H)-dione dans 75 mL d'éthanol. On additionne 7,8 mL d'hydrate d'hydrazine. La suspension est agitée 1 h30 à TA puis portée au reflux. Après 1h30 de chauffage, on peut observer un épaississement du mélange qui prend une couleur jaune. On essore à chaud la suspension sur fritté et le précipité est lavé par de l'éthanol. Le filtrat est concentré sous PR. On purifie par flash-chromatographie sur gel de silice (15-40 µm), en éluant par du DCM puis un mélange DCM/méthanol (95/5 vol) pour conduire à 9,45 g de 5-phényl-1 H-pyrazol-4-amine (solide marron). LC-MS-DAD-ELSD : 163(+)=(M+H)(+).
Etape 4 : Dans un ballon 250 ml muni d'une agitation magnétique, on introduit 10 g de 5-phényl-1H-pyrazol-4-ylamine dans 45 mL de DCM et 45 mL de pyridine. Le milieu réactionnel est refroidi à 0°C à l'aide d'un bain de glace et 17,4 mL du chlorure de l'acide 2,6-difluorobenzoïque sont additionnés. Le mélange est agité 3 h à TA. Après évaporation du solvant sous PR, on ajoute 50 ml d'eau sur le brut réactionnel puis la phase aqueuse est extraite par deux fois 50 ml d'AcOEt. Les extraits organiques sont regroupés, lavés avec une solution saturée en NaCl, séchés sur MgSO₄. On évapore alors l'AcOEt sous vide et obtient ainsi un mélange de 2,6-difluoro-N-[5-phényl-1H-pyrazol-4-yl]-benzamide et de N-[1-(2,6-difluoro-benzoyl)-5-phényl-1H-pyrazol-4-yl]-2,6-difluoro-benzamide sous forme d'une huile marron Le brut réactionnel est traité de façon à obtenir uniquement le produit attendu: dans un ballon de 250 ml, on dissout à TA et sous argon, le mélange de 2,6-difluoro-N-[5-phényl-1H-pyrazol-4-yl]-benzamide et de N-[1-(2,6-difluoro-benzoyl)-5-phényl-1H-pyrazol-4-yl]-2,6-difluoro-benzamide dans 20 mL de méthanol et 20 mL de solution aqueuse de soude 5N. Après 30 min à TA, on coule le mélange dans 50 mL d'eau et on extrait à l'AcOEt, lave les phases organiques avec une solution saturée en NaCl, sèche sur MgSO₄ puis concentre sous PR. On obtient un produit brut que l'on met en suspension dans du DCM et on agite 15 min au reflux du DCM. Après refroidissement à l'aide d'un bain de glace, on essore le précipité sur fritté, le lave deux fois avec du DCM glacé. On obtient ainsi 7,7 g de 2,6-difluoro-N-(5-phényl-1H-pyrazol-4-yl)benzamide (solide ocre). LC-MS-DAD-ELSD : 300(+)=(M+H)(+).
Etape 5 : Dans un tricol de 500 mL, muni d'une agitation magnétique et d'une arrivée d'argon, on introduit 12,5 g de 2,6-difluoro-N-(5-phényl-1H-pyrazol-4-yl)benzamide, 218 mL d'oxychlorure de phosphore et 68,2 g de P₂O₅. Après 30 min à reflux, le milieu prend en masse. 20 ml d'oxychlorure de phosphore sont ajoutés. Après 5 h de reflux, le mélange réactionnel est refroidi à TA puis versé lentement dans 2 L d'eau glacée en maintenant la température inférieure à 40°C. On neutralise par addition lente de carbonate de potassium et on extrait 3 fois avec 500 mL d'AcOEt. Les extraits organiques sont regroupés, lavés avec une solution saturée en NaCl et séchés sur MgSO₄, puis concentrés sous PR. Le solide obtenu est purifié par flash-chromatographie sur gel de silice (15-40 µm), en éluant par du DCM puis un mélange DCM/AcOEt (80/20 vol). On obtient 6,4 g de 5-(2,6-difluorophényl)-1H-pyrazolo[4,3-c]isoquinoléine (meringue jaune). LC-MS-DAD-ELSD 282(+)=(M+H)(+).
Etape 6 : Dans un ballon de 50 mL, muni d'une agitation magnétique et d'un septum surmonté d'une arrivée d'argon, on introduit 700 mg de 5-(2,6-difluorophényl)-1H-pyrazolo[4,3-c]isoquinoléine dans 25 mL de DMF. On ajoute 447 mg de KOH puis 0.26 mL de brome 447 mg de KOH. Après 1 h à TA, on verse le mélange sur 100 ml d'eau glacée. La phase aqueuse est extraite avec 3 fois 100 mL d'AcOEt. Les extraits organiques sont regroupés, lavés avec une solution saturée en NaCl, séchés sur MgSO₄ et évaporés sous PR. On obtient 950 mg de 3-bromo-5-(2,6-difluorophényl)-1H-pyrazolo[4,3-c]isoquinoléine (solide orange). Le produit sera utilisé tel quel dans l'étape suivante. LC-MS-DAD-ELSD : 362(+)=(M+H)(+).
Etape 7 : Dans un ballon de 50 mL, muni d'une agitation magnétique, d'un septum surmonté d'une arrivée d'argon, on introduit 890 mg de 3-bromo-5-(2,6-difluorophényl)-1H-pyrazolo[4,3-c]isoquinoléine dans 20 mL de DMF, 2,46 ml de N,N-diisopropyléthylamine et 658 µl de 2-(triméthylsilyl)éthoxyméthyl chloride. Après 16 h à TA, le mélange est versé dans 50 ml d'eau glacée. La phase aqueuse est extraite avec 3 fois 50 mL d'AcOEt, les extraits organiques sont regroupés, lavés avec une solution saturée en NaCl, séchés sur MgSO₄ et concentrés sous PR. On purifie par flash-chromatographie sur gel de silice (15-40 µm), en éluant par un mélange de cyclohexane et de DCM (80/20 vol) puis (50/50 vol), on obtient d'une part 498 mg de 3-bromo-5-(2,6-difluorophényl)-1-{[2-(triméthylsilyl)éthoxy]méthyl}-1H-pyrazolo[4,3-c]isoquinoléine sous forme d'une gomme blanche LC-MS-DAD-ELSD : 492(+)=(M+H)(+) et d'autre part 200 mg de 3-bromo-5-(2,6-difluorophényl)-2-{[2-(triméthylsilyl)éthoxy]méthyl}-2H-pyrazolo[4,3-c]isoquinoléine (gomme blanche). LC-MS-DAD-ELSD : 492(+)=(M+H)(+).
Etape 8 : Dans un tricol de 25 mL, muni d'une agitation magnétique et d'une arrivée d'argon, on introduit 200 mg de 3-bromo-5-(2,6-difluorophényl)-1-{[2-(triméthylsilyl)éthoxy]méthyl}-1H-pyrazolo[4,3-c]isoquinoléine, dans 6 mL de 1-4-dioxane. Après un barbotage de 10 min d'argon dans le milieu réactionnel, on ajoute 73 mg de 1-méthylsulfonyl-pipéridin-4-ylamine, 505 mg de carbonate de césium, 36 mg de 9,9-diméthyl-4,5-bis(diphénylphosphino)xanthène et 10 mg d'acétate de palladium (II). Le mélange est chauffé 2 h à 120°C. Après refroidissement, le mélange est coulé dans de l'eau, extrait à l'AcOEt. La phase organique est lavée avec une solution saturée en NaCl, séchée sur MgSO₄, filtrée et concentrée sous PR. On purifie par flash-chromatographie sur gel de silice (15-40 µm), en éluant par un mélange de cyclohexane et d'AcOEt (50/50 vol), on obtient ainsi 71 mg de 5-(2,6-difluorophényl)-3-{[1-(éthylsulfonyl)pipéridin-4-yl]amino}-1-{[2-(triméthylsilyl)éthoxy]méthyl}-1H-pyrazolo[4,3-c]isoquinoléine (gomme jaune). LC-MS-DAD-ELSD : 588(+)=(M+H)(+).
Etape 9 : Dans un ballon de 10 mL, on introduit 71 mg de 5-(2,6-difluorophényl)-3-{[1-(méthylsulfonyl)pipéridin-4-yl]amino}-1-{[2-(triméthylsilyl)éthoxy]méthyl}-1H-pyrazolo[4,3-c]isoquinoléine dans 3 mL de DCM. Après refroidissement vers 0°C à l'aide d'un bain de glace, on ajoute 0,3 mL de TFA et on agite à TA pendant 16 h. Le mélange réactionnel est concentré sous PR et le solide obtenu est repris dans 20 mL d'eau et extrait avec trois fois 20 mL d'AcOEt. La phase organique est séchée sur MgSO₄, filtrée et concentrée sous PR. On purifie par flash-chromatographie sur gel de silice (15-40 µm), en éluant par du DCM puis un mélange de DCM et de méthanol (95/5 vol) pour conduire à 9 mg de 5-(2,6-difluorophényl)-3-{[1-(méthylsulfonyl)pipéridin-4-yl]amino}-1H-pyrazolo[4,3-c]isoquinoléine (solide beige). ¹H RMN (400 MHz, DMSO-d6) δ ppm 1,67 (m, 2 H) 2,11 (m, 2 H) 2,88 (s, 3 H) 2,90 (m, 2 H) 3,56 (m, 2 H) 3,79 (s large, 1 H) 6,04 (m étalé, 1 H) 7,32 (m, 2 H) 7,52 - 7,74 (m, 3 H) 7,92 (m, 1 H) 8,40 (d, 1 H) 12,76 (s large, 1 H) ; LC-MS-DAD-ELSD : 458(+)=(M+H)(+).

### Exemple 11: 5-(2,6-difluorophényl)-3-{[1-(cyclopropylsulfonyl)pipéridin-4-yl]amino}-1H-pyrazolo[4,3-c]isoquinoléine-7-carbonitrile

Etape 1 : Dans un tricol de 25 mL, muni d'une agitation magnétique et d'une arrivée d'argon, on introduit 150 mg de 3-bromo-5-(2,6-difluorophényl)-1-{[2-(triméthylsilyl)éthoxy]méthyl}-1H-pyrazolo[4,3-c]isoquinoléine-7-carbonitrile, préparé à l'étape 11 de l'ex.1 dans 4 mL de 1-4-dioxane. Après un barbotage de 10 min d'argon dans le mélange, on ajoute 60 mg de 1-cyclopropylsulfonylpipéridin-4-ylamine, 361 mg de carbonate de césium, 25 mg de 9,9-diméthyl-4,5-bis(diphénylphosphino)xanthène et 7 mg d'acétate de palladium (II). Le mélange est chauffé 2 h à 120°C. Après refroidissement, le mélange est coulé dans de l'eau, extrait à l'AcOEt. La phase organique est lavée avec une solution saturée en NaCl, séchée sur MgSO₄, filtrée et concentrée sous PR. On purifie par flash-chromatographie sur gel de silice (15-40 µm), en éluant par un mélange de cyclohexane et d'AcOEt (50/50 vol), on obtient ainsi, 57 mg de 5-(2,6-difluorophényl)-3-{[1-(cyclopropyllsulfonyl)pipéridin-4-yl]amino}-1-{[2-(triméthylsilyl)éthoxy]méthyl}-1H-pyrazolo[4,3-c]isoquinoléine-7-carbonitrile (solide jaune). LC-MS-DAD-ELSD : 639 (+)=(M+H)(+).
Etape 2 : Dans un ballon de 10 mL, muni d'une agitation magnétique, on introduit 57 mg de 5-(2,6-difluorophényl)-3-{[1-(cyclopropylsulfonyl)pipéridin-4-yl]amino}-1-{[2-(triméthylsilyl)éthoxy]méthyl}-1H-pyrazolo[4,3-c]isoquinoléine-7-carbonitrile dans 5 mL de DCM. Après refroidissement vers 0°C à l'aide d'un bain de glace, on ajoute 0,3 mL de TFA et on agite 3 h à TA. Le mélange est concentré sous PR et le solide obtenu est repris dans 20 mL d'eau et extrait avec trois fois 20 mL d'AcOEt, séché sur MgSO₄, filtré et concentré sous PR. On purifie par flash-chromatographie sur gel de silice (15-40 µm), en éluant par un mélange de cyclohexane et d'AcOEt (80/20 vol) pour conduire à 16 mg de 5-(2,6-difluorophényl)-3-{[1-(cyclopropylsulfonyl)pipéridin-4-yl]amino}-1H-pyrazolo[4,3-c]isoquinoléine-7-carbonitrile (solide jaune). ¹H NMR (400 MHz, DMSO-d6) δ ppm 0,89 - 1,04 (m, 4 H) 1,67 (m, 2 H) 2,10 (m, 2 H) 2,58 (m, 1 H) 3,00 (m, 2 H) 3,62 (m, 2 H) 3,82 (m, 1 H) 6,21 (d, *J*=7,8 Hz, 1 H) 7,34 (t, *J*=7.8 Hz, 2 H) 7,70 (m, 1 H), 8,19 (s large, 1 H) 8,27 (dd, *J*=8,5, 1,5 Hz, 1 H) 8,56 (d, *J*=8,5 Hz, 1 H) 13,07 (s, 1 H) LC-MS-DAD-ELSD : 509(+)=(M+H)(+).

### Exemple 12 5-(2,6-difluorophényl)-3-{[1-(méthylsulfonyl)pipéridin-4-yl]amino}-1H-pyrazolo[4,3-c]isoquinoléine-7-carbonitrile

Etape 1 : Dans un tricol de 25 mL, muni d'une agitation magnétique et d'une arrivée d'argon, on introduit 525 mg de 3-bromo-5-(2,6-difluorophényl)-1-{[2-(triméthylsilyl)éthoxy]méthyl}-1H-pyrazolo[4,3-c]isoquinoléine-7-carbonitrile, préparé à l'étape 11 de l'ex. 1, dans 12 mL de 1-4-dioxane. Après un barbotage de 10 min d'argon dans le milieu réactionnel, on ajoute 294 mg de 1-méthylsulfonyl-pipéridin-4-ylamine, 1,26 g de carbonate de césium, 89 mg de 9,9-diméthyl-4,5-bis(diphénylphosphino)xanthène et 23 mg d'acétate de palladium(II). Le mélange réactionnel est chauffé à 120°C pendant 2 h. Après refroidissement, le mélange est coulé dans de l'eau, extrait à l'AcOEt. La phase organique est lavée avec une solution saturée en NaCl, séchée sur MgSO₄, filtrée et concentrée sous PR. On purifie par flash-chromatographie sur gel de silice (15-40 µm), en éluant par un mélange cyclohexane /AcOEt (80/20 vol) puis (50/50 vol) pour conduire à 470 mg de 5-(2,6-difluorophényl)-3-{[1-(éthylsulfonyl)pipéridin-4-yl]amino}-1-{[2-(triméthylsilyl)éthoxy]méthyl}-1 H-pyrazolo[4,3-c]isoquinoléine-7-carbonitrile (solide jaune). LC-MS-DAD-ELSD : 613(+)=(M+H)(+).
Etape 2 : Dans un tube micro-ondes de capacité maximale 20 ml muni d'une agitation magnétique, on introduit 300 mg de 5-(2,6-difluorophényl)-3-{[1-(méthylsulfonyl)pipéridin-4-yl]amino}-1-{[2-(triméthylsilyl)éthoxy]méthyl}-1H-pyrazolo[4,3-c]isoquinoléine-7-carbonitrile dans 12 mL d'acide chlorhydrique 5N. Le mélange réactionnel est chauffé 5 min à 100°C au micro-ondes puis versé dans 20 ml d'eau, ramené à pH 7 par addition d'une solution 9,3 N d'hydroxyde de sodium et extrait avec trois fois 20 mL d'AcOEt. Les phases organiques réunies sont séchées sur MgSO₄, filtrées et concentrées sous PR. On purifie par flash-chromatographie sur gel de silice (15-40 µm), en éluant par un mélange de DCM et d'AcOEt (50/50 vol) pour conduire à 180 mg de 5-(2,6-difluorophényl)-3-{[1-(méthylsulfonyl)pipéridin-4-yl]amino}-1H-pyrazolo[4,3-c]isoquinoléine-7-carbonitrile (solide jaune). ¹H NMR (400 MHz, DMSO-d6) 1,67 (m, 2 H) 2,11 (m, 2 H) 2,87 (s, 3 H) 2,90 (m, 2 H) 3,56 (m,2 H) 3,80 (m, 1 H) 6,20 (d large, J=7,7 Hz, 1 H) 7,34 (m, 2 H) 7,70 (m, 1 H) 8,18 (s large, 1 H) 8,28 (dlarge, J=8.6 Hz, 1 H) 8,56 (d, J=8,6 Hz, 1 H) 13,07 (s, 1 H) LC-MS-DAD-ELSD : 483(+)=(M+H)(+).

### Exemple 13 5-(2,6-difluorophényl)-3-{[1-(éthylsulfonyl)pipéridin-4-yl]amino}-1H-pyrazolo[4,3-c]isoquinoléine-7-carbonitrile

Etape 1 : Dans un tricol de 25 mL, muni d'une agitation magnétique et d'une arrivée d'argon, on introduit 150 mg de 3-bromo-5-(2,6-difluorophényl)-1-{[2-(triméthylsilyl)éthoxy]méthyl}-1H-pyrazolo[4,3-c]isoquinoléine-7-carbonitrile, préparé à l'étape 11 de l'ex. 1, dans 4 mL de 1-4-dioxane. Après un barbotage de 10 min d'argon dans le milieu réactionnel, on ajoute 56 mg de 1-éthylsulfonyl-pipéridin-4-ylamine, 361 mg de carbonate de césium, 25 mg de 9,9-diméthyl-4,5-bis(diphénylphosphino)xanthène et 7 mg d'acétate de palladium (II). Le mélange est chauffé 2 h à 120°C. Après refroidissement, le mélange est coulé dans de l'eau, extrait à l'AcOEt. La phase organique est lavée avec une solution saturée en NaCl, séchée sur MgSO₄, filtrée et concentrée sous PR. On purifie par flash-chromatographie sur gel de silice (15-40 µm), en éluant par un mélange cyclohexane/AcOEt (80/20 vol) puis (50/50 vol), on obtient ainsi 60 mg de 5-(2,6-difluorophényl)-3-{[1-(éthylsulfonyl)pipéridin-4-yl]amino}-1-{[2-(triméthylsilyl)éthoxy]méthyl}-1H-pyrazolo[4,3-c]isoquinoléine-7-carbonitrile (solide jaune). LC-MS-DAD-ELSD : 627(+)=(M+H)(+).
Etape 2 : Dans un ballon de 10 mL, muni d'une agitation magnétique, on introduit 50 mg de 5-(2,6-difluorophényl)-3-{[1-(éthylsulfonyl)pipéridin-4-yl]amino}-1-{[2-(triméthylsilyl)éthoxy]méthyl}-1H-pyrazolo[4,3-c]isoquinoléine-7-carbonitrile dans 5 mL de DCM. Après refroidissement vers 0°C à l'aide d'un bain de glace, on ajoute 0,1 mL de TFAet on agite à TA pendant 3 h. Le mélange est concentré sous PR et le solide obtenu est repris dans 20 mL d'eau et extrait avec trois fois 20 mL d'AcOEt. Les phases organiques réunies sont sèchées sur MgSO₄, filtrées et concentrées sous PR. On purifie par flash-chromatographie sur gel de silice (15-40 µm), en éluant par du DCM puis un mélange de DCM et de méthanol (90/10 vol) pour conduire à 27 mg de 5-(2,6-difluorophényl)-3-{[1-(éthylsulfonyl)pipéridin-4-yl]amino}-1H-pyrazolo[4,3-c]isoquinoléine-7-carbonitrile (solide jaune). ¹H NMR (400 MHz, DMSO-d6) 1,23 (t, J=7,3 Hz, 3 H) 1,63 (m, 2 H) 2,09 (m, 2 H) 2,97 (m, 2 H) 3,05 (q, J=7,3 Hz, 2 H) 3,61 (m, 2 H) 3,81 (m, 1 H) 6,23 (m étalé, 1 H) 7,34 (t, J=7,8 Hz, 2 H) 7,70 (m, 1 H) 8,18 (s large, 1 H) 8,27 (d large, J=8,5 Hz, 1 H) 8,56 (d, J=8,5 Hz, 1 H) 13,07 (s large, 1 H) LC-MS-DAD-ELSD : 497(+)=(M+H)(+).

### Exemple 14 5-(2,6-difluorophényl)-3-{[1-(cyclopropylsulfonyl)pipéridin-4-yl]amino}-1H-pyrazolo[4,3-c]isoquinoléine-7-carboxamide

Dans un ballon de 10 mL, on introduit 29.7 mg de 5-(2,6-difluorophényl)-3-{[1-(cyclopropylsulfonyl)pipéridin-4-yl]amino}-1H-pyrazolo[4,3-c]isoquinoléine-7-carbonitrile préparé à l'ex. 11, 1,3 mL d'éthanol et 125 µL d'hydroxyde de sodium 9,3N. Après 30 min à reflux, le mélange est concentré sous PR et le solide obtenu est repris dans 10 mL d'eau et extrait avec trois fois 10 mL d'AcOEt. Les phases organiques réunies sont séchées sur MgSO₄, filtrées et concentrées sous PR pour conduire à 17 mg de 5-(2,6-difluorophényl)-3-{[1-(cyclopropylsulfonyl)pipéridin-4-yl]amino}-1H-pyrazolo[4,3-c]isoquinoléine-7-carboxamide (solide jaune) ¹H NMR (400 MHz, DMSO-d6) δ ppm 0,89 - 1,02 (m, 4 H) 1,67 (m, 2 H) 2,11 (m, 2 H) 2,58 (m, 1 H) 3,00 (m, 2 H) 3,63 (m, 2 H) 3,80 (m, 1 H) 6,09 (d, J=7,3 Hz, 1 H) 7,35 (d, J=7,8 Hz, 2 H) 7,56 (s large, 1H) 7,68 (m, 1 H) 8,17 (s large, 1 H) 8,23 (s large, 1 H) 8,35 (d large, J=8,5 Hz, 1 H) 8,45 (d, J=8,5 Hz, 1 H) 12,87 (s, 1 H)LC-MS-DAD-ELSD : 527(+)=(M+H)(+).

### Exemple 15 5-(2,6-difluorophényl)-3-{[1-(méthylsulfonyl)pipéridin-4-yl]amino}-1H-pyrazolo[4,3-c]isoquinoléine-7-carbaldéhyde

Dans un ballon de 10 mL, on introduit 54 mg de 5-(2,6-difluorophényl)-3-{[1-(méthylsulfonyl)pipéridin-4-yl]amino}-1-{[2-(triméthylsilyl)éthoxy]méthyl}-1H-pyrazolo[4,3-c]isoquinoléine-7-carbaldéhyde, préparé à l'étape 5 de l'ex.2 dans 5 mL de DCM. Après refroidissement vers 0°C à l'aide d'un bain de glace, on ajoute 0,3 mL de TFA et agite 16 h à TA. Le mélange est concentré sous PR et le solide obtenu est repris dans 5 mL d'une solution aqueuse saturée de NaHCO₃. On extrait à l'AcOEt, sèche sur MgSO₄, filtre et concentre sous PR. On purifie par flash-chromatographie sur gel de silice (15-40 µm), en éluant par un mélange de DCM/AcOEt (50/50 vol). On obtient ainsi 9 mg de 5-(2,6-difluorophényl)-3-{[1-(méthylsulfonyl)pipéridin-4-yl]amino}-1H-pyrazolo[4,3-c]isoquinoléine-7-carbaldéhyde (solide jaune).¹H NMR (400 MHz, DMSO-d6) δ ppm 1,68 (m, 2 H) 2,11 (m, 2 H) 2,88 (s, 3 H) 2,90 (m, 2 H) 3,57 (m,2 H) 3,81 (m, 1 H) 6,16 (m large, 1 H) 7,36 (t, J=7,8 Hz, 2 H) 7,71 (m, 1 H) 8,23 (s large, 1 H) 8,34 (dlarge, J=8,5 Hz, 1 H) 8,57 (d, J=8,2 Hz, 1 H) 10,10 (s, 1 H) 13,02 (s large, 1 H) ; LC-MS-DAD-ELSD : 486(+)=(M+H)(+).

### Exemple 16 5-(2,6-difluorophényl)-N-hydroxy-3-{[1-(méthylsulfonyl)pipéridin-4-yl]amino}-1H-pyrazolo[4,3-c]isoquinoléine-7-carboximidamide

Dans un ballon de 10 mL, on introduit 30 mg de 5-(2,6-difluorophényl)-3-{[1-(méthylsulfonyl)pipéridin-4-yl]amino}-1H-pyrazolo[4,3-c]isoquinoléine-7-carbonitrile préparé à l'ex.12, 1,3 mL de méthanol, 0,8 ml de THF et 62 mg de triéthylamine. 44 mg d'hydroxyamine sous forme de chlorhydrate sont additionnés. Le mélange chauffé 3 h à 65°C puis évaporé sous PR. Le solide obtenu est repris dans 10 mL d'eau et extrait à l'AcOEt. La phase organique est séchée sur MgSO₄, filtrée et concentrée sous PR. On purifie par flash-chromatographie sur gel de silice (15-40 µm), en éluant avec du DCM puis un mélange de DCM/méthanol (80/20 vol) pour conduire à 9,7 mg de 5-(2,6-difluorophényl)-N-hydroxy-3-{[1-(méthylsulfonyl)pipéridin-4-yl]amino}-1H-pyrazolo[4,3-c]isoquinoléine-7-carboximidamide sous forme de solide jaune.¹H NMR (300 MHz, DMSO-d6) δ ppm 1,67 (m, 2 H) 2,11 (m, 2 H) 2,91 (m, 2 H) 2,87 (s, 3 H) 3,56 (m, 2 H) 3,79 (m, 1 H) 5,95 (s large, 2 H) 6,03 (d, J=7,9 Hz, 1 H) 7,31 (m, 2 H) 7,66 (m, 1 H) 7,95 (s large, 1 H) 8,18 (dd, J=8,5, 1,6 Hz, 1 H) 8,39 (d, J=8,5 Hz, 1 H) 9,82 (s, 1 H) 12,76 (s,1 H) ; LC-MS-DAD-ELSD : 516(+)=(M+H)(+).

### Exemple 17 5-(2,6-difluorophényl)-7-(fluorométhyl)-N-[1-(méthylsulfonyl)pipéridin-4-yl]-1H-pyrazolo[4,3-c]isoquinoléin-3-amine

Etape 1 : A partir de 377 mg de [5-(2,6-difluorophényl)-3-{[1-(méthylsulfonyl)pipéridin-4-yl]amino}-1-{[2-(triméthylsilyl)éthoxy]méthyl}-1H-pyrazolo[4,3-c]isoquinoléin-7-yl]-méthanol préparé à l'étape 1 de l'exemple 3, et de 7 mL de DCM. On refroidit à -70°C à l'aide d'un bain de carboglace et ajoute 88 µL de trifluorure de diéthylaminosufure (DAST). Après 30 min le mélange est coulé dans une solution aqueuse saturée de bicarbonate de sodium, extrait à l'AcOEt, lavé avec une solution saturée en NaCl, séché sur MgSO₄, filtré et concentré sous PR. On purifie par flash-chromatographie sur gel de silice (40-63 µm), en éluant par un mélange DCM/AcOEt (85/15 vol), on obtient ainsi 90 mg de [5-(2,6-difluorophényl)-7-(fluorométhyl)-N-[1-(méthylsulfonyl)pipéridin-4-yl]-2-{[2-(triméthylsilyl)éthoxy]méthyl}-2H-pyrazolo[4,3-c]isoquinoléin-3-amine (solide jaune). SM (E/I) : m/z = 619 (M+) ; RMN ¹H : -0,05 (s, 9 H) 0,88 (m, 2 H) 1,60 (m, 2 H) 2,10 (m, 2 H) 2,74 (m, 2 H) 2,84 (s, 3 H) 3,58 (m, 2H) 3,66 (m, 2H) 4,44 (m large, 1 H) 5,58 (d, J=47,4 Hz, 2H) 5,65 (s, 2H) 6,48 (d, J=7,6 Hz, 1H) 7,33 (t, J=7,5 Hz, 2H) 7,52 (s large, 1H) 7,64 (m, 1H) 7,82 (d large, J=8,1 Hz, 1 H) 8,38 (d, J=8,1 Hz, 1 H).
Etape 2 : Dans un ballon de 30 mL, muni d'une agitation magnétique, d'un septum surmonté d'une arrivée d'argon, on introduit 87 mg de [5-(2,6-difluorophényl)-7-(fluorométhyl)-N-[1-(méthylsulfonyl)pipéridin-4-yl]-2-{[2-(triméthylsilyl)éthoxy]méthyl}-2H-pyrazolo[4,3-c]isoquinoléin-3-amine dans 7 mL de DCM. Après refroidissement vers 0°C à l'aide d'un bain de glace, on ajoute 0,85 mL de TFA et on agite 3 h à TA. Le mélange est concentré sous PR et le solide obtenu est repris dans 24 mL d'eau et 1,1 mL d'une solution aqueuse 0,75M d'ammoniaque. On extrait à l'acétate d'éthyle, sèche sur MgSO₄, filtre et concentre sous PR. On purifie par flash-chromatographie sur gel de silice (15-40 µm), en éluant par un mélange de DCM/MeOH (95/05 vol). On obtient ainsi, 27 mg de [5-(2,6-difluorophényl)-7-(fluorométhyl)-N-[1-(méthylsulfonyl)pipéridin-4-yl]-1H-pyrazolo[4,3-c]isoquinoléin-3-amine (solide jaune). RMN ¹H : 1,67 (m, 2 H) 2,11 (m, 2 H) 2,87 (s, 3 H) 2,90 (m, 2 H) 3,56 (m, 2 H) 3,80 (m large, 1 H) 5,58 (d, *J*=47,2 Hz, 2 H) 6,05 (d large, J=8,0 Hz, 1H) 7,33 (t, *J*=7,7 Hz, 2 H) 7,57-7,73 (m, 2 H) 7,95 (d large, *J*=8,3 Hz, 1 H) 8,45 (d, *J*=8,3 Hz, 1 H) 12,81 (s, 1 H)

### Exemple 18 :5-(2,6-difluorophényl)-3-{[1-(éthylsulfonyl)pipéridin-4-yl]amino}-1H-pyrazolo[4,3-c]isoquinoléine-7-carboxamide

Etape 1 : Dans un ballon de 100 mL, muni d'une agitation magnétique et d'un septum surmonté d'une arrivée d'argon, on introduit 600 mg de 3-bromo-5-(2,6-difluorophényl)-1-{[2-(triméthylsilyl)éthoxy]méthyl}-1H-pyrazolo[4,3-c]isoquinoléine-7-carbonitrile dans 15 mL de 1-4-dioxane. Après un barbotage de 10 min d'argon dans le milieu réactionnel, on ajoute 448 mg de 1-éthanesulfonyl-pipéridin-4-ylamine, 1,4 g de carbonate de césium, 80 mg de 9,9-diméthyl-4,5-bis(diphénylphosphino)xanthène et 26 mg d'acétate de palladium (II). Le mélange est chauffé 4 h à reflux. Après refroidissement, le mélange est coulé dans de l'eau, extrait à AcOEt, lavé avec une solution saturée en NaCl, séché sur MgSO₄, filtré et concentré sous PR. On purifie par flash-chromatographie sur gel de silice (40-63 µm), en éluant par un mélange de DCM et AcOEt (95/05 vol), on obtient ainsi, 436 mg de 5-(2,6-difluorophényl)-3-{[1-(éthylsulfonyl)pipéridin-4-yl]amino}-1-{[2-(triméthylsilyl)éthoxy]méthyl}-1H-pyrazolo[4,3-c]isoquinoléine-7-carbonitrile (solide jaune). SM (E/I) : m/z = 626 (M+) ; RMN ¹H : -0,06 (s, 9H) ; 0,88 (m, 2H) ; 1,18 (t, J=7,3 Hz, 3H) ; 1,58 (m, 2H) ; 2,07 (m, 2H) ; 2,82 (m, 2H) ; 3,03 (q, J=7,3 Hz, 2H) ; 3,56 - 3,70 (m, 4H) ; 4,47 (m, 1 H) ; 5,67 (s, 2H) ; 6,69 (d, J = 8,1 Hz, 1H) ; 7,33 (t, J = 7,8 Hz, 2H) ; 7,66 (m, 1H) ; 7,96 (d, J=1,7 Hz, 1H) ; 8,13 (dd, J = 8,3 et 1,7 Hz, 1 H) ; 8,47 (d, J = 8,3 Hz, 1 H).
Etape 2 : Dans un ballon de 100 mL, muni d'une agitation magnétique, d'un septum surmonté d'une arrivée d'argon, on introduit 396 mg de 5-(2,6-difluorophényl)-3-{[1-(éthylsulfonyl)pipéridin-4-yl]amino}-1-{[2-(triméthylsilyl)éthoxy]méthyl}-1H-pyrazolo[4,3-c]isoquinoléine-7-carbonitrile dans 4 mL de toluène Après refroidissement vers 0°C à l'aide d'un bain de glace, on ajoute 0,64 mL d'acide sulfurique à 95% et agite à TA pendant 15 h. Le mélange est refroidi à 0°C et neutralisé par 9 mL d'une solution aqueuse NH₃ 7,5 M. On extrait à AcOEt, sèche sur MgSO₄, filtre et concentre sous PR. On purifie par flash-chromatographie sur gel de silice (15-40 µm), en éluant par un mélange de DCM et de MeOH (95/05 vol). On obtient ainsi, 174 mg de 5-(2,6-difluorophényl)-3-{[1-(éthylsulfonyl)pipéridin-4-yl]amino}-1H-pyrazolo[4,3-c]isoquinoléine-7-carboxamide (solide jaune). SM (E/I) : m/z = 514 (M+) ; RMN ¹H: 1,23 (t, J=7,4 Hz, 3H) ; 1,65 (m, 2H) ; 2,09 (m, 2H) ; 2,98 (m, 2H) ; 3,05 (q, J=7,4 Hz, 2H) ; 3,62 (m, 2H) ; 3,81 (m, 1H) ; 6,09 (d, J = 8,3 Hz, 1H) ; 7,33 (m, 2H) ; 7,55 (s large, 1 H) ; 7,68 (m, 1 H) ; 8,17 (s large, 1 H) ; 8,23 (s large, 1 H) ; 8,35 (d large, J = 8,8 Hz, 1H) ; 8,45 (d, J=8,8 Hz, 1 H) ; 12,85 (s, 1 H).

### Exemple 19 5-(2,6-difluorophényl)-3-{[1-(éthylsulfonyl)pipéridin-4-yl]amino}-1H-pyrazolo[4,3-c]isoquinoléine-7-carbaldéhyde

Etape 1 : Dans un tricol de 50 mL, muni d'une agitation magnétique et d'une arrivée d'argon, on introduit 350 mg de 3-bromo-5-(2,6-difluorophényl)-2-(2-triméthylsilanyl-éthoxyméthyl)-2H-pyrazolo[4,3-c]isoquinoléine-7-carbaldéhyde, préparé à l'étape 5 de l'exemple 2, dans 7,8 mL de 1-4 dioxanne. On ajoute 195 mg de 1-éthylsulfonyl-pipéridin-4-ylamine, 880 mg de carbonate de césium, 59 mg de 9,9-diméthyl-4,5-bis(diphénylphosphino)xanthène et 15 mg d'acétate de palladium (II). Le mélange est chauffé 2 h à reflux. Après refroidissement, le mélange est coulé dans de l'eau, extrait à l'AcOEt. La phase organique est lavée avec une solution saturée en NaCl, séchée sur MgSO₄, filtrée et concentrée sous PR. On purifie par flash-chromatographie sur gel de silice (15-40 µm), en éluant par un mélange de cyclohexane et d'AcOEt (75/25 vol) pour conduire à 195 mg de 5-(2,6-difluorophényl)-3-{[1-(éthylsulfonyl)pipéridin-4-yl]amino}-1-{[2-(triméthylsilyl)éthoxy]méthyl}-1H-pyrazolo[4,3-c]isoquinoléine-7-carbaldéhyde (meringue jaune). LC-MS-DAD-ELSD 628=(M-H)(+).
Etape 2 : Dans un tube micro-ondes de capacité maximale 20 ml, on introduit 195 mg de 5-(2,6-difluorophényl)-3-{[1-(éthylsulfonyl)pipéridin-4-yl]amino}-1-{[2-(triméthylsilyl)éthoxy]méthyl}-1H-pyrazolo[4,3-c]isoquinoléine-7-carbaldéhyde, 10 ml d'une solution aqueuse 5 N d'HCl. Le mélange est chauffé 20 min aux micro-ondes à 100°C puis coulé dans de l'eau, extrait à l'AcOEt. La phase organique est lavée avec une solution saturée en NaCl, séchée sur MgSO₄, filtrée et concentrée sous PR. On purifie par flash-chromatographie sur gel de silice (15-40 µm), en éluant par un mélange de DCM et de MeOH (95/5 vol). On obtient 90 mg de 5-(2,6-difluorophényl)-3-{[1-(éthylsulfonyl)pipéridin-4-yl]amino}-1H-pyrazolo[4,3-c]isoquinoléine-7-carbaldéhyde sous forme d'une meringue jaune. RMN ¹H (500 MHz, DMSO-d6) δ ppm 1,23 (t, J=7,4 Hz, 3 H) 1,64 (m, 2 H) 2,10 (m, 2 H) 2,98 (m, 2H) 3,05 (q, J=7,4 Hz, 2 H) 3,62 (m, 2 H) 3,83 (m, 1 H) 6,18 (d, J=7,7 Hz, 1 H) 7,36 (t, J=7,8 Hz, 2 H) 7,71 (m, 1 H) 8,24 (s large, 1 H) 8,34 (d large, J=8,4 Hz, 1 H) 8,57 (d, J=8,4 Hz, 1 H) 10,11 (s, 1 H) 13,02 (s, 1 H). [M+H]+ m/z = 500

### Exemple 20 5-(2,6-difluorophényl)-3-{[1-(éthylsulfonyl)pipéridin-4-yl]amino}-1H-pyrazolo[4,3-c]isoquinoléine-7-carbaldéhyde oxime

Dans un ballon de 25 mL, on introduit 30 mg de 5-(2,6-difluorophényl)-3-{[1-(éthylsulfonyl)pipéridin-4-yl]amino}-1H-pyrazolo[4,3-c]isoquinoléine-7-carbaldéhyde préparé à l'ex. 19 dans 2 mL de pyridine. 7 mg d'hydroxyalmine sous forme de chlorhydrate sont additionnés. Après 1 h à TA, on évapore le mélange sous PR. On purifie par flash-chromatographie sur gel de silice (15-40 µm), en éluant par un mélange DCM/méthanol (95/5 vol) pour conduire à 22 mg de 5-(2,6-difluorophényl)-3-{[1-(éthylsulfonyl)pipéridin-4-yl]amino}-1H-pyrazolo[4,3-c]isoquinoléine-7-carbaldéhyde oxime (solide jaune). RMN ¹H (500 MHz, DMSO-d6) δ ppm avec l'isomérie E ou Z : 1,23 (t, J=7,4 Hz, 3 H) 1,63 (m, 2 H) 2,09 (m, 2 H) 2,97 (m, 2 H) 3,05 (q, J=7,4 Hz, 2 H) 3,61 (m, 2 H) 3,80 (m, 1 H) 6,08 (m large, 1 H) 7,33 (m, 2 H) 7,66 (m, 1 H) 7,86 (s large, 1 H) 8,14 (d large, J=8,3 Hz, 1 H) 8,30 (s, 1 H) 8,41 (d, J=8,3 Hz, 1 H) 11,45 (m large, 1 H) 12,82 (m large, 1 H). [M+H]+ m/z = 515

### Exemple 21 5-(2,6-difluorophényl)-3-{[1-(éthylsulfonyl)pipéridin-4-yl]amino}-1H-pyrazolo[4,3-c]isoquinoléin-7-yl]-méthanol

Dans un ballon de 25 mL, on introduit 30 mg de 5-(2,6-difluorophényl)-3-{[1-(éthylsulfonyl)pipéridin-4-yl]amino}-1H-pyrazolo[4,3-c]isoquinoléine-7-carbaldéhyde préparé exemple 19 dans 2 mL de méthanol. Le milieu est refroidi à 5°C. 12 mg de borohydrure de sodium sont additionnés. Après 30 min à 5°C, on évapore le mélange sous PR. Le solide obtenu est repris dans 10 mL d'une solution aqueuse 0,5 M d'HCl. On extrait à l'AcOEt, sèche la phase organique sur MgSO₄, filtre et concentre sous PR. On purifie par flash-chromatographie sur gel de silice (15-40 µm), en éluant par un mélange DCM/méthanol (95/5 vol) pour conduire à 12 mg de 5-(2,6-difluorophényl)-3-{[1-(éthylsulfonyl)pipéridin-4-yl]amino}-1H-pyrazolo[4,3-c]isoquinoléin-7-yl]-méthanol (solide jaune).RMN ¹H (500 MHz, DMSO-d6) δ ppm 1,23 (t, J=7,3 Hz, 3 H) 1,62 (m, 2 H) 2,09 (m, 2 H) 2,97 (m, 2 H) 3,05 (q, J=7,3 Hz, 2 H) 3,61 (m, 2 H) 3,79 (m, 1 H) 4,63 (d, J=5,5 Hz, 2 H) 5,36 (t, J=5,5 Hz, 1 H) 6,01 (d, J=7,8 Hz, 1 H) 7,32 (m, 2 H) 7,59 (s large, 1 H) 7,66 (m, 1 H) 7,83 (d large, J=8,3 Hz, 1 H) 8,35 (d, J=8,3 Hz, 1 H) 12,68 (s, 1 H). [M+H]+ m/z = 502

### Exemple 22 5-(2,6-difluorophényl)-3-[(1-sulfamoylpipéridin-4-yl)amino]-2H-pyrazolo[4,3-c]isoquinoléine-7-carboxamide

Etape1 : On introduit 707 mg de 3-bromo-5-(2,6-difluorophényl)-1-{[2-(triméthylsilyl)éthoxy]méthyl}-1H-pyrazolo[4,3-c]isoquinoléine-7-carbonitrile dans 17 mL de 1-4-dioxane. Après un barbotage de 10 min d'argon dans le mélange, on ajoute 846 mg de 4-aminopipéridine-1-sulfonamide, 2,15 g de carbonate de césium, 95 mg de 9,9-diméthyl-4,5-bis(diphénylphosphino)xanthène et 30 mg d'acétate de palladium (II). Le mélange est chauffé 20 h au reflux. Après refroidissement, le mélange est coulé dans de l'eau, extrait à l'AcOEt, lavé avec une solution saturée en NaCl, séché sur MgSO₄, filtré et concentré sous PR. On purifie par flash-chromatographie sur gel de silice (40-63 µm), en éluant par un mélange DCM/EtOH/NH3 (solution 7N dans du MeOH) (90/10/1 vol), on obtient ainsi, 407 mg de 5-(2,6-difluorophényl)-3-(pipéridin-4-ylamino)-2-[2-(triméthylsilyl)éthoxy]méthyl}-2H-pyrazolo[4,3-c]isoquinoléine-7-carbonitrile (huile jaune). RMN ¹H : -0,05 (s large, 9 H) 0,88 (t, *J*=7,9 Hz, 2 H) 1,39 (m, 2 H) 1,93 (m, 2 H) 2,08 (m étalé, 1 H) 2,39 (m, 2 H) 2,93 (m, 2 H) 3,71 (t, *J*=7,9 Hz, 2 H) 4,42 (m, 1 H) 5,66 (s, 2H) 6,58 (d, *J*=8,1 Hz, 1 H) 7,33 (t, *J*=7,9 Hz, 2 H) 7,66 (m, 1 H) 7,95 (s large, 1 H) 8,12 (d large, *J*=8,3 Hz, 1 H) 8,46 (d, *J*=8,3 Hz, 1 H).
Etape 2: 375 mg de 5-(2,6-difluorophényl)-3-(pipéridin-4-ylamino)-2-[2-(triméthylsilyl)éthoxy]méthyl}-2H-pyrazolo[4,3-c]isoquinoléine-7-carbonitrile, 2 mL de DME et 84 mg de sulfamide sont mélangés et chauffés aux micro-ondes 1 h à 150°C. Après évaporation du solvant, on fait précipiter le sulfamide en excès par ajout d'éther éthylique puis on l'essore sur fritté et concentre sous PR le filtrat. On obtient 439 mg de 4-{[7-cyano-5-(2,6-difluorophényl)-2-{[2-(triméthylsilyl)éthoxy]méthyl}-2H-pyrazolo[4,3-c]isoquinoléin-3-yl]amino}pipéridine-1-sulfonamide (solide jaune). RMN ¹H : -0,05 (m, 9 H) 0,88 (t, *J*=7,9 Hz, 2 H) 1,61 (m, 2 H) 2,09 (m, 2H) 2,53 (m partiellement masqué, 2 H) 3,49 (m, 2 H) 3,67 (t, *J*=7,9 Hz, 2 H) 4,40 (m, 1 H) 5,67 (s, 2 H) 6,67 (d, *J*=8,1 Hz, 1 H) 6,73 (s, 2 H) 7,34 (t, *J*=7,9 Hz, 2 H) 7,68 (m, 1 H) 7,95 (d, *J*=1,5 Hz, 1 H) 8,13 (dd, *J*=8,3, 1,5 Hz, 1 H) 8,47 (d, *J*=8,3 Hz, 1 H)
Etape 3 On introduit 54 mg de 4-{[7-cyano-5-(2,6-difluorophényl)-2-{[2-(triméthylsilyl)éthoxy]méthyl}-2H-pyrazolo[4,3-c]isoquinoléin-3-yl]amino}pipéridine-1-sulfonamide dans 0,5 mL de toluène. Après refroidissement vers 0°C à l'aide d'un bain de glace, on ajoute 90 µL d'acide sulfurique à 95% et agite à TA pendant 24 h. Le mélange rest refroidi à 0°C et neutralisé par 9 mL d'une solution aqueuse d'ammoniaque 7,5 M. On extrait à l'acétate d'éthyle, sèche sur MgSO₄, filtre et concentre sous PR. On purifie par flash-chromatographie sur gel de silice (15-40 µm), en éluant par un mélange DCM/méthanol (95/05 en volumes). On obtient 7 mg de 5-(2,6-difluorophényl)-3-[(1-sulfamoylpipéridin-4-yl)amino]-2H-pyrazolo[4,3-c]isoquinoléine-7-carboxamide (solide jaune). RMN ¹H : 1,67 (m, 2 H) 2,11 (m, 2 H) 2,67 (m, 2 H) 3,47 (m, 2 H) 3,71 (m,1 H) 6,01 (d, *J*=8,2 Hz, 1 H) 6,70 (s large, 2 H) 7,33 (t, *J*=7,7 Hz, 2 H) 7,55 (s large, 1 H) 7,68 (m, 1 H) 8,17 (s large, 1 H) 8,22 (s large, 1 H) 8,35 (d large, *J*=8,5 Hz, 1 H) 8,45 (d, *J*=8,5 Hz, 1 H) 12,84 (s, 1 H)

### Exemple 23 4-({5-(2,6-difluorophényl)-7-[(E/Z)-(hydroxyimino)méthyl]-1H-pyrazolo[4,3-c]isoquinoléin-3-yl}amino)pipéridine-1-sulfonamide

Etape 1 A partir de 406 mg de 4-{[7-cyano-5-(2,6-difluorophényl)-2-{[2-(triméthylsilyl)éthoxy]méthyl}-2H-pyrazolo[4,3-c]isoquinoléin-3-yl]amino}pipéridine-1-sulfonamide prépraré à l'étape 2 de l'ex. 22 et de 4 mL de toluène, on ajoute 3 mL de DCM et on refroidit à 0°C à l'aide d'un bain de glace. On ajoute goutte à goutte 970 µL d'hydrure de diisobutylaluminium et agite 2 h vers 0°C. Le mélange est coulé dans de la soude 0,1 M, extrait à l'AcOEt, lavé avec une solution saturée en NaCl, séché sur MgSO₄, filtré et concentré sous PR. On purifie par flash-chromatographie sur gel de silice (40-63 µm), en éluant par un mélange DCM/méthanol (90/10 en volumes). On obtient 251 mg de 4-{[5-(2,6-difluorophényl)-7-formyl-2-{[2-(triméthylsilyl)éthoxy]méthyl}-2H-pyrazolo[4,3-c]isoquinoléin-3-yl]amino}pipéridine-1-sulfonamide (huile jaune) utilisée telle quelle dans l'étape suivante.
Etape 2 : On introduit 250 mg de 4-{[5-(2,6-difluorophényl)-7-formyl-2-{[2-(triméthylsilyl)éthoxy]méthyl}-2H-pyrazolo[4,3-c]isoquinoléin-3-yl]amino}pipéridine-1-sulfonamide dans 5 mL de toluène. Après refroidissement vers 0°C à l'aide d'un bain de glace, on ajoute 206 µL d'acide sulfurique à 95% et poursuit l'agitation 1 h à 0°C. Le mélange est neutralisé par 10 mL d'une solution aqueuse d'ammoniaque 7,5 M. On extrait à l'acétate d'éthyle, sèche sur MgSO₄, filtre et concentre sous PR. On purifie par flash-chromatographie sur gel de silice (15-40µm), en éluant par un mélange DCM/éthanol (90/10 en volumes). On obtient 38 mg de 4-{[5-(2,6-difluorophényl)-7-formyl-1H-pyrazolo[4,3-c]isoquinoléin-3-yl]amino}pipéridine-1-sulfonamide (solide jaune). RMN ¹H : 1,62 - 1,72 (m, 2 H) 2,11 (m, 2 H) 2,62 - 2,71 (m, 2 H) 3,47 (m, 2 H) 3,67 - 3,76 (m, 1 H) 6,14 (m étalé, 1 H) 6,73 (s, 2 H) 7,36 (t, *J*=7,8 Hz, 2 H) 7,67 - 7,75 (m, 1 H) 8,23 (s large, 1 H) 8,34 (d large, *J*=8,3 Hz, 1 H) 8,57 (d, *J*=8,3 Hz, 1 H) 10,09 (s, 1 H) 13,04 (s large, 1 H)
Etape 3 : On introduit 38 mg de 4-{[5-(2,6-difluorophényl)-7-formyl-1H-pyrazolo[4,3-c]isoquinoléin-3-yl]amino}pipéridine-1-sulfonamide dans 2 mL de pyridine puis on ajoute 9 mg de chlorhydrate d'hydroxylamine et on agite 48 h à TA. Le mélange est concentré sous PR et le solide obtenu est repris dans de l'AcOEt, lavé avec une solution aqueuse 1 M de phosphate de potassium puis avec une solution aqueuse saturée en NaCl, séché sur MgSO₄, filtré et concentré sous PR. On purifie par flash-chromatographie sur gel de silice (20-45 µm), en éluant par un mélange DCM/EtOH (80/20 vol), on obtient 19 mg de 4-({5-(2,6-difluorophényl)-7-[(E/Z)-(hydroxyimino)méthyl]-1H-pyrazolo[4,3-c]isoquinoléin-3-yl}amino)pipéridine-1-sulfonamide (solide jaune). RMN ¹H : 1,60 - 1,73 (m, 2 H) 2,10 (m, 2 H) 2,68 (m, 2 H) 3,44 - 3,53 (m, 2 H) 3,70 (m, 1 H) 5,97 (d large, *J*=6,4 Hz, 1 H) 6,70 (s, 2 H) 7,32 (t, *J*=7,9 Hz, 2 H) 7,68 (m, 1 H) 7,84 (s large, 1 H) 8,14 (d large, *J*=8,7 Hz, 1 H) 8,29 (s, 1 H) 8,41 (d, *J*=8,7 Hz, 1 H) 11,42 (s large, 1 H) 12,79 (s large, 1 H)

### Exemple 24 5-(2,6-difluorophényl)-N-[1-(méthylsulfonyl)pipéridin-4-yl]-7-(1H-pyrazol-4-yl)-1H-pyrazolo[4,3-c]isoquinoléin-3-amine

Etape 1 . On introduit 342 mg de 3-bromo-5-(2,6-difluorophényl)-2-(2-triméthylsilanyl-éthoxyméthyl)-7-iodo-2H-pyrazolo[4,3-c]isoquinoléine dans 13 mL de 1-4-dioxane. On ajoute 74 mg d'acide 4-pyrazole boronique, 77 mg de Pd(PPh₃)₄ et 165 mg de carbonate de sodium. Le mélange est chauffé 4h30 à reflux. Après refroidissement, le mélange est coulé dans de l'eau, extrait à l'AcOEt, lavé avec une solution saturée en NaCl, séché sur MgSO₄, filtré et concentré sous PR. On purifie par flash-chromatographie sur gel de silice (15-40 µm), en éluant par un mélange DCM/méthanol (95/05 vol), on obtient ainsi, 136 mg de 3-bromo-5-(2,6-difluorophényl)-7-(1H-pyrazol-4-yl)-2-{[2-(triméthylsilyl)éthoxy]méthyl}-2H-pyrazolo[4,3-c]isoquinoléine (meringue blanche) utilisé tel quel dans l'étape suivante.
Etape 2: On introduit 115 mg de 3-bromo-5-(2,6-difluorophényl)-7-(1H-pyrazol-4-yl)-2-{[2-(triméthylsilyl)éthoxy]méthyl}-2H-pyrazolo[4,3-c]isoquinoléine dans 2 mL de DMF, 0,26 mL de diisopropyléthylamine et 77 µL de 2-(triméthylsilyl)éthoxyméthyl chloride. On agite le milieu réactionnel pendant 18 h à TA puis on coule dans de l'eau, extrait à l'AcOEt, lave avec une solution saturée en NaCl, sèche sur MgSO₄, filtre et concentre sous PR. On purifie par chromatographie sur gel de silice (20-45 µm), en éluant par un mélange de DCM et de méthanol (98/02 vol), on obtient ainsi 116 mg de 3-bromo-5-(2,6-difluorophényl)-2-{[2-(triméthylsilyl)éthoxy]méthyl}-7-(1-{[2-(triméthylsilyl)éthoxy]méthyl}-1H-pyrazol-4-yl)-2H-pyrazolo[4,3-c]isoquinoléine (poudre jaune). RMN ¹H : -0,01 (d, *J*=5,9 Hz, 4 H) 0,82 - 1,00 (m, 1 H) 3,60 (t, *J*=7,8 Hz, 1 H) 3,77 (t, 1 H) 5,46 (s, 1 H) 5,92 (s, 1 H) 7,42 (t, 1 H) 7,71 - 7,83 (m, 1 H) 8,03 (s, 1 H) 8,28 (dd, 1 H) 8,43 (s, 1 H) 8,58 (d, *J*=8,3 Hz, 1 H).
Etape 3 : On introduit 110 mg de 3-bromo-5-(2,6-difluorophényl)-2-{[2-(triméthylsilyl)éthoxy]méthyl}-7-(1-{[2-(triméthylsilyl)éthoxy]méthyl}-1H-pyrazol-4-yl)-2H-pyrazolo[4,3-c]isoquinoléine dans 5 mL de 1-4-dioxane. Après un barbotage de 10 min d'argon dans le milieu, on ajoute 57 mg de 1-méthanesulfonyl-pipéridin-4-ylamine, 199 mg de carbonate de césium, 16 mg de 9,9-diméthyl-4,5-bis(diphénylphosphino)xanthène et 5 mg d'acétate de palladium (II). Le mélange est chauffé 2 h au reflux. Après refroidissement, le mélange est coulé dans de l'eau, extrait à l'AcOEt, lavé avec une solution saturée en NaCl, séché sur MgSO₄, filtré et concentré sous PR. On purifie par flash-chromatographie sur gel de silice (40-63 µm), en éluant par un mélange DCM/méthanol (97,5/2,5 vol), on obtient 77 mg de 5-(2,6-difluorophényl)-N-[1-(méthylsulfonyl)pipéridin-4-yl]-2-{[2-(triméthylsilyl)éthoxy]méthyl}-7-(1-{[2-(triméthylsilyl)éthoxy]méthyl}-1H-pyrazol-4-yl)-2H-pyrazolo[4,3-c]isoquinoléin-3-amine (meringue jaune). RMN ¹H : -0,07 (s, 9 H) -0,02 (s, 9 H) 0,84 (t , *J*=8,3 Hz, 2H) 0,90 (t, *J=*8,3 Hz, 2 H) 1,56 - 1,68 (m, 2 H) 2,11 (m, 2 H) 2,76 (m, 2 H) 2,86 (s, 3 H) 3,56 (t, *J*=8,3 Hz, 2 H) 3,61 (m, 2H) 3,68 (t, *J*=8,3 Hz, 2 H) 4,47 - 4,57 (m, 1 H) 5,42 (s, 2 H) 5,65 (s, 2 H) 6,46 (d, *J*=8,0 Hz, 1 H) 7,34 (t, *J*=7,9 Hz, 2 H) 7,59 (s large, 1 H) 7,66 (m, 1 H) 7,92 (s, 1 H) 8,05 (dd, *J*=8,3, 1,7 Hz, 1 H) 8,31 (s, 1 H) 8,34 (d, *J*=8,3 Hz, 1 H)
Etape 4 : On introduit 73 mg de 5-(2,6-difluorophényl)-N-[1-(méthylsulfonyl)pipéridin-4-yl]-2-{[2-(triméthylsilyl)éthoxy]méthyl}-7-(1-{[2-(triméthylsilyl)éthoxy]méthyl}-1H-pyrazol-4-yl)-2H-pyrazolo[4,3-c]isoquinoléin-3-amine dans 5 mL de DCM. Après refroidissement vers 0°C à l'aide d'un bain de glace, on ajoute 0,58 mL de TFA et on agite à TA pendant 5 h. Le mélange réactionnel est concentré sous PR et le solide obtenu est repris dans 23 mL d'eau, neutralisé par une solution aqueuse d'ammoniaque 7,5 M. On extrait à l'acétate d'éthyle, sèche sur MgSO₄, filtre et concentre sous PR. On purifie par flash-chromatographie sur gel de silice (15-40 µm), en éluant par du DCM puis par un mélange DCM/méthanol (90/10 vol) pour conduire à 5 mg de 5-(2,6-difluorophényl)-N-[1-(méthylsulfonyl)pipéridin-4-yl]-7-(1H-pyrazol-4-yl)-1H-pyrazolo[4,3-c]isoquinoléin-3-amine (solide jaune).RMN ¹H : 1,61 - 1,74 (m, 2 H) 2,06 - 2,16 (m, 2 H) 2,87 (s, 3 H) 2,89 - 2,95 (m, 2 H) 3,52 - 3,60 (m, 2 H) 3,75 - 3,84 (m, 1 H) 5,98 (d, *J*=8,2 Hz,1 H) 7,31 (t, *J*=8,0 Hz, 2 H) 7,65 - 7,71 (m, 2 H) 7,82 - 7,88 (m étalé, 1 H) 8,15 - 8,24 (m étalé, 1 H) 8,18 (d large, *J*=8,3 Hz, 1 H) 8,40 (d, *J*=8,3 Hz, 1 H) 12,66 (s, 1 H) 13,05 (s large, 1 H)

### Exemple 25 5-(2,6-difluorophényl)-N-[1-(méthylsulfonyl)pipéridin-4-yl]-7-(2H-1,2,3-triazol-4-yl)-1H-pyrazolo[4,3-c]isoquinoléin-3-amine

Etape 1 : On introduit 239 mg de 5-(2,6-difluorophényl)-3-{[1-(méthylsulfonyl)pipéridin-4-yl]amino}-2-{[2-(triméthylsilyl)éthoxy]méthyl}-7-éthynyl-2H-pyrazolo[4,3-c]isoquinoléine préparé à l'étape 4 de l'ex. 7 dans 4 mL de méthanol et 4 mL de DMF, on ajoute 208 µL de triméthylsilyl azide et 15 mg d'iodure de cuivre (I). Après 2 h de chauffage à 100°C, on ajoute 104 µL de triméthylsilyl azide et 7 mg d'iodure de cuivre (I) et poursuit le chauffage 2 h à 100°C. Après refroidissement, le mélange est coulé dans de l'eau, extrait à l'AcOEt, lavé avec une solution saturée en NaCl, séché sur MgSO₄, filtré et concentré sous PR. On purifie par flash-chromatographie sur gel de silice (40-63 µm), en éluant par un mélange DCM/méthanol (95/05 vol), on obtient 121 mg de 5-(2,6-difluorophényl)-N-[1-(méthylsulfonyl)pipéridin-4-yl]-7-(2H-1,2,3-triazol-4-yl)-2-{[2-(triméthylsilyl)éthoxy]méthyl}-2H-pyrazolo[4,3-c]isoquinoléine-3-amine (meringue jaune). RMN ¹H : -0,04 (s, 9 H) 0,90 (m, 2 H) 1,61 (m, 2 H) 2,12 (m, 2 H) 2,75 (m, 2 H) 2,85 (s, 3 H) 3,57 (m, 2 H) 3,68 (m, 2 H) 4,47 (m, 1 H) 5,66 (s, 2 H) 6,49 (d, *J*=8,4 Hz, 1 H) 7,36 (t, *J*=8,0 Hz, 2 H) 7,67 (m, 1 H) 8,01 (s large, 1 H) 8,25 (d large, *J*=8,5 Hz, 1 H) 8,41 (d, *J*=8,5 Hz, 1 H) 8,43 (m étalé partiellement masqué, 1 H) 15,28 (m étalé, 1 H)
Etape 2 : On introduit 116 mg de 5-(2,6-difluorophényl)-N-[1-(méthylsulfonyl)pipéridin-4-yl]-7-(2H-1,2,3-triazol-4-yl)-2-{[2-(triméthylsilyl)éthoxy]méthyl}-2H-pyrazolo[4,3-c]isoquinoléin-3-amine dans 10 mL de DCM. Après refroidissement vers 0°C à l'aide d'un bain de glace, on ajoute 1,1 mL de TFA et on agite 5 h à TA. Le mélange est concentré sous PR et le solide obtenu est repris dans 60 mL d'eau, neutralisé par une solution aqueuse d'ammoniaque 7,5 M. On extrait à l'acétate d'éthyle, sèche sur MgSO₄, filtre et concentre sous PR. On purifie par flash-chromatographie sur gel de silice (15-40 µm), en éluant par un mélange DCM/méthanol (92,5/7,5 vol) pour conduire à 10 mg de 5-(2,6-difluorophényl)-N-[1-(méthylsulfonyl)pipéridin-4-yl]-7-(2H-1,2,3-triazol-4-yl)-1H-pyrazolo[4,3-c]isoquinoléin-3-amine (solide jaune). RMN ¹H: 1,62 - 1,75 (m, 2 H) 2,10 (m, 2 H) 2,82 - 2,97 (m, 5 H) 3,57 (m, 2 H) 3,74 - 3,86 (m, 1 H) 6,04 (d, *J*=8,0 Hz, 1 H) 7,35 (t, *J*= 8,0 Hz, 2 H) 7,63 - 7,75 (m, 1 H) 8,13 (s large,1 H) 8,39 (d large, *J*=8,6 Hz, 1 H) 8,45 (m étalé, 1 H) 8,49 (d, *J*=8,6 Hz, 1 H) 12,77 (s, 1 H) 15,23 (m étalé,1 H)

### Exemple 26 4-{[7-cyano-5-(2,6-difluorophényl)-1H-pyrazolo[4,3-c]isoquinoléin-3-yl]amino}pipéridine-1-sulfonamide

On introduit 306 mg de 4-{[7-cyano-5-(2,6-difluorophényl)-2-{[2-(triméthylsilyl)éthoxy]méthyl}-2H-pyrazolo[4,3-c]isoquinoléin-3-yl]amino}pipéridine-1-sulfonamide dans 20 mL de DCM. Après refroidissement vers 0°C à l'aide d'un bain de glace, on ajoute 1,5 mL de TFA et on agite 16 h à TA. Le mélange est concentré sous PR et le solide obtenu est repris dans 30 mL d'eau, neutralisé par 2 mL d'une solution aqueuse d'ammoniaque 7,5 M. On extrait à l'acétate d'éthyle, sèche sur MgSO₄, filtre et concentre sous PR. On purifie par flash-chromatographie sur gel de silice (15-40 µm), en éluant par un mélange DCM/méthanol/NH₄OH (92,5/7,5/0,75 en volumes). On obtient 8 mg de 4-{[7-cyano-5-(2,6-difluorophényl)-1H-pyrazolo[4,3-c]isoquinoléin-3-yl]amino}pipéridine-1-sulfonamide. RMN ¹H : 1,61 - 1,73 (m, 2 H) 2,09 (m, 2 H) 2,63-2,72 (m, 2 H) 3,47 (m, 2 H) 3,67 - 3,76 (m, 1 H) 6,13 (d, *J*=7,0 Hz, 1 H) 6,70 (s, 2 H) 7,34 (t, *J*=7,8 Hz, 2 H) 7,65 - 7,74 (m, 1 H) 8,18 (s large, 1 H) 8,28 (d large, *J*=8,6 Hz, 1 H) 8,56 (d, *J*=8,6 Hz, 1 H) 13,05 (s, 1 H)

### Exemple 27 4-{[5-(2,6-difluorophényl)-7-(hydroxyméthyl)-1H-pyrazolo[4,3-c]isoquinoléin-3-yl]amino}pipéridine-1-sulfonamide

Etape 1 On introduit 449 mg de 4-{[5-(2,6-difluorophényl)-7-formyl-2-{[2-(triméthylsilyl)éthoxy]méthyl}-2H-pyrazolo[4,3-c]isoquinoléin-3-yl]amino}pipéridine-1-sulfonamide préparé à l'étape 1 de l'exemple 23 dans 20 mL de méthanol. Après refroidissement vers 0°C à l'aide d'un bain de glace, on ajoute 34 mg de NaBH₄ et on agite pendant 1 h. On ajoute 3 mL d'acétone puis on coule le mélange dans une solution aqueuse saturée de bicarbonate de sodium. On extrait à l'AcOEt, lave avec une solution saturée en NaCl, sèche sur MgSO₄, filtre et concentre sous PR. On purifie par flash-chromatographie sur gel de silice (15-40 µm), en éluant par un mélange DCM/méthanol/NH₄OH (95/5/0,5 en volumes). On obtient 197 mg de 4-{[5-(2,6-difluorophényl)-7-(hydroxyméthyl)-2-{[2-(triméthylsilyl)éthoxy]méthyl}-2H-pyrazolo[4,3-c]isoquinoléin-3-yl]amino}pipéridine-1-sulfonamide (meringue jaune). RMN ¹H: - 0,05 (s, 9 H) 0,88 (t, *J*=8,3, 2 H) 1,58 (m, 2 H) 2,09 (m, 2 H) 2,52 (m partiellement masqué, 2 H) 3,48 (m, 2 H) 3,66 (t, *J*=8,3 Hz, 2 H) 4,34 - 4.43 (m, 1 H) 4,58 (d, *J*=5,7 Hz, 2 H) 5,38 (t, *J*=5,7 Hz, 1 H) 5,62 (s, 2 H) 6,39 (d, *J*=8,3 Hz, 1 H) 6,70 (s, 2 H) 7,30 (t, *J*=7,8 Hz, 2 H) 7,43 (s large, 1 H) 7,58 - 7,67 (m, 1 H) 7,70 (dd, *J*=8,3, 1,5 Hz, 1 H) 8,33 (d, *J*=8,3 Hz, 1 H)
Etape 2 On introduit 194 mg de 4-{[5-(2,6-difluorophényl)-7-(hydroxyméthyl)-2-{[2-(triméthylsilyl)éthoxy]méthyl}-2H-pyrazolo[4,3-c]isoquinoléin-3-yl]amino}pipéridine-1-sulfonamide dans 25 mL de DCM. Après refroidissement vers 0°C à l'aide d'un bain de glace, on ajoute 1,9 mL de TFA et on agite 2h30 à TA. Le mélange est concentré sous PR et le solide obtenu est repris dans 50 mL d'eau, neutralisé par 2,5 mL d'une solution aqueuse d'ammoniaque 7,5 M. On extrait à l'acétate d'éthyle, sèche sur MgSO₄, filtre et concentre sous PR. On purifie par flash-chromatographie sur gel de silice (15-40 µm), en éluant par un mélange DCM/méthanol/NH₄OH (90/10/1 en volumes). On obtient 28 mg de 4-{[5-(2,6-difluorophényl)-7-(hydroxyméthyl)-1H-pyrazolo[4,3-c]isoquinoléin-3-yl]amino}pipéridine-1-sulfonamide. RMN ¹H: 1,60 - 1,73 (m, 2 H) 2,05 - 2,16 (m, 2 H) 2,62 - 2,73 (m, 2 H) 3,46 (d, *J*=11,2 Hz, 2 H) 3,64 - 3,76 (m, 1 H) 4,63 (d, *J*=5,4 Hz, 2 H) 5,34 (t, *J*=5,9 Hz, 1 H) 5,75 (s, 1 H) 5,91 (d, 1 H) 6,70 (s, 2 H) 7,32 (t, 2 H) 7,59 (s, 1 H) 7,61 - 7,71 (m, 1 H) 7,84 (dd, 1 H) 8,35 (d, *J*=8,3 Hz, 1 H) 12,66 (s, 1 H)

### Exemple 28 4-{[5-(2,6-difluorophényl)-7-fluoro-1H-pyrazolo[4,3-c]isoquinoléin-3-yl]amino}pipéridine-1-sulfonamide

Etape 1 : On procède comme à l'étape 3 de l'ex.24, en introduisant 310 mg de 3-bromo-5-(2,6-difluorophényl)-7-fluoro-2-{[2-(triméthylsilyl)éthoxy]méthyl}-1H-pyrazolo[4,3-c]isoquinoléine dans 8 mL de 1-4-dioxane. Après un barbotage de 10 min d'argon dans le mélange, on ajoute 244 mg de 4-aminopipéridine-1-carboxylate de 2-méthylpropan-2-yle, 755 mg de carbonate de césium, 63 mg de 9,9-diméthyl-4,5-bis(diphénylphosphino)xanthène et 20 mg d'acétate de palladium (II). Le mélange est chauffé 3 h au reflux. Après refroidissement, le mélange est coulé dans de l'eau, extrait à l'AcOEt, lavé avec une solution saturée en NaCl, séché sur MgSO₄, filtré et concentré sous PR. On purifie par flash-chromatographie sur gel de silice (15-40 µm), en éluant par un mélange DCM/méthanol (90/10 vol), on obtient 340 mg de 4-{[5-(2,6-difluorophényl)-7-fluoro-2-{[2-(triméthylsilyl)éthoxy]méthyl}-2H-pyrazolo[4,3-c]isoquinoléin-3-yl]amino}pipéridine-1-carboxylate de 2-méthylpropan-2-yle (solide jaune). LC-MS-DAD-ELSD : 628 =(M+H)
Etape 2 : On introduit 340 mg de 4-{[5-(2,6-difluorophényl)-7-fluoro-2-{[2-(triméthylsilyl)éthoxy]méthyl}-2H-pyrazolo[4,3-c]isoquinoléin-3-yl]amino}pipéridine-1-carboxylate de 2-méthylpropan-2-yle dans 16 mL de méthanol et 1,5 mL de THF. Après refroidissement vers 0°C à l'aide d'un bain de glace, on ajoute 2,7 mL de solution d'acide chlorhydrique 4N dans le dioxanne et on agite à TA pendant 5 h. Le mélange est versé sur 200 mL d'une solution saturée en bicarbonate de sodium puis extrait à l'acétate d'éthyle, on sèche sur MgSO₄, filtre et concentre sous PR. On purifie par flash-chromatographie sur gel de silice (15-40 µm), en éluant par un mélange DCM/méthanol/ammoniaque (90/10/1 vol) pour conduire à 180 mg de 5-(2,6-difluorophényl)-7-fluoro-N-(pipéridin-4-yl)-2-{[2-(triméthylsilyl)éthoxy]méthyl}-2H-pyrazolo[4,3-c]isoquinoléin-3-amine (solide jaune). LC-MS-DAD-ELSD : 528 =(M+H)
Etape 3 : 180 mg de 5-(2,6-difluorophényl)-7-fluoro-N-(pipéridin-4-yl)-2-{[2-(triméthylsilyl)éthoxy]méthyl}-2H-pyrazolo[4,3-c]isoquinoléin-3-amine, 2 mL de DME et 41 mg de sulfamide sont mélangés et chauffés aux micro-ondes 1 h à 150°C. Le mélange est coulé dans de l'eau, extrait à l'AcOEt, lavé avec une solution saturée en NaCl, séché sur MgSO₄, filtré et concentré sous PR. On purifie par flash-chromatographie sur gel de silice (15-40 µm), en éluant par un mélange DCM/méthanol/ammoniaque (95/05/0,5 vol), on obtient 183 mg de 4-{[5-(2,6-difluorophényl)-7-fluoro-2-{[2-(triméthylsilyl)éthoxy]méthyl}-2H-pyrazolo[4,3-c]isoquinoléin-3-yl]amino}pipéridine-1-sulfonamide (solide jaune). LC-MS-DAD-ELSD : 607 =(M+H)

### Etape 4

On introduit 183 mg de 4-{[5-(2,6-difluorophényl)-7-fluoro-2-{[2-(triméthylsilyl)éthoxy]méthyl}-2H-pyrazolo[4,3-c]isoquinoléin-3-yl]amino}pipéridine-1-sulfonamide dans 24 mL de DCM. Après refroidissement vers 0°C à l'aide d'un bain de glace, on ajoute 1,9 mL de TFA et on agite 2h30 à TA. Le mélange est concentré sous PR et le solide obtenu est repris dans 50 mL d'eau, neutralisé par 2,5 mL d'une solution aqueuse d'ammoniaque 7,5 M. On extrait à l'acétate d'éthyle, sèche sur MgSO₄, filtre et concentre sous PR. On purifie par flash-chromatographie sur gel de silice (15-40 µm), en éluant par un mélange DCM/méthanol/ammoniaque (90/10/1 en volumes). On obtient ainsi, 28 mg 4-{[5-(2,6-difluorophényl)-7-fluoro-1H-pyrazolo[4,3-c]isoquinoléin-3-yl]amino}pipéridine-1-sulfonamide (solide jaune pâle). LC-MS-DAD-ELSD : 477=(M+H) RMN ¹H: 1,66 (m, 2 H) 2,11 (m, 2 H) 2,67 (m, 2 H) 3,46 (m, 2 H) 3,70 (m, 1 H) 6,03 (d, *J*=7,7 Hz, 1 H) 6,72 (s, 2 H) 7,26 - 7,40 (m, 3 H) 7,68 (m, 1 H) 7,88 (td *J*=9,0, 2,5 Hz, 1 H) 8,50 (dd, *J*=9,0, 5,5 Hz, 1 H) 12,79 (s, 1 H)

### Détermination de IC50 pour cellules HeLa (ATCC CCL-2)

Pour ce test, on utilise des lignées cellulaires tumorales adhérentes. Leur prolifération est mesurée par l'intermédiaire de la quantité de ¹⁴C-thymidine incorporée à l'intérieur des cellules. Les cellules sont mises dans des plaques Cytostar 96 puits avec du milieu de culture et incubées pendant 4 h à 37°C - 5% CO₂. Les produits à tester sont ensuite ajoutés et les plaques sont remises à l'incubateur. La ¹⁴C-thymidine est ajoutée au milieu après 72 h de traitement et les cellules sont ensuite incubées pendant 24 h. La mesure de l'incorporation de ¹⁴C-thymidine est réalisée à l'aide d'un lecteur MicroBeta (Perkin Elmer) après ces 24 h de « pulse ». La durée totale de traitement des cellules avec le produit à tester est de 96 h.

Pour chaque essai, les plaques sont préparées aux concentrations cellulaires retenues. Les produits sont testés à 10 concentrations en duplicate. Le plus couramment la première concentration est 10 µM mais elle peut varier ainsi que la raison de la dilution en fonction du niveau d'activité des produits. Les dilutions sériées sont faites dans DMSO pur puis chaque concentration est diluée dans du milieu de culture. Tous les produits sont testés dans deux essais indépendants

### Matériel utilisé

Dulbecco (Gibco 11960-044)
Sérum de veau foetal inactivé (SVF) (Gibco 10500-056)
Pénicilline - Streptomycine - Glutamine (PSG) (Gibco 10378-016)
Plaque 96 puits Cytostar (Amersham RPNQ0162)
DMSO (Sigma D2650)
¹⁴C-Thymidine (NEN NEC-568)

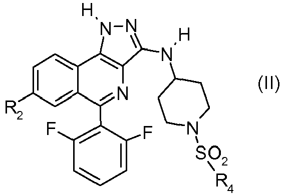

**Tableau I**

| **exemple** | **schéma de synthèse** | **R₂** | **R₄** | **IC₅₀ HeLa [nM]** |
|---|---|---|---|---|
| **1** | 1 - voie 3 | -C(=O)NH₂ | -Me | 259 |
| **2** | 1 - voie 2 | -CH=N-OH | -Me | 100 |
| **3** | 1 - voie 2 | -CH₂OH | -Me | 248 |
| **4** | 1 - voie 2 | -C(=O)NH₂ | -CF₃ | 789 |
| **5** | 1 - voie 1 | -CH=CH₂ | -Me | 2580 |
| **6** | 1 - voie 1 | -F | -Me | 890 |
| **7** | 1 - voie 2 | -C≡CH | -Me | 1500 |
| **8** | 1 - voie 1 | -C(=O)OMe | -Me | 7285 |
| **9** | 1 - voie 3 | -C(=S)NH₂ | -Me | 597 |
| **10** | 1 - voie 1 | -H | -Me | 2044 |
| **11** | 1 - voie 1 | -CN | -Cy | 909 |
| **12** | 1 - voie 1 | -CN | -Me | 899 |
| **13** | 1 - voie 1 | -CN | -Et | 954 |
| **14** | 1 - voie 3 | -C(=O)NH₂ | -Cy | 413 |
| **15** | 1 - voie 2 | -C(=O)H | -Me | 488 |
| **16** | 1 - voie 3 | 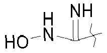 | -Me | 2350 |
| **17** | 1 - voie 2 | -CH₂F | -Me | 263 |
| **18** | 1 - voie 2 | -C(=O)NH₂ | -Et | 554 |
| **19** | 1 - voie 1 | -C(=O)H | -Et | 451 |
| **20** | 1 - voie 2 | -CH=N-OH | -Et | 138 |
| **21** | 1 - voie 2 | -CH₂OH | -Et | 338 |
| **22** | 1 - voie 2 | -C(=O)NH₂ | -NH₂ | 411 |
| **23** | 1 - voie 3 | -CH=N-OH | -NH₂ | 126 |
| **24** | 1 - voie 2 | 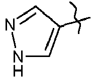 | -Me | 2125 |
| **25** | 1 - voie 2 | 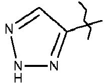 | -Me | 2283 |
| **26** | 1 - voie 1 | -CN | -NH₂ | 1660 |
| **27** | 1 - voie 2 | -CH₂OH | -NH₂ | 456 |
| **28** | 6 | -F | -NH₂ | 230 |

| | | | | |
|---|---|---|---|---|
| Me : méthyle, Et : éthyle, Cy : cyclopropyle | | | | |

## Revendications

1. Composé de formule (I) : 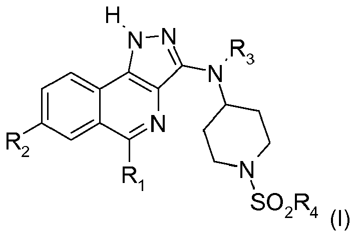 dans laquelle:
• **R₁** représente un groupe phényle éventuellement substitué par un ou plusieurs atome(s) d'halogène ;
• **R₂** représente :
- un atome d'hydrogène ou d'halogène ou un groupe cyano ;
- un groupe -C(=O)Y dans lequel Y représente un atome d'hydrogène, un groupe-NH₂ ou -ORₐ;
- un groupe -C(=S)NH₂ ;
- un groupe -C(=NH)NH-OH ;
- un groupe -CH₂OH ou -CH₂F ;
- un groupe -CH=N-OH ;
- un groupe -CH=CH₂ ou -C≡C-Rₐ ;
- un groupe 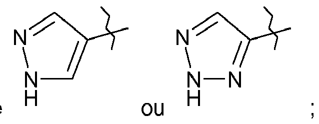 **Rₐ** représentant un atome d'hydrogène ou un groupe (C₁-C₄)alkyle ;
• **R₃** représente un atome d'hydrogène ou un groupe (C₁-C₄)alkyle ;
• **R₄** représente un groupe -NH₂, (C₁-C₄)alkyle, (C₁-C₄)fluoroalkyle ou (C₃-C₇)cycloalkyle.

2. Composé selon la revendication 1 **caractérisé en ce que R₁** représente le groupe 2,6-difluoro-phényle et R₃ représente un atome d'hydrogène.

3. Composé selon la revendication 1 ou 2 **caractérisé en ce que R₂** est choisi parmi : H, -C(=O)H, -C(=O)NH₂, -CH=N-OH, -CH₂OH, -CH=CH₂, -F, -C≡CH, -C(=O)OCH₃, -C(=S)NH₂, -CN,

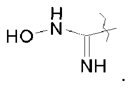

4. Composé selon l'une des revendications 1 à 3 **caractérisé en ce que R₄** est choisi parmi : -CH₃, -CH₂CH₃, -CF₃ ou le groupe cyclopropyle.

5. Composé selon l'une quelconque des revendications précédentes choisi dans la liste suivante :
■ 5-(2,6-difluorophényl)-3-{[1-(méthylsulfonyl)pipéridin-4-yl]amino}-1H-pyrazolo[4,3-c]isoquinoléine-7-carboxamide ;
■ 5-(2,6-difluorophényl)-3-{[1-(méthylsulfonyl)pipéridin-4-yl]amino}-1H-pyrazolo[4,3-c]isoquinoléine-7-carbaldéhyde oxime ;
■ [5-(2,6-difluorophényl)-3-{[1-(méthylsulfonyl)pipéridin-4-yl]amino}-1H-pyrazolo[4,3-c]isoquinoléin-7-yl]-méthanol ;
■ 5-(2,6-difluoro-phényl)-3-{[1-(trifluorométhanesulfonyl)pipéridin-4-yl]amino}-1H-pyrazolo[4,3-c]isoquinoléine-7-carboxamide ;
■ 5-(2,6-difluorophényl)-3-{[1-(méthylsulfonyl)pipéridin-4-yl]amino}-1H-vinyl-1H-pyrazolo[4,3-c]isoquinoléine ;
■ 5-(2,6-difluorophényl)-7-fluoro-3-{[1-(méthylsulfonyl)pipéridin-4-yl]amino}-1H-pyrazolo[4,3-c]isoquinoléine;
■ 5-(2,6-difluorophényl)-3-{[1-(méthylsulfonyl)pipéridin-4-yl]amino}-7-éthynyl-1H-pyrazolo[4,3-c]isoquinoléine ;
■ 5-(2,6-difluorophényl)-3-{[1-(méthylsulfonyl)pipéridin-4-yl]amino}-1H-pyrazolo[4,3c]isoquinoléine-7-carboxylate de méthyle ;
■ 5-(2,6-difluorophényl)-3-{[1-(méthylsulfonyl)pipéridin-4-yl]amino}-1H-pyrazolo[4,3-c]isoquinoléine-7-carbothioamide ;
■ 5-(2,6-difluorophényl)-3-{[1-(méthylsulfonyl)pipéridin-4-yl]amino}-1H-pyrazolo[4,3-c]isoquinoléine;
■ 5-(2,6-difluorophényl)-3-{[1-(cyclopropylsulfonyl)pipéridin-4-yl]amino}-1H-pyrazolo[4,3-c]isoquinoléine-1-carbonitrile ;
■ 5-(2,6-difluorophényl)-3-{[1-(méthylsulfonyl)pipéridin-4-yl]amino}-1H-pyrazolo[4,3-c]isoquinoléine-1-carbonitrile ;
■ 5-(2,6-difluorophényl)-3-{[1-(éthylsulfonyl)pipéridin-4-yl]amino}-1H-pyrazolo[4,3-c]isoquinoléine-7-carbonitrile ;
■ 5-(2,6-difluorophényl)-3-{[1-(cyclopropylsulfonyl)pipéridin-4-yl]amino}-1H-pyrazolo[4,3-c]isoquinoléine-7-carboxamide ;
■ 5-(2,6-difluorophényl)-3-{[1-(méthylsulfonyl)pipéridin-4-yl]amino}-1H-pyrazolo[4,3-c]isoquinoléine-7-carbaldéhyde ;
■ 5-(2,6-difluorophényl)-N-hydroxy-3-{[1-(méthylsulfonyl)pipéridin-4-yl]amino}-1H-pyrazolo[4,3-c]isoquinoléine-7-carboximidamide ;
■ 5-(2,6-difluorophényl)-7-(fluorométhyl)-N-[1-(méthylsulfonyl)pipéridin-4-yl]-1 H-pyrazolo[4,3-c]isoquinoléin-3-amine ;
■ 5-(2,6-difluorophényl)-3-{[1-(éthylsulfonyl)pipéridin-4-yl]amino}-1H-pyrazolo[4,3-c]isoquinoléine-7-carboxamide ;
■ 5-(2,6-difluorophényl)-3-{[1-(éthylsulfonyl)pipéridin-4-yl]amino}-1H-pyrazolo[4,3-c]isoquinoléine-7-carbaldéhyde ;
■ 5-(2,6-difluorophényl)-3-{[1-(éthylsulfonyl)pipéridin-4-yl]amino}-1H-pyrazolo[4,3-c]isoquinoléine-7-carbaldéhyde oxime ;
■ 5-(2,6-difluorophényl)-3-{[1-(éthylsulfonyl)pipéridin-4-yl]amino}-1H-pyrazolo[4,3-c]isoquinoléin-7-yl]-méthanol ;
■ 5-(2,6-difluorophényl)-3-[(1-sulfamoylpipéridin-4-yl)amino]-2H-pyrazolo[4,3-c]isoquinoléine-7-carboxamide ;
■ 4-({5-(2,6-difluorophényl)-7-[(E/Z)-(hydroxyimino)méthyl]-1H-pyrazolo[4,3-c]isoquinoléin-3-yl}amino)pipéridine-1-sulfonamide ;
■ 5-(2,6-difluorophényl)-N-[1-(méthylsulfonyl)pipéridin-4-yl]-7-(1H-pyrazol-4-yl)-1H-pyrazolo[4,3-c]isoquinoléin-3-amine;
■ 5-(2,6-difluorophényl)-N-[1-(méthylsulfonyl)pipéridin-4-yl]-1-(2H-1,2,3-triazol-4-yl)-1H-pyrazolo[4,3-c ]isoquinoléin-3-amine ;
■ 4-{[7-cyano-5-(2,6-difluorophényl)-1H-pyrazolo[4,3-c]isoquinoléin-3-yl]amino}pipéridine-1-sulfonamide;
■ 4-{[5-(2,6-difluorophényl)-1-(hydroxyméthyl)-1H-pyrazolo[4,3-c]isoquinoléin-3-yl]amino}pipéridine-1-sulfonamide;
■ 4-{[5-(2,6-difluorophényl)-7-fluoro-1H-pyrazolo[4,3-c]isoquinoléin-3-yl]amino}pipéridine-1-sulfonamide.

6. Composé selon l'une quelconque des revendications précédentes sous forme de base ou d'un sel d'addition à un acide.

7. Procédé de préparation d'un composé de formule (I) selon l'une quelconque des revendications précédentes : 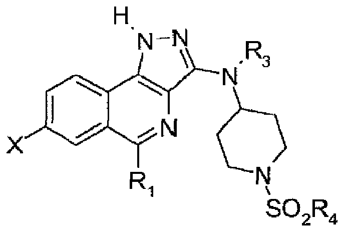 dans laquelle **R₁, R₃** et **R₄** sont tels que définis à l'une des revendications 1 à 6, et **X** représente un atome d'hydrogène ou de fluor, un groupe cyano, -C(=O)H, -CH=CH₂ , - C≡C-SiMe₃, -COOR₃, **Rₐ** représentant un atome d'hydrogène ou un groupe (C₁-C₄)alkyle, ou le groupe 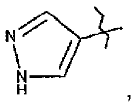 consistant à :
(i) coupler les composés **P₁** et **P₂** en présence d'un complexe de palladium et éventuellement d'une base : 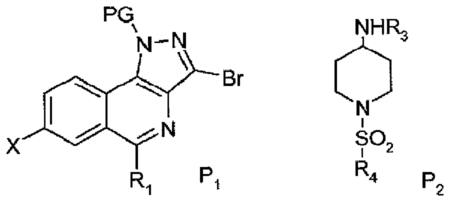
(ii) déproteger le produit obtenu à l'étape (i) précédente.
ou bien consistant à :
(i') faire réagir le composé **P'₄** de formule : 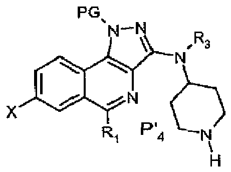 avec R₄SO₂Cl,
(ii') déproteger le produit obtenu à l'étape (i) précédente,
PG représentant un groupe protecteur de la fonction NH du cycle pyrazole.

8. Procédé de préparation d'un composé de formule (I) selon l'une quelconque des revendications 1 à 6 : 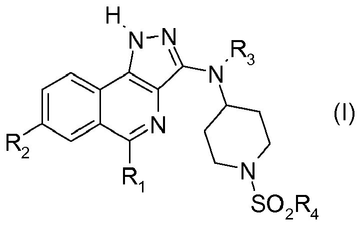 dans laquelle **R₁**, **R₃** et **R₄** sont tels que définis à l'une des revendications 1 à 6, et **R₂** représente l'un des groupes suivants : -C(=O)NH₂, -C(=S)NH₂, -C(=O)H, -CH=N-OH, -
CH₂OH, -CH₂F, -C≡CH, 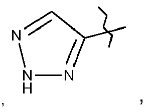 à partir du composé de formule : 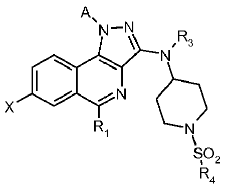 dans laquelle **X** représente un groupe -CN, -CHO, -CH₂OH, -C≡CH, -C≡C-SiMe₃ et **A** représente un atome d'hydrogène ou un groupe protecteur PG protégeant la fonction NH du cycle pyrazole, consistant à :
(i)
• hydrolyser le groupe X= -CN en groupe R₂= -C(=O)NH₂ ou -C(=O)OH ;
• transformer le groupe X= -CN en groupe R₂= -C(=S)NH₂ en présence de sulfure d'ammonium sous microondes ;
• réduire le groupe X= -CN en groupe R₂= - C(=O)H ;
• transformer le groupe X= -C(=O)H en groupe R₂= -CH=N-OH en présence de NH₂OH;
• réduire le groupe X= -C(=O)H en groupe R₂= -CH₂OH ;
• transformer le groupe X= -C≡C-SiMe₃ en groupe R₂= -C≡CH ;
• fluorer le groupe X= -CH₂OH en groupe R₂= -CH₂F ;
• transformer le groupe X= -C≡CH en groupe R₂= 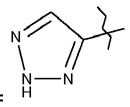 par cycloaddition en présence d'azoture de triméthylsilyle N₃SiMe₃.
(ii) le cas échéant déprotéger le produit obtenu à l'étape (i) précédente.

9. Médicament **caractérisé en ce qu'**il comprend un composé selon l'une des revendications 1 à 6.

10. Composition pharmaceutique **caractérisée en ce qu'**elle comprend un composé selon l'une des revendications 1 à 6 ainsi qu'au moins un excipient pharmaceutiquement acceptable.

11. Composé selon la revendication 1 à 6 pour son utilisation comme agent anticancéreux.

12. Utilisation d'un composé selon l'une des revendications 1 à 6 pour la fabrication d'un anticancéreux.

13. Composé de formule :
ou de formule 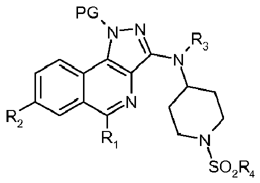 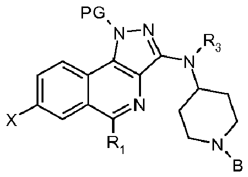 dans lesquelles :
■ **R₁**, **R₂**, **R₃** et **R₄** sont tels que définis à l'une des revendications 1 à 5 ;
■ **X** représente un atome d'hydrogène ou de fluor, un groupe cyano, -C(=O)H, - CH=CH₂ , -C≡C-SiMe₃, -C≡CH, -COORₐ, **Rₐ** représentant un atome
d'hydrogène ou un groupe (C₁-C₄)alkyle, ou le groupe 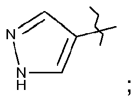
■ **PG** représente un groupe protecteur de la fonction NH du groupe pyrazole susceptible de protéger ladite fonction lors d'une réaction de couplage Büchwald-Hartwig;
■ **B** représente un atome d'hydrogène ou un groupe protecteur **PG'** de la fonction amine susceptible de protéger ladite fonction lors d'une réaction de couplage Büchwald-Hartwig.

14. Composé selon la revendication 13 dans lequel **PG** représente le SEM ou SO₂NMe₂ et/ou **PG'** représente le BOC.

15. Utilisation d'un composé selon la revendication 13 ou 14 comme intermédiaire réactionnel pour la préparation d'un composé selon la revendication 1 à 6.

## Patentansprüche

1. Verbindung der Formel (I): 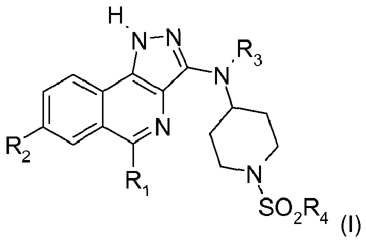 worin:
• **R₁** für eine gegebenenfalls durch ein oder mehrere Halogenatome substituierte Phenylgruppe steht;
• **R₂** für:
- ein Wasserstoff- oder Halogenatom oder eine Cyanogruppe;
- eine -C(=O)Y-Gruppe, worin Y für ein Wasserstoffatom oder eine -NH₂- oder -ORₐ-Gruppe steht;
- eine -C(=S)NH₂-Gruppe;
- eine -C(=NH)NH-OH-Gruppe;
- eine -CH₂OH- oder -CH₂F-Gruppe;
- eine -CH=N-OH-Gruppe;
- eine -CH=CH₂- oder -C≡C-Rₐ-Gruppe;
- eine Gruppe 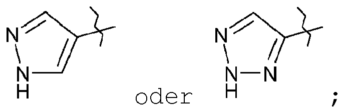 wobei **Rₐ** für ein Wasserstoffatom oder eine (C₁-C₄)-Alkylgruppe steht;
steht;
• **R₃** für ein Wasserstoffatom oder eine (C₁-C₄) - Alkylgruppe steht;
• **R₄** für eine -NH₂-, (C₁-C₄) -Alkyl-, (C₁-C₄)-Fluoralkyl- oder (C₃-C₇)-Cycloalkylgruppe steht.

2. Verbindung nach Anspruch 1, **dadurch gekennzeichnet, dass** R₁ für die 2,6-Difluorphenylgruppe steht und R₃ für ein Wasserstoffatom steht.

3. Verbindung nach Anspruch 1 oder 2, **dadurch gekennzeichnet, dass R₂** aus H, -C(=O)H, -C(=O)NH₂,
- CH=N-OH, -CH₂OH, -CH=CH₂, -F, -C≡CH, -C(=O)OCH₃,
- C(=S)NH₂, -CN, 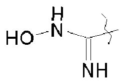 ausgewählt ist.

4. Verbindung nach einem der Ansprüche 1 bis 3, **dadurch gekennzeichnet, dass R₄** aus -CH₃, -CH₂CH₃,
- CF₃ oder der Cyclopropylgruppe ausgewählt ist.

5. Verbindung nach einem der vorhergehenden Ansprüche, ausgewählt aus der folgenden Liste:
■ 5-(2,6-Difluorphenyl)-3-{[1-(methylsulfonyl)-piperidin-4-yl]amino}-1H-pyrazolo[4,3-c]-isochinolin-7-carboxamid;
■ 5-(2,6-Difluorphenyl)-3-{[1-(methylsulfonyl)-piperidin-4-yl]amino}-1H-pyrazolo[4,3-c]-isochinolin-7-carbaldehydoxim;
■ 5-(2,6-Difluorphenyl)-3-{[1-(methylsulfonyl)-piperidin-4-yl]amino}-1H-pyrazolo[4,3-c]-isochinolin-7-yl]methanol;
■ 5-(2,6-Difluorphenyl)-3-{[1-(trifluormethan-sulfonyl)piperidin-4-yl]amino}-1H-pyrazolo[4,3-c]isochinolin-7-carboxamid;
■ 5-(2,6-Difluorphenyl)-3-{[1-(methylsulfonyl)-piperidin-4-yl]amino}-7-vinyl-1H-pyrazolo[4,3-c]isochinolin;
■ 5-(2,6-Difluorphenyl)-7-fluor-3-{[1-(methyl-sulfonyl)piperidin-4-yl]amino}-1H-pyrazolo[4,3-c]isochinolin;
■ 5-(2,6-Difluorphenyl)-3-{[1-(methylsulfonyl)-piperidin-4-yl]amino}-7-ethynyl-1H-pyrazolo-[4,3-c]isochinolin;
■ 5-(2,6-Difluorphenyl)-3-{[1-(methylsulfonyl)-piperidin-4-yl]amino}-1H-pyrazolo[4,3-c]-isochinolin-7-carbonsäuremethylester;
■ 5-(2,6-Difluorphenyl)-3-{[1-(methylsulfonyl)-piperidin-4-yl]amino}-1H-pyrazolo[4,3-c]-isochinolin-7-carbothioamid;
■ 5-(2,6-Difluorphenyl)-3-{[1-(methylsulfonyl)-piperidin-4-yl]amino}-1H-pyrazolo[4,3-c]-isochinolin;
■ 5-(2,6-Difluorphenyl)-3-{[1-(cyclopropyl-sulfonyl)piperidin-4-yl]amino}-1H-pyrazolo[4,3-c]isochinolin-7-carbonitril;
■ 5-(2,6-Difluorphenyl)-3-{[1-(methylsulfonyl)-piperidin-4-yl]amino}-1H-pyrazolo[4,3-c]-isochinolin-7-carbonitril;
■ 5-(2,6-Difluorphenyl)-3-{[1-(ethylsulfonyl)-piperidin-4-yl]amino}-1H-pyrazolo[4,3-c]-isochinolin-7-carbonitril;
■ 5-(2,6-Difluorphenyl)-3-{[1-(cyclopropyl-sulfonyl)piperidin-4-yl]amino}-1H-pyrazolo[4,3-c]isochinolin-7-carboxamid;
■ 5-(2,6-Difluorphenyl)-3-{[1-(methylsulfonyl)-piperidin-4-yl]amino}-1H-pyrazolo[4,3-c]-isochinolin-7-carbaldehyd;
■ 5-(2,6-Difluorphenyl)-N-hydroxy-3-{[1-(methyl-sulfonyl)piperidin-4-yl]amino}-1H-pyrazolo[4,3-c]isochinolin-7-carboximidamid;
■ [5-(2,6-Difluorphenyl)-7-(fluormethyl)-N-[1-(methylsulfonyl)piperidin-4-yl]-1H-pyrazolo-[4,3-c]isochinolin-3-amine;
■ 5-(2,6-Difluorphenyl)-3-{[1-(ethylsulfonyl)-piperidin-4-yl]amino}-1H-pyrazolo[4,3-c]-isochinolin-7-carboxamid;
■ 5-(2,6-Difluorphenyl)-3-{[1-(ethylsulfonyl)-piperidin-4-yl]amino}-1H-pyrazolo[4,3-c]-isochinolin-7-carbaldehyd;
■ 5-(2,6-Difluorphenyl)-3-{[1-(ethylsulfonyl)-piperidin-4-yl]amino}-1H-pyrazolo[4,3-c]-isochinolin-7-carbaldehydoxim;
■ 5-(2,6-Difluorphenyl)-3-{[1-(ethylsulfonyl)-piperidin-4-yl]amino}-1H-pyrazolo[4,3-c]-isochinolin-7-yl]methanol;
■ 5-(2,6-Difluorphenyl)-3-[(1-sulfamoylpiperidin-4-yl)amino]-2H-pyrazolo[4,3-c]isochinolin-7-carboxamid;
■ 4-({5-(2,6-Difluorphenyl)-7-[(E/Z)-(hydroxyl-imino)methyl]-1H-pyrazolo[4,3-c]isochinolin-3-yl}amino)piperidin-1-sulfonamid;
■ 5-(2,6-Difluorphenyl)-N-[1-(methylsulfonyl)-piperidin-4-yl]-7-(1H-pyrazol-4-yl)-1H-pyrazolo[4,3-c]isochinolin-3-amin;
■ 5-(2,6-Difluorphenyl)-N-[1-(methylsulfonyl)-piperidin-4-yl]-7-(2H-1,2,3-triazol-4-yl)-1H-pyrazolo[4,3-c]isochinolin-3-amin;
■ 4-{[7-Cyano-5-(2,6-difluorphenyl)-1H-pyrazolo-[4,3-c]isochinolin-3-yl]amino}piperidin-1-sulfonamid;
■ 4-{[5-(2,6-Difluorphenyl)-7-(hydroxymethyl)-1H-pyrazolo[4,3-c]isochinolin-3-yl]amino}-piperidin-1-sulfonamid;
■ 4-{[5-(2,6-Difluorphenyl)-7-fluor-1H-pyrazolo-[4,3-c]isochinolin-3-yl]amino}piperidin-1-sulfonamid.

6. Verbindung nach einem der vorhergehenden Ansprüche in Basen- oder Säureadditionssalzform.

7. Verfahren zur Herstellung einer Verbindung der Formel (I) nach einem der vorhergehenden Ansprüche: 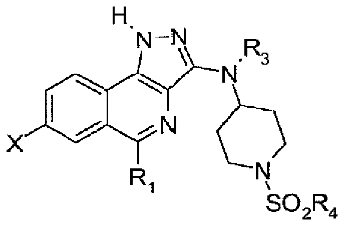 worin **R₁, R₃** und **R₄** wie in einem der Ansprüche 1 bis 6 definiert sind und **X** für ein Wasserstoff- oder Fluoratom, eine Cyanogruppe, oder eine - C(=O)H-, -CH=CH₂-, -C≡C-SiMe₃- oder -COORₐ-Gruppe, wobei **Rₐ** für ein Wasserstoffatom oder eine (C₁-C₄)-Alkylgruppe steht, oder die Gruppe 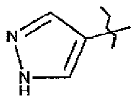 steht, bei dem man:
(i) die Verbindungen **P₁** und **P₂** in Gegenwart eines Palladiumkomplexes und gegebenenfalls einer Base kuppelt: 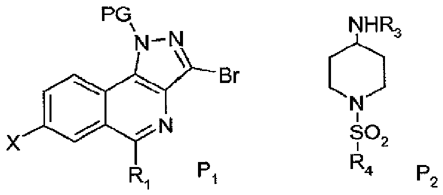
(ii) das im obigen Schritt (i) erhaltene Produkt entschützt;
oder bei dem man:
(i') die Verbindung P'₄ der Formel: 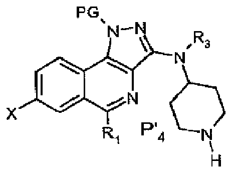 mit R₄SO₂Cl umsetzt,
(ii') das im obigen Schritt (i) erhaltene Produkt entschützt;
wobei PG für eine Schutzgruppe der NH-Funktion des Pyrazolrings steht.

8. Verfahren zur Herstellung einer Verbindung der Formel (I) nach einem der Ansprüche 1-6: 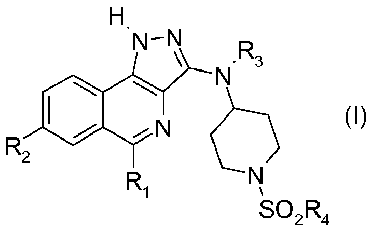 worin **R₁, R₃** und **R₄** wie in einem der Ansprüche 1 bis 6 definiert sind und **R₂** für eine der folgenden Gruppen steht: -C(=O)NH₂, -C(=S)NH₂, -C(=O)H,
- CH=N-OH, -CH₂OH, -CH₂F, -C≡CH, 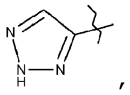
aus der Verbindung der Formel: 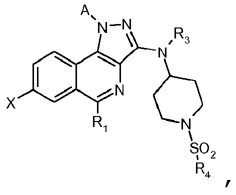 worin **X** für eine -CN-, -CHO-, -CH₂OH-, -C≡CH- oder
- C≡C-SiMe₃-Gruppe steht und **A** für ein Wasserstoffatom oder eine Schutzgruppe PG der NH-Funktion des Pyrazolrings steht,
bei dem man:
(i)
• die Gruppe X = -CN zu der Gruppe R₂ = -C(=O)NH₂ oder -C(=O)OH hydrolysiert;
• die Gruppe X = -CN in Gegenwart von Ammoniumsulfid unter Mikrowellen in die Gruppe R₂ = -C(=S)NH₂ umwandelt;
• die Gruppe X = -CN zu der Gruppe R₂ = -C(=O)H reduziert;
• die Gruppe X = -C(=O)H in Gegenwart von NH₂OH in die Gruppe R₂ = -CH=N-OH umwandelt;
• die Gruppe X = -C(=O)H zu der Gruppe R₂ = -CH₂OH reduziert;
• die Gruppe X = -C≡C-SiMe₃ in die Gruppe R₂ = -C≡CH umwandelt;
• die Gruppe X = -CH₂OH zu der Gruppe R₂ = -CH₂F fluoriert;
• die Gruppe X = -C≡CH durch Cycloaddition in Gegenwart von Trimethylsilylazid N₃SiMe₃ in die
Gruppe R₂ = 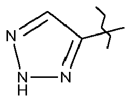 umwandelt;
(ii) gegebenenfalls das im obigen Schritt (i) erhaltene Produkt entschützt.

9. Medikament, **dadurch gekennzeichnet, dass** es eine Verbindung nach einem der Ansprüche 1 bis 6 umfasst.

10. Pharmazeutische Zusammensetzung, **dadurch gekennzeichnet, dass** sie eine Verbindung nach einem der Ansprüche 1 bis 6 sowie mindestens einen pharmazeutisch unbedenklichen Hilfsstoff umfasst.

11. Verbindung nach Anspruch 1 bis 6 zur Verwendung als Antikrebsmittel.

12. Verwendung einer Verbindung nach einem der Ansprüche 1 bis 6 zur Herstellung eines Antikrebsmittels.

13. Verbindung der Formel: 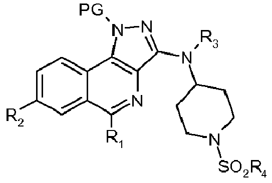 oder der Formel: 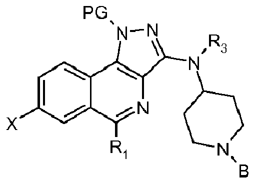 worin:
■ **R₁, R₂, R₃** und **R₄** wie in einem der Ansprüche 1 bis 5 definiert sind;
■ **X** für ein Wasserstoff- oder Fluoratom oder eine Cyano- -C(=O)H-, -CH=CH₂-, -C≡C-SiMe₃-, -C≡CH- oder -COORₐ-Gruppe, wobei **Rₐ** für ein Wasserstoffatom oder eine (C₁-C₄)-Alkylgruppe
steht, oder die Gruppe 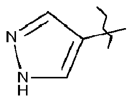 steht,
■ **PG** für eine Schutzgruppe der NH-Funktion der Pyrazolgruppe, die die Funktion während einer Büchwald-Hartwig-Kupplungsreaktion schützen kann, steht;
■ **B** für ein Wasserstoffatom oder eine Schutzgruppe **PG'** der Aminfunktion, die die Funktion während einer Büchwald-Hartwig-Kupplungsreaktion schützen kann, steht.

14. Verbindung nach Anspruch 13, worin **PG** für SEM oder SO₂NMe₂ steht und/oder **PG'** für BOC steht.

15. Verwendung einer Verbindung nach Anspruch 13 oder 14 als Reaktionszwischenprodukt zur Herstellung einer Verbindung nach Anspruch 1 bis 6.

## Claims

1. Compound of formula (I): 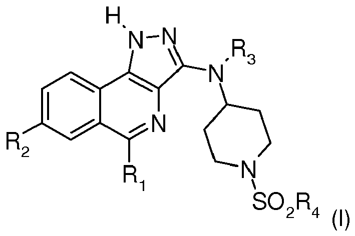 in which:
• **R₁** represents a phenyl group which is optionally substituted by one or more halogen atoms;
• **R₂** represents:
- a hydrogen or halogen atom or a cyano group;
- a group -C(=O)Y in which Y represents a hydrogen atom or a group -NH₂ or -ORₐ;
- a group -C(=S)NH₂;
- a group -C(=NH)NH-OH;
- a group -CH₂OH or -CH₂F;
- a group -CH=N-OH;
- a group -CH=CH₂ or -C≡C-Rₐ;
- a group 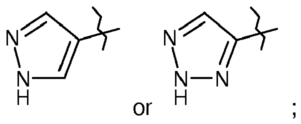 where **Rₐ** represents a hydrogen atom or a (C₁-C₄)alkyl group;
• **R₃** represents a hydrogen atom or a (C₁-C₄)alkyl group;
• **R₄** represents an -NH₂, (C₁-C₄)alkyl, (C₁-C₄)fluoroalkyl or (C₃-C₇)cycloalkyl group.

2. Compound according to Claim 1, **characterized in that R₁** represents the 2,6-difluorophenyl group and **R₃** represents a hydrogen atom.

3. Compound according to Claim 1 or 2, **characterized in that R₂** is selected from: H, -C(=O)H, -C(=O)NH₂, -CH=N-OH, -CH₂OH, -CH=CH₂, -F, -C≡CH, -C(=O)OCH₃, -C(=S)NH₂, -CN, -C(=O)H,

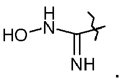

4. Compound according to any of Claims 1 to 3, **characterized in that R₄** is selected from: -CH₃, -CH₂CH₃, -CF₃ or the cyclopropyl group.

5. Compound according to any one of the preceding claims selected from the following list:
■ 5-(2,6-difluorophenyl)-3-{[1-(methylsulphonyl)piperidin-4-yl]amino}-1H-pyrazolo[4,3-c]isoquinoline-7-carboxamide;
■ 5-(2,6-difluorophenyl)-3-{[1-(methylsulphonyl)piperidin-4-yl]amino}-1H-pyrazolo[4,3-c]isoquinoline-7-carbaldehydeoxime;
■ [5-(2,6-difluorophenyl)-3-{[1-(methylsulphonyl)piperidin-4-yl]amino}-1H-pyrazolo[4,3-c]isoquinolin-7-yl]methanol;
■ 5-(2,6-difluorophenyl)-3-{[1-(trifluoromethanesulphonyl)piperidin-4-yl]amino}-1H-pyrazolo[4,3-c]isoquinoline-7-carboxamide;
■ 5-(2,6-difluorophenyl)-3-{[1-(methylsulphonyl)piperidin-4-yl]amino}-7-vinyl-1H-pyrazolo[4,3-c]isoquinoline;
■ 5-(2,6-difluorophenyl)-7-fluoro-3-{[1-(methylsulphonyl)piperidin-4-yl]amino}-1H-pyrazolo[4,3-c]isoquinoline;
■ 5-(2,6-difluorophenyl)-3-{[1-(methylsulphonyl)piperidin-4-yl]amino}-7-ethynyl-1H-pyrazolo[4,3-c]isoquinoline;
■ methyl5-(2,6-difluorophenyl)-3-{[1-(methylsulphonyl)piperidin-4-yl]amino}-1H-pyrazolo[4,3c]isoquinoline-7-carboxylate;
■ 5-(2,6-difluorophenyl)-3-{[1-(methylsulphonyl)piperidin-4-yl]amino}-1H-pyrazolo[4,3-c]isoquinoline-7-carbothioamide;
■ 5-(2,6-difluorophenyl)-3-{[1-(methylsulphonyl)piperidin-4-yl]amino}-1H-pyrazolo[4,3-c]isoquinoline;
■ 5-(2,6-difluorophenyl)-3-{[1-(cyclopropylsulphonyl)piperidin-4-yl]amino}-1H-pyrazolo[4,3-c]isoquinoline-7-carbonitrile;
■ 5-(2,6-difluorophenyl)-3-{[1-(methylsulphonyl)piperidin-4-yl]amino}-1H-pyrazolo[4,3-c]isoquinoline-7-carbonitrile;
■ 5-(2,6-difluorophenyl)-3-{[1-(ethylsulphonyl)piperidin-4-yl]amino}-1H-pyrazolo[4,3-c]isoquinoline-7-carbonitrile;
■ 5-(2,6-difluorophenyl)-3-{[1-(cyclopropylsulphonyl)piperidin-4-yl]amino}-1H-pyrazolo[4,3-c]isoquinoline-7-carboxamide;
■ 5-(2,6-difluorophenyl)-3-{[1-(methylsulphonyl)piperidin-4-yl]amino}-1H-pyrazolo[4,3-c]isoquinoline-7-carbaldehyde;
■ 5-(2,6-difluorophenyl)-N-hydroxy-3-{[1-(methylsulphonyl)piperidin-4-yl]amino}-1H-pyrazolo[4,3-c]isoquinoline-7-carboximidamide;
■ [5-(2,6-difluorophenyl)-7-(fluoromethyl)-N-[1-(methylsulphonyl)piperidin-4-yl]-1H-pyrazolo[4,3-c]isoquinolin-3-amine;
■ 5-(2,6-difluorophenyl)-3-{[1-(ethylsulphonyl)piperidin-4-yl]amino}-1H-pyrazolo[4,3-c]isoquinoline-7-carboxamide;
■ 5-(2,6-difluorophenyl)-3-{[1-(ethylsulphonyl)piperidin-4-yl]amino}-1H-pyrazolo[4,3-c]isoquinoline-7-carbaldehyde;
■ 5-(2,6-difluorophenyl)-3-{[1-(ethylsulphonyl)piperidin-4-yl]amino}-1 H-pyrazolo[4,3-c]isoquinoline-7-carbaldehyde oxime;
■ 5-(2,6-difluorophenyl)-3-{[1-(ethylsulphonyl)piperidin-4-yl]amino}-1H-pyrazolo[4,3-c]isoquinolin-7-yl]methanol;
■ 5-(2,6-difluorophenyl)-3-[(1-sulphamoylpiperidin-4-yl)amino]-2H-pyrazolo[4,3-c]isoquinoline-7-carboxamide;
■ 4-({5-(2,6-difluorophenyl)-7-[(E/Z)-(hydroxyimino)methyl]-1H-pyrazolo[4,3-c]isoquinolin-3-yl}amino)piperidine-1-sulphonamide;
■ 5-(2,6-difluorophenyl)-N-[1-(methylsulphonyl)piperidin-4-yl]-7-(1H-pyrazol-4-yl)-1 H-pyrazolo[4,3-c]isoquinolin-3-amine;
■ 5-(2,6-difluorophenyl)-N-[1-(methylsulphonyl)piperidin-4-yl]-7-(2H-1,2,3-triazol-4-yl)-1H-pyrazolo[4,3-c]isoquinolin-3-amine;
■ 4-{[7-cyano-5-(2,6-difluorophenyl)-1H-pyrazolo[4,3-c]isoquinolin-3-yl]amino}piperidine-1-sulphonamide;
■ 4-{[5-(2,6-difluorophenyl)-7-(hydroxymethyl)-1H-pyrazolo[4,3-c]isoquinolin-3-yl]amino}piperidine-1-sulphonamide;
■ 4-{[5-(2,6-difluorophenyl)-7-fluoro-1H-pyrazolo[4,3-c]isoquinolin-3-yl]amino}piperidine-1-sulphonamide.

6. Compound according to any one of the preceding claims, in the form of a base or an acid addition salt.

7. Process for preparing a compound of formula (I) according to any one of the preceding claims: 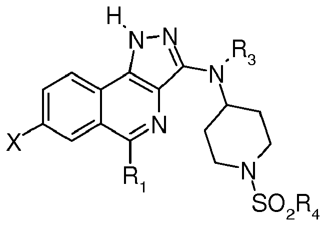 in which **R₁, R₃** and **R₄** are as defined in any of Claims 1 to 6, and **X** represents a hydrogen or fluorine atom or a group cyano, -C(=O)H, -CH=CH₂, -C≡C-SiMe₃, -COORₐ,
**Rₐ** representing a hydrogen atom or a (C₁-C₄)alkyl group, or the group 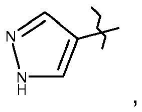 comprising:
(i) coupling the compounds **P₁** and **P₂** in the presence of a palladium complex and optionally of a base: 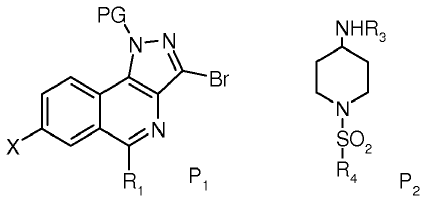
(ii) deprotecting the product obtained in the preceding step (i),
or else comprising:
(i') reacting the compound **P'₄** of formula: 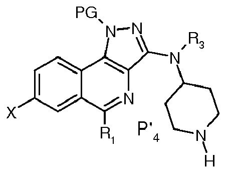 with R₄SO₂Cl,
(ii') deprotecting the product obtained in the preceding step (i'),
where PG represents a protective group for the NH function of the pyrazole ring.

8. Process for preparing a compound of formula (I) according to any one of claims 1 to 6: 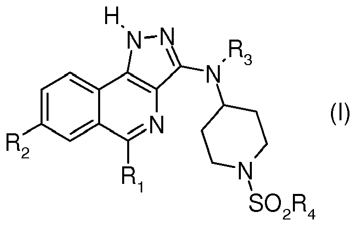 in which **R₁, R₃** and **R₄** are as defined in any of Claims 1 to 6, and R₂ represents one of the following groups: -C(=O)NH₂, -C(=S)NH₂, -C(=O)H, -CH=N-OH, -CH₂OH, -CH₂F, -C≡CH 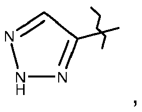 from the compound of formula: 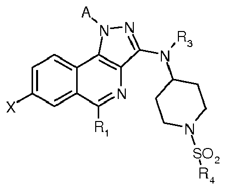 in which **X** represents a group -CN, -CHO, -CH₂OH, -C≡CH or -C≡C-SiMe₃ and **A** represents a hydrogen atom or a protective group PG protecting the NH function of the pyrazole ring, comprising:
(i)
• hydrolysing the group X= -CN to group R₂= -C(=O)NH₂ or -C(=O)OH;
• converting the group X= -CN to group R₂= -C(=S)NH₂ in the presence of ammonium sulphide with microwaves;
• reducing the group X= -CN to group R₂= - C(=O)H;
• converting the group X= -C(=O)H to group R₂= -CH=N-OH in the presence of NH₂OH;
• reducing the group X= -C(=O)H to group R₂= -CH₂OH;
• converting the group X= -C≡C-SiMe₃ to group R₂= -C≡CH;
• fluorinating the group X= -CH₂OH to group R₂= -CH₂F;
• converting the group X= -C≡CH to group R₂= 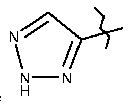 by cycloaddition in the presence of trimethylsilyl azide N₃SiMe₃;
(ii) where appropriate, deprotecting the product obtained in the preceding step (i).

9. Medicament **characterized in that** it comprises a compound according to any of Claims 1 to 6.

10. Pharmaceutical composition **characterized in that** it comprises a compound according to any of Claims 1 to 6 and at least one pharmaceutically acceptable excipient.

11. Compound according to Claims 1 to 6 for its use as anticancer agent.

12. Use of a compound according to any of Claims 1 to 6 for preparing an anticancer agent.

13. Compound of formula: 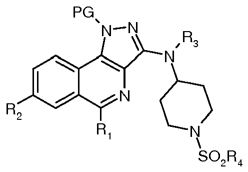 or of formula 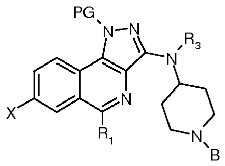 in which:
■ **R₁, R₂, R₃** and **R₄** are as defined in any of Claims 1 to 5;
■ **X** represents a hydrogen or fluorine atom, a group cyano, -C(=O)H, -CH=CH₂ , -C≡C-SiMe₃, -C≡CH, -COORₐ, where **Rₐ** represents a hydrogen atom or a (C₁-C₄)alkyl group, or the group 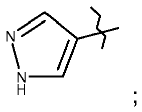
■ **PG** represents a protective group for the NH function of the pyrazole group allowing protection of said function during a Büchwald-Hartwig coupling;
■ **B** represents a hydrogen atom or a protective group **PG'** for the amine function allowing protection of said function during a Büchwald-Hartwig coupling.

14. Compound according to Claim 13, in which **PG** represents SEM or SO₂NMe₂ and/or **PG'** represents BOC.

15. Use of a compound according to Claim 13 or 14 as a reaction intermediate for preparing a compound according to Claims 1 to 6.
